(19)

Europäisches
Patentamt
European
Patent Office
Office européen
des brevets

(11)  **EP 2 444 409 A2**

(12)  **EUROPEAN PATENT APPLICATION**

(43) Date of publication:
    **25.04.2012 Bulletin 2012/17**

(51) Int Cl.:
    ***C07H 21/04*** (2006.01)          *C07K 14/47* (2006.01)

(21) Application number: **11189800.3**

(22) Date of filing: **15.09.2003**

(84) Designated Contracting States:
    **AT BE BG CH CY CZ DE DK EE ES FI FR GB GR
    HU IE IT LI LU MC NL PT RO SE SI SK TR**

(30) Priority: **16.09.2002 US 411392 P**

(62) Document number(s) of the earlier application(s) in
    accordance with Art. 76 EPC:
    **03768511.2 / 1 578 364**

(71) Applicant: **Genentech, Inc.**
    **South San Francisco, CA 94080 (US)**

(72) Inventors:
    • **Chiu, Henry**
      **San Francisco**
      **CA 94116 (US)**
    • **Clark, Hilary**
      **San Francisco**
      **CA 941341 (US)**
    • **Dennis, Kathryn**
      **San Jose**
      **CA 95129 (US)**

    • **Fong, Sherman**
      **Kalaheo**
      **HI 96741 (US)**
    • **Schoenfeld, Jill R.**
      **Ashland**
      **OR 97520 (US)**
    • **Wood, William I.**
      **Cupertino**
      **CA 95014 (US)**
    • **Wu, Thomas D.**
      **San Francisco**
      **CA 94110 (US)**

(74) Representative: **Woolley, Lindsey Claire**
    **Mewburn Ellis LLP**
    **33 Gutter Lane**
    **London**
    **EC2V 8AS (GB)**

Remarks:
    This application was filed on 18-11-2011 as a
    divisional application to the application mentioned
    under INID code 62.

(54)  **Compositions and methods for the treatment of immune related diseases**

(57)    The present invention relates to compositions containing novel proteins and methods of using those compositions for the diagnosis and treatment of immune related diseases.

EP 2 444 409 A2

**Description**

Field of the Invention

The present invention relates to compositions and methods useful for the diagnosis and treatment of immune related diseases.

Background of the Invention

The B lymphocytes play a major role in the humoral immune response as the antibody producing cells. The B cells can generate a highly diverse antibody repertoire that is reactive to almost all potential antigens. Through a process of maturation and clonal selection in the bone marrow, a highly diverse B cell population develops with each B cell clone expressing an antigen specific cell surface receptor, the B cell receptor (BCR), which bear the same specificity as the secreted antibody made by the B cell. These mature cells are involved in immunity against foreign and infectious agents, as well as autoimmunity, whereby they produce autoantibodies against self constituents.

The BCR complex on mature cells is composed of membrane IgM and IgD molecules associated with the invariant Igα and Igβ heterodimers, which contain two immunoreceptor tyrosine-based activation motifs (ITAM) in their cytoplasmic tails. Mature BCR bearing B cells seed the peripheral blood and recirculate through the primary lymphoid tissues, such as the lymph nodes, spleen, and mucosal lymphoid tissues. Cross-linking of membrane Ig by multivalent antigen triggers clustering of the Igα and Igβ heterodimers and leads to tyrosine phosphorylation of the ITAMs by the SRC-family protein tyrosine kinases (PTKs), such as Lyn, Fyn, Blk, and Lck. Since the BCR complex lacks intrinsic kinase activity and is believed excluded from lipid rafts in the membrane, oligomerized BCR are translocated to lipid rafts, where Lyn resides constitutively to mediate tyrosine phosphorylation of the ITAM domains. This BCR signaling process is dependent on a receptor-inducible assembly mechanism, associated with the recruitment of PTKs, adaptors or linker proteins, and effector enzymes to the cytoplasmic side of the plasma membrane. The linker proteins, such as BLNK, BCAP, GAB, PAG, and LAT help localize enzymatic complexes to the appropriate subcellular site for signaling. These linker proteins link cell surface receptors with effector enzymes and help modulate signal transduction by mediating protein-protein or protein-lipid interactions.

The stimulation of B cells with anti-CD40 can inimic B cell activation via BCR. CD40 ligation has been shown to induce B cell growth, survival, differentiation, Ig switching, germinal center formation, and enhancement of antigen presentation by B cells. CD40 ligation not only enhances the expression of PIM-1, a protooncogene that encodes a serine/threonine protein kinase, via NF-κB activation, but stimulates JNK, p38 kinases, and protein kinase C independent activation of ERK2, similar to stimulation of B cells with anti-IgM. CD40 ligation also induces phosphorylation of tyrosine kinases Lyn, Fyn, and Syk. The combination of IL-4 and anti-CD40 stimulation leads to enhanced B cell proliferation and Ig secretion. Therefore, a microarray experiment comparing differential expression of RNA from anti-CD40 and IL-4 stimulated vs resting B cells, can reveal new genes associated with B cell activation. Gene products associated with B cell activation can be targets for therapeutic drug development in the treatment of autoimmune mediated inflammatory diseases and B cell malignancies, as well as provide insights into genes that are defective in immune deficiency disorders.

Despite the above identified advances in B cell research, there is a great need for additional diagnostic and therapeutic agents capable of detecting the presence of a B cell mediated disorder in a mammal and for effectively reducing this disorder. Accordingly, it is an objective of the present invention to identify polypeptides that are overexpressed in activated B cells as compared to resting B cells, and to use those polypeptides, and their encoding nucleic acids, to produce compositions of matter useful in the therapeutic treatment and diagnostic detection of B cell mediated disorders in mammals.

Summary of the Invention

A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (e.g., neutralizing antibodies) can be used therapeutically where attenuation of the immune response would be beneficial (e.g., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) stimulating or enhancing an immune response in a mammal in need thereof, (b) increasing the proliferation of B-lymphocytes in a mammal in need thereof in response to an antigen, (c) increasing the Ig secretion of B-lymphocytes. In a further aspect, when the composition comprises an immune inhibiting molecule, the composition is useful for: (a) inhibiting or reducing an immune response in a mammal in need thereof, (b) decreasing the proliferation of B-lymphocytes or (c) decreasing the Ig secretion by B-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:

(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known

3

normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:

(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:

(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:

(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of B-lymphocytes in a mammal comprising administering to said mammal (a) a PRO polypeptide, (b) an agonist of a PRO polypeptide, or (c) an antagonist of a PRO polypeptide, wherein the activity of B-lymphocytes in the mammal is increased.

In a still further embodiment, the invention provides a method of decreasing the activity of B-lymphocytes in a mammal comprising administering to said mammal (a) a PRO polypeptide, (b) an agonist of a PRO polypeptide, or (c) an antagonist of a PRO polypeptide, wherein the activity of B-lymphocytes in the mammal is decreased.

In a still further embodiment, the invention provides a method of increasing the proliferation of B-lymphocytes in a mammal comprising administering to said mammal (a) a PRO polypeptide, (b) an agonist of a PRO polypeptide, or (c) an antagonist of a PRO polypeptide, wherein the proliferation of B-lymphocytes in the mammal is increased.

In a still further embodiment, the invention provides a method of decreasing the proliferation of B-lymphocytes in a mammal comprising administering to said mammal (a) a PRO polypeptide, (b) an agonist of a PRO polypeptide, or (c) an antagonist of a PRO polypeptide, wherein the proliferation of B-lymphocytes in the mammal is decreased.

B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

## BRIEF DESCRIPTION OF THE DRAWINGS

Figure 1 shows a nucleotide sequence (SEQ ID NO:1) of a native sequence cDNA, wherein SEQ ID NO:1 is a clone designated herein as "DNA328999".

Figure 2A-B shows a nucleotide sequence (SEQ ID NO:2) of a native sequence PRO12560 cDNA, wherein SEQ ID NO:2A-B is a clone designated herein as "DNA150956".

Figure 3 shows the amino acid sequence (SEQ ID NO:3) derived from the coding sequence of SEQ ID NO:2 shown in Figure 2A-B.

Figure 4 shows a nucleotide sequence (SEQ ID NO:4) of a native sequence PR0329 cDNA, wherein SEQ ID NO: 4 is a clone designated herein as "DNA323978".

Figure 5 shows the amino acid sequence (SEQ ID NO:5) derived from the coding sequence of SEQ ID NO:4 shown in Figure 4.

Figure 6 shows a nucleotide sequence (SEQ ID NO:6) of a native sequence PRO712 cDNA, wherein SEQ ID NO: 6 is a clone designated herein as "DNA304829".

Figure 7 shows the amino acid sequence (SEQ ID NO:7) derived from the coding sequence of SEQ ID NO:6 shown in Figure 6.

Figure 8 shows a nucleotide sequence (SEQ ID NO:8) of a native sequence PRO1265 cDNA, wherein SEQ ID NO: 8 is a clone designated herein as "DNA304827".

Figure 9 shows the amino acid sequence (SEQ ID NO:9) derived from the coding sequence of SEQ ID NO:8 shown in Figure 8.

Figure 10 shows a nucleotide sequence (SEQ ID NO:10) of a native sequence PR071045 cDNA, wherein SEQ ID NO:10 is a clone designated herein as "DNA304469".

Figure 11 shows the amino acid sequence (SEQ ID NO:11) derived from the coding sequence of SEQ ID NO:10 shown in Figure 10.

Figure 12 shows a nucleotide sequence (SEQ ID NO:12) of a native sequence PRO71049 cDNA, wherein SEQ ID NO:12 is a clone designated herein as "DNA304475".

Figure 13 shows the amino acid sequence (SEQ ID NO:13) derived from the coding sequence of SEQ ID NO:12 shown in Figure 12.

Figure 14 shows a nucleotide sequence (SEQ ID NO:14) of a native sequence PRO37162 cDNA, wherein SEQ ID NO:14 is a clone designated herein as "DNA226699".

Figure 15 shows the amino acid sequence (SEQ ID NO:15) derived from the coding sequence of SEQ ID NO:14 shown in Figure 14.

Figure 16 shows a nucleotide sequence (SEQ ID NO:16) of a native sequence PRO160 cDNA, wherein SEQ ID NO:16 is a clone designated herein as "DNA59763".

Figure 17 shows the amino acid sequence (SEQ ID NO:17) derived from the coding sequence of SEQ ID NO:16 shown in Figure 16.

Figure 18 shows a nucleotide sequence (SEQ ID NO:18) of a native sequence PRO37534 cDNA, wherein SEQ ID NO:18 is a clone designated herein as "DNA227071".

Figure 19 shows the amino acid sequence (SEQ ID NO:19) derived from the coding sequence of SEQ ID NO:18 shown in Figure 18.

Figure 20 shows a nucleotide sequence (SEQ ID NO:20) of a native sequence PR037544 cDNA, wherein SEQ ID NO:20 is a clone designated herein as "DNA227801".

Figure 21 shows the amino acid sequence (SEQ ID NO:21) derived from the coding sequence of SEQ ID NO:20 shown in Figure 20.

Figure 22 shows a nucleotide sequence (SEQ ID NO:22) of a native sequence PR021787 cDNA, wherein SEQ ID NO:22 is a clone designated herein as "DNA188293".

Figure 23 shows the amino acid sequence (SEQ ID NO:23) derived from the coding sequence of SEQ ID NO:22 shown in Figure 22.

Figure 24 shows a nucleotide sequence (SEQ ID NO:24) of a native sequence PRO34330 cDNA, wherein SEQ ID NO:24 is a clone designated herein as "DNA218278".

Figure 25 shows the amino acid sequence (SEQ ID NO:25) derived from the coding sequence of SEQ ID NO:24 shown in Figure 24.

Figure 26 shows a nucleotide sequence (SEQ ID NO:26) of a native sequence PR02540 cDNA, wherein SEQ ID NO:26 is a clone designated herein as "DNA76514".

Figure 27 shows the amino acid sequence (SEQ ID NO:27) derived from the coding sequence of SEQ ID NO:26 shown in Figure 26.

Figure 28 shows a nucleotide sequence (SEQ ID NO:28) of a native sequence PR07 cDNA, wherein SEQ ID NO: 28 is a clone designated herein as "DNA35629".

Figure 29 shows the amino acid sequence (SEQ ID NO:29) derived from the coding sequence of SEQ ID NO:28 shown in Figure 28.

Figure 30 shows a nucleotide sequence (SEQ ID NO:30) of a native sequence PRO34288 cDNA, wherein SEQ ID NO:30 is a clone designated herein as "DNA217246".

Figure 31 shows the amino acid sequence (SEQ ID NO:31) derived from the coding sequence of SEQ ID NO:30 shown in Figure 30.

Figure 32 shows a nucleotide sequence (SEQ ID NO:32) of a native sequence PR084690 cDNA, wherein SEQ ID NO:32 is a clone designated herein as "DNA329000".

Figure 33 shows the amino acid sequence (SEQ ID NO:33) derived from the coding sequence of SEQ ID NO:32 shown in Figure 32.

Figure 34 shows a nucleotide sequence (SEQ ID NO:34) of a native sequence PR02134 cDNA, wherein SEQ ID NO:34 is a clone designated herein as "DNA88034".

Figure 35 shows the amino acid sequence (SEQ ID NO:35) derived from the coding sequence of SEQ ID NO:34 shown in Figure 34.

Figure 36A-B shows a nucleotide sequence (SEQ ID NO:36) of a native sequence PR021928 cDNA, wherein SEQ ID NO:36 is a clone designated herein as "DNA188400".

Figure 37 shows the amino acid sequence (SEQ ID NO:37) derived from the coding sequence of SEQ ID NO:36 shown in Figure 36A-B.

Figure 38 shows a nucleotide sequence (SEQ ID NO:38) of a native sequence PR023974 cDNA, wherein SEQ ID NO:38 is a clone designated herein as "DNA194652".

Figure 39 shows the amino acid sequence (SEQ ID NO:39) derived from the coding sequence of SEQ ID NO:38 shown in Figure 38.

Figure 40 shows a nucleotide sequence (SEQ ID NO:40) of a native sequence PR034457 cDNA, wherein SEQ ID NO:40 is a clone designated herein as "DNA328555".

Figure 41 shows the amino acid sequence (SEQ ID NO:41) derived from the coding sequence of SEQ ID NO:40 shown in Figure 40.

Figure 42 shows a nucleotide sequence (SEQ ID NO:42) of a native sequence PR037158 cDNA, wherein SEQ ID NO:42 is a clone designated herein as "DNA226695".

Figure 43 shows the amino acid sequence (SEQ ID NO:43) derived from the coding sequence of SEQ ID NO:42 shown in Figure 42.

Figure 44A-B shows a nucleotide sequence (SEQ ID NO:44) of a native sequence PRO2537 cDNA, wherein SEQ ID NO:44 is a clone designated herein as "DNA76504".

Figure 45 shows the amino acid sequence (SEQ ID NO:45) derived from the coding sequence of SEQ ID NO:44 shown in Figure 44A-B.

Figure 46A-B shows a nucleotide sequence (SEQ ID NO:46) of a native sequence PRO36827 cDNA, wherein SEQ ID NO:46 is a clone designated herein as "DNA226364".

Figure 47 shows the amino acid sequence (SEQ ID NO:47) derived from the coding sequence of SEQ ID NO:46 shown in Figure 46.

Figure 48 shows a nucleotide sequence (SEQ ID NO:48) of a native sequence PR026296 cDNA, wherein SEQ ID NO:48 is a clone designated herein as "DNA329001".

Figure 49 shows the amino acid sequence (SEQ ID NO:49) derived from the coding sequence of SEQ ID NO:48 shown in Figure 48.

Figure 50A-B shows a nucleotide sequence (SEQ ID NO:50) of a native sequence PR036766 cDNA, wherein SEQ ID NO:50 is a clone designated herein as "DNA287217".

Figure 51 shows the amino acid sequence (SEQ ID NO:51) derived from the coding sequence of SEQ ID NO:50 shown in Figure 50A-B.

Figure 52 shows a nucleotide sequence (SEQ ID NO:52) of a native sequence PR04912 cDNA, wherein SEQ ID NO:52 is a clone designated herein as "DNA329002".

Figure 53 shows the amino acid sequence (SEQ ID NO:53) derived from the coding sequence of SEQ ID NO:52 shown in Figure 52.

Figure 54 shows a nucleotide sequence (SEQ ID NO:54) of a native sequence PR04769 cDNA, wherein SEQ ID NO:54 is a clone designated herein as "DNA328387".

Figure 55 shows the amino acid sequence (SEQ ID NO:55) derived from the coding sequence of SEQ ID NO:54 shown in Figure 54.

Figure 56 shows a nucleotide sequence (SEQ ID NO:56) of a native sequence PR036899 cDNA, wherein SEQ ID NO:56 is a clone designated herein as "DNA226436".

Figure 57 shows the amino acid sequence (SEQ ID NO:57) derived from the coding sequence of SEQ ID NO:56 shown in Figure 56.

Figure 58 shows a nucleotide sequence (SEQ ID NO:58) of a native sequence PR033667 cDNA, wherein SEQ ID NO:58 is a clone designated herein as "DNA210121".

Figure 59 shows the amino acid sequence (SEQ ID NO:59) derived from the coding sequence of SEQ ID NO:58 shown in Figure 58.

Figure 60 shows a nucleotide sequence (SEQ ID NO:60) of a native sequence PRO37695 cDNA, wherein SEQ ID NO:60 is a clone designated herein as "DNA227232".

Figure 61 shows the amino acid sequence (SEQ ID NO:61) derived from the coding sequence of SEQ ID NO:60 shown in Figure 60.

Figure 62 shows a nucleotide sequence (SEQ ID NO:62) of a native sequence PR038069 cDNA, wherein SEQ ID NO:62 is a clone designated herein as "DNA227606".

Figure 63 shows the amino acid sequence (SEQ ID NO:63) derived from the coding sequence of SEQ ID NO:62 shown in Figure 62.

Figure 64 shows a nucleotide sequence (SEQ ID NO:64) of a native sequence PRO21716 cDNA, wherein SEQ ID NO:64 is a clone designated herein as "DNA188204".

Figure 65 shows the amino acid sequence (SEQ ID NO:65) derived from the coding sequence of SEQ ID NO:64 shown in Figure 64.

Figure 66 shows a nucleotide sequence (SEQ ID NO:66) of a native sequence PR036124 cDNA, wherein SEQ ID NO:66 is a clone designated herein as "DNA225661".

Figure 67 shows the amino acid sequence (SEQ ID NO:67) derived from the coding sequence of SEQ ID NO:66 shown in Figure 66.

Figure 68 shows a nucleotide sequence (SEQ ID NO:68) of a native sequence PR077694 cDNA, wherein SEQ ID NO:68 is a clone designated herein as "DNA329003".

Figure 69 shows the amino acid sequence (SEQ ID NO:69) derived from the coding sequence of SEQ ID NO:68 shown in Figure 68.

Figure 70 shows a nucleotide sequence (SEQ ID NO:70) of a native sequence PRO37957 cDNA, wherein SEQ ID NO:70 is a clone designated herein as "DNA227494".

Figure 71 shows the amino acid sequence (SEQ ID NO:71) derived from the coding sequence of SEQ ID NO:70 shown in Figure 70.

Figure 72A-B shows a nucleotide sequence (SEQ ID NO:72) of a native sequence PR025114 cDNA, wherein SEQ ID NO:72 is a clone designated herein as "DNA196641".

Figure 73 shows the amino acid sequence (SEQ ID NO:73) derived from the coding sequence of SEQ ID NO:72 shown in Figure 72A-B.

Figure 74 shows a nucleotide sequence (SEQ ID NO:74) of a native sequence PR037553 cDNA, wherein SEQ ID NO:74 is a clone designated herein as "DNA227090".

Figure 75 shows the amino acid sequence (SEQ ID NO:75) derived from the coding sequence of SEQ ID NO:74 shown in Figure 74.

Figure 76 shows a nucleotide sequence (SEQ ID NO:76) of a native sequence PR081979 cDNA, wherein SEQ ID NO:76 is a clone designated herein as "DNA329004".

Figure 77 shows the amino acid sequence (SEQ ID NO:77) derived from the coding sequence of SEQ ID NO:76 shown in Figure 76.

Figure 78 shows a nucleotide sequence (SEQ ID NO:78) of a native sequence PR06013 cDNA, wherein SEQ ID NO:78 is a clone designated herein as "DNA304828".

Figure 79 shows the amino acid sequence (SEQ ID NO:79) derived from the coding sequence of SEQ ID NO:79 shown in Figure 78.

Figure 80 shows a nucleotide sequence (SEQ ID NO:80) of a native sequence PR021960 cDNA, wherein SEQ ID NO:80 is a clone designated herein as "DNA192060".

Figure 81 shows the amino acid sequence (SEQ ID NO:81) derived from the coding sequence of SEQ ID NO:80 shown in Figure 80.

Figure 82 shows a nucleotide sequence (SEQ ID NO:82) of a native sequence PRO34276 cDNA, wherein SEQ ID NO:82 is a clone designated herein as "DNA216689".

Figure 83 shows the amino acid sequence (SEQ ID NO:83) derived from the coding sequence of SEQ ID NO:82 shown in Figure 82.

Figure 84 shows a nucleotide sequence (SEQ ID NO:84) of a native sequence PRO1717 cDNA, wherein SEQ ID NO:84 is a clone designated herein as "DNA82342".

Figure 85 shows the amino acid sequence (SEQ ID NO:85) derived from the coding sequence of SEQ ID NO:84 shown in Figure 84.

Figure 86 shows a nucleotide sequence (SEQ ID NO:86) of a native sequence PR034107 cDNA, wherein SEQ ID NO:86 is a clone designated herein as "DNA199788".

Figure 87 shows the amino acid sequence (SEQ ID NO:87) derived from the coding sequence of SEQ ID NO:86 shown in Figure 86.

Figure 88 shows a nucleotide sequence (SEQ ID NO:88) of a native sequence PRO12612 cDNA, wherein SEQ ID NO:88 is a clone designated herein as "DNA329005".

Figure 89 shows the amino acid sequence (SEQ ID NO:89) derived from the coding sequence of SEQ ID NO:88 shown in Figure 88.

Figure 90 shows a nucleotide sequence (SEQ ID NO:90) of a native sequence PR037946 cDNA, wherein SEQ ID NO:90 is a clone designated herein as "DNA227483".

Figure 91 shows the amino acid sequence (SEQ ID NO:91) derived from the coding sequence of SEQ ID NO:90 shown in Figure 90.

Figure 92 shows a nucleotide sequence (SEQ ID NO:92) of a native sequence PRO12865 cDNA, wherein SEQ ID NO:92 is a clone designated herein as "DNA329006".

Figure 93 shows the amino acid sequence (SEQ ID NO:93) derived from the coding sequence of SEQ ID NO:92 shown in Figure 92.

Figure 94A-B shows a nucleotide sequence (SEQ ID NO:94) of a native sequence PR037029 cDNA, wherein SEQ ID NO:94 is a clone designated herein as "DNA329007".

Figure 95 shows the amino acid sequence (SEQ ID NO:95) derived from the coding sequence of SEQ ID NO:94 shown in Figure 94A-B.

Figure 96 shows a nucleotide sequence (SEQ ID NO:96) of a native sequence PR038337 cDNA, wherein SEQ ID NO:96 is a clone designated herein as "DNA227874".

Figure 97 shows the amino acid sequence (SEQ ID NO:97) derived from the coding sequence of SEQ ID NO:96 shown in Figure 96.

Figure 98 shows a nucleotide sequence (SEQ ID NO:98) of a native sequence PR04710 cDNA, wherein SEQ ID NO:98 is a clone designated herein as "DNA103380".

Figure 99 shows the amino acid sequence (SEQ ID NO:99) derived from the coding sequence of SEQ ID NO:98 shown in Figure 98.

Figure 100 shows a nucleotide sequence (SEQ ID NO:100) of a native sequence PRO12570 cDNA, wherein SEQ ID NO:100 is a clone designated herein as "DNA 150990".

Figure 101 shows the amino acid sequence (SEQ ID NO:101) derived from the coding sequence of SEQ ID NO: 100 shown in Figure 100.

Figure 102 shows a nucleotide sequence (SEQ ID NO:102) of a native sequence PRO12890 cDNA, wherein SEQ ID NO:102 is a clone designated herein as "DNA151802".

Figure 103 shows the amino acid sequence (SEQ ID NO:103) derived from the coding sequence of SEQ ID NO: 102 shown in Figure 102.

Figure 104 shows a nucleotide sequence (SEQ ID NO:104) of a native sequence PRO37121 cDNA, wherein SEQ ID NO:104 is a clone designated herein as "DNA226658".

Figure 105 shows the amino acid sequence (SEQ ID NO:105) derived from the coding sequence of SEQ ID NO: 104 shown in Figure 104.

Figure 106 shows a nucleotide sequence (SEQ ID NO:106) of a native sequence PRO3813 cDNA, wherein SEQ ID NO:106 is a clone designated herein as "DNA196579".

Figure 107 shows the amino acid sequence (SEQ ID NO:107) derived from the coding sequence of SEQ ID NO: 106 shown in Figure 106.

Figure 108 shows a nucleotide sequence (SEQ ID NO:108) of a native sequence PR024934 cDNA, wherein SEQ ID NO:108 is a clone designated herein as "DNA196439".

Figure 109 shows the amino acid sequence (SEQ ID NO:109) derived from the coding sequence of SEQ ID NO: 108 shown in Figure 108.

Figure 110A-B shows a nucleotide sequence (SEQ ID NO:110) of a native sequence PRO12458 cDNA, wherein SEQ ID NO:110 is a clone designated herein as "DNA150765".

Figure 111 shows the amino acid sequence (SEQ ID NO:111) derived from the coding sequence of SEQ ID NO: 110 shown in Figure 110A-B.

Figure 112 shows a nucleotide sequence (SEQ ID NO:112) of a native sequence PR037843 cDNA, wherein SEQ ID NO:112 is a clone designated herein as "DNA328570".

Figure 113 shows the amino acid sequence (SEQ ID NO:113) derived from the coding sequence of SEQ ID NO: 112 shown in Figure 112.

Figure 114 A-B shows a nucleotide sequence (SEQ ID NO:114) of a native sequence PR037547 cDNA, wherein SEQ ID NO:114 is a clone designated herein as "DNA227084".

Figure 115 shows the amino acid sequence (SEQ ID NO:115) derived from the coding sequence of SEQ ID NO: 114 shown in Figure 114A-B.

Figure 116 shows a nucleotide sequence (SEQ ID NO:116) of a native sequence PR036300 cDNA, wherein SEQ ID NO:116 is a clone designated herein as "DNA225837".

Figure 117 shows the amino acid sequence (SEQ ID NO:117) derived from the coding sequence of SEQ ID NO: 116 shown in Figure 116.

Figure 118 shows a nucleotide sequence (SEQ ID NO:118) of a native sequence PRO37192 cDNA, wherein SEQ ID NO:118 is a clone designated herein as "DNA226729".

Figure 119 shows the amino acid sequence (SEQ ID NO:119) derived from the coding sequence of SEQ ID NO:

118 shown in Figure 118.

Figure 120 shows a nucleotide sequence (SEQ ID NO:120) of a native sequence PRO12832 cDNA, wherein SEQ ID NO:120 is a clone designated herein as "DNA329008".

Figure 121 shows the amino acid sequence (SEQ ID NO:121) derived from the coding sequence of SEQ ID NO: 120 shown in Figure 120.

Figure 122A-B shows a nucleotide sequence (SEQ ID NO:122) of a native sequence PRO25147 cDNA, wherein SEQ ID NO:122 is a clone designated herein as "DNA196681".

Figure 123 shows the amino acid sequence (SEQ ID NO:123) derived from the coding sequence of SEQ ID NO: 122 shown in Figure 122A-B.

Figure 124A-B shows a nucleotide sequence (SEQ ID NO:124) of a native sequence PRO12876 cDNA, wherein SEQ ID NO:124 is a clone designated herein as "DNA151420".

Figure 125 shows the amino acid sequence (SEQ ID NO:125) derived from the coding sequence of SEQ ID NO: 124 shown in Figure 124A-B.

Figure 126 shows a nucleotide sequence (SEQ ID NO:126) of a native sequence PRO12155 cDNA, wherein SEQ ID NO:126 is a clone designated herein as "DNA150935".

Figure 127 shows the amino acid sequence (SEQ ID NO:127) derived from the coding sequence of SEQ ID NO: 126 shown in Figure 126.

Figure 128A-B shows a nucleotide sequence (SEQ ID NO:128) of a native sequence PRO12370 cDNA, wherein SEQ ID NO:128 is a clone designated herein as "DNA150614".

Figure 129 shows the amino acid sequence (SEQ ID NO:129) derived from the coding sequence of SEQ ID NO: 128 shown in Figure 128A-B.

Figure 130 shows a nucleotide sequence (SEQ ID NO:130) of a native sequence PRO12320 cDNA, wherein SEQ ID NO:130 is a clone designated herein as "DNA329009".

Figure 131 shows the amino acid sequence (SEQ ID NO:131) derived from the coding sequence of SEQ ID NO: 130 shown in Figure 130.

Figure 132 shows a nucleotide sequence (SEQ ID NO:132) of a native sequence PR023370 cDNA, wherein SEQ ID NO:132 is a clone designated herein as "DNA329010".

Figure 133 shows the amino acid sequence (SEQ ID NO:133) derived from the coding sequence of SEQ ID NO: 132 shown in Figure 132.

Figure 134 shows a nucleotide sequence (SEQ ID NO:134) of a native sequence PR025266 cDNA, wherein SEQ ID NO:134 is a clone designated herein as "DNA196825".

Figure 135 shows the amino acid sequence (SEQ ID NO:135) derived from the coding sequence of SEQ ID NO: 134 shown in Figure 134.

Figure 136 shows a nucleotide sequence (SEQ ID NO:136) of a native sequence PR04785 cDNA, wherein SEQ ID NO:136 is a clone designated herein as "DNA329011".

Figure 137 shows the amino acid sequence (SEQ ID NO:137) derived from the coding sequence of SEQ ID NO: 136 shown in Figure 136.

Figure 138 shows a nucleotide sequence (SEQ ID NO:138) of a native sequence PRO4660 cDNA, wherein SEQ ID NO:138 is a clone designated herein as "DNA329012".

Figure 139 shows the amino acid sequence (SEQ ID NO:139) derived from the coding sequence of SEQ ID NO: 138 shown in Figure 138.

Figure 140 shows a nucleotide sequence (SEQ ID NO:140) of a native sequence PR071095 cDNA, wherein SEQ ID NO:140 is a clone designated herein as "DNA340668".

Figure 141 shows the amino acid sequence (SEQ ID NO:141) derived from the coding sequence of SEQ ID NO: 140 shown in Figure 140.

Figure 142 shows a nucleotide sequence (SEQ ID NO:142) of a native sequence PR04658 cDNA, wherein SEQ ID NO:142 is a clone designated herein as "DNA103328".

Figure 143 shows the amino acid sequence (SEQ ID NO:143) derived from the coding sequence of SEQ ID NO: 142 shown in Figure 142.

Figure 144A-B shows a nucleotide sequence (SEQ ID NO:144) of a native sequence PR02757 cDNA, wherein SEQ ID NO:144 is a clone designated herein as "DNA88348".

Figure 145 shows the amino acid sequence (SEQ ID NO:145) derived from the coding sequence of SEQ ID NO: 112 shown in Figure 112.

Figure 146 shows a nucleotide sequence (SEQ ID NO:146) of a native sequence PRO12789 cDNA, wherein SEQ ID NO:146 is a clone designated herein as "DNA150710".

Figure 147 shows the amino acid sequence (SEQ ID NO:147) derived from the coding sequence of SEQ ID NO: 146 shown in Figure 146.

Figure 148 shows a nucleotide sequence (SEQ ID NO:148) of a native sequence PR020128 cDNA, wherein SEQ ID NO:148 is a clone designated herein as "DNA329013".

Figure 149 shows the amino acid sequence (SEQ ID NO:149) derived from the coding sequence of SEQ ID NO: 148 shown in Figure 148.

Figure 150 shows a nucleotide sequence (SEQ ID NO:150) of a native sequence PR02834 cDNA, wherein SEQ ID NO:150 is a clone designated herein as "DNA88541".

Figure 151 shows the amino acid sequence (SEQ ID NO:151) derived from the coding sequence of SEQ ID NO: 150 shown in Figure 150.

Figure 152 shows a nucleotide sequence (SEQ ID NO:152) of a native sequence PR09998 cDNA, wherein SEQ ID NO:152 is a clone designated herein as "DNA329014".

Figure 153 shows the amino acid sequence (SEQ ID NO:153) derived from the coding sequence of SEQ ID NO: 152 shown in Figure 152.

Figure 154 shows a nucleotide sequence (SEQ ID NO:154) of a native sequence PRO84691 cDNA, wherein SEQ ID NO:154 is a clone designated herein as "DNA329015".

Figure 155 shows the amino acid sequence (SEQ ID NO:155) derived from the coding sequence of SEQ ID NO: 154 shown in Figure 154.

Figure 156A-B shows a nucleotide sequence (SEQ ID NO:156) of a native sequence PRO12904 cDNA, wherein SEQ ID NO:156 is a clone designated herein as "DNA151841".

Figure 157 shows the amino acid sequence (SEQ ID NO:157) derived from the coding sequence of SEQ ID NO: 156 shown in Figure 156A-B.

Figure 158 shows a nucleotide sequence (SEQ ID NO:158) of a native sequence PR04887 cDNA, wherein SEQ ID NO:158 is a clone designated herein as "DNA329016".

Figure 159 shows the amino acid sequence (SEQ ID NO:159) derived from the coding sequence of SEQ ID NO: 158 shown in Figure 158.

Figure 160 shows a nucleotide sequence (SEQ ID NO:160) of a native sequence PRO12082 cDNA, wherein SEQ ID NO:160 is a clone designated herein as "DNA150713".

Figure 161 shows the amino acid sequence (SEQ ID NO:161) derived from the coding sequence of SEQ ID NO: 160 shown in Figure 160.

Figure 162 shows a nucleotide sequence (SEQ ID NO:162) of a native sequence PR037975 cDNA, wherein SEQ ID NO:162 is a clone designated herein as "DNA227512".

Figure 163 shows the amino acid sequence (SEQ ID NO:163) derived from the coding sequence of SEQ ID NO: 162 shown in Figure 162.

Figure 164 shows a nucleotide sequence (SEQ ID NO:164) of a native sequence PRO37653 cDNA, wherein SEQ ID NO: 164 is a clone designated herein as "DNA227190".

Figure 165 shows the amino acid sequence (SEQ ID NO:165) derived from the coding sequence of SEQ ID NO: 164 shown in Figure 164.

Figure 166A-B shows a nucleotide sequence (SEQ ID NO:166) of a native sequence PR04776 cDNA, wherein SEQ ID NO:166 is a clone designated herein as "DNA103449".

Figure 167 shows the amino acid sequence (SEQ ID NO:167) derived from the coding sequence of SEQ ID NO: 166 shown in Figure 166A-B.

Figure 168A-B shows a nucleotide sequence (SEQ ID NO:168) of a native sequence PRO12073 cDNA, wherein SEQ ID NO:168 is a clone designated herein as "DNA150498".

Figure 169 shows the amino acid sequence (SEQ ID NO:169) derived from the coding sequence of SEQ ID NO: 168 shown in Figure 168A-B.

Figure 170 shows a nucleotide sequence (SEQ ID NO:170) of a native sequence PRO38457 cDNA, wherein SEQ ID NO:170 is a clone designated herein as "DNA227994".

Figure 171 shows the amino acid sequence (SEQ ID NO:171) derived from the coding sequence of SEQ ID NO: 170 shown in Figure 170.

Figure 172 shows a nucleotide sequence (SEQ ID NO:172) of a native sequence PR04767 cDNA, wherein SEQ ID NO:172 is a clone designated herein as "DNA103440".

Figure 173 shows the amino acid sequence (SEQ ID NO:173) derived from the coding sequence of SEQ ID NO: 172 shown in Figure 172.

Figure 174A-B shows a nucleotide sequence (SEQ ID NO:174) of a native sequence PRO12884 cDNA, wherein SEQ ID NO:174 is a clone designated herein as "DNA151707".

Figure 175 shows the amino acid sequence (SEQ ID NO:175) derived from the coding sequence of SEQ ID NO: 174 shown in Figure 174A-B.

Figure 176 shows a nucleotide sequence (SEQ ID NO:176) of a native sequence PR012586 cDNA, wherein SEQ ID NO:176 is a clone designated herein as "DNA151037".

Figure 177 shows the amino acid sequence (SEQ ID NO:177) derived from the coding sequence of SEQ ID NO:

176 shown in Figure 176.

Figure 178A-B shows a nucleotide sequence (SEQ ID NO:178) of a native sequence PR084692 cDNA, wherein SEQ ID NO:178 is a clone designated herein as "DNA329017".

Figure 179 shows the amino acid sequence (SEQ ID NO:179) derived from the coding sequence of SEQ ID NO: 178 shown in Figure 178A-B.

Figure 180 shows a nucleotide sequence (SEQ ID NO:180) of a native sequence PRO12280 cDNA, wherein SEQ ID NO:180 is a clone designated herein as "DNA150478".

Figure 181 shows the amino acid sequence (SEQ ID NO:181) derived from the coding sequence of SEQ ID NO: 180 shown in Figure 180.

Figure 182 shows a nucleotide sequence (SEQ ID NO:182) of a native sequence PRO23859 cDNA, wherein SEQ ID NO:182 is a clone designated herein as "DNA328957".

Figure 183 shows the amino acid sequence (SEQ ID NO:183) derived from the coding sequence of SEQ ID NO: 182 shown in Figure 182.

Figure 184 shows a nucleotide sequence (SEQ ID NO:184) of a native sequence PRO2577 cDNA, wherein SEQ ID NO:184 is a clone designated herein as "DNA328542".

Figure 185 shows the amino acid sequence (SEQ ID NO:185) derived from the coding sequence of SEQ ID NO: 184 shown in Figure 184.

Figure 186 shows a nucleotide sequence (SEQ ID NO:186) of a native sequence PRO37696 cDNA, wherein SEQ ID NO:186 is a clone designated herein as "DNA227233".

Figure 187 shows the amino acid sequence (SEQ ID NO:187) derived from the coding sequence of SEQ ID NO: 186 shown in Figure 186.

Figure 188 shows a nucleotide sequence (SEQ ID NO:188) of a native sequence PRO24075 cDNA, wherein SEQ ID NO:188 is a clone designated herein as "DNA194805".

Figure 189 shows the amino acid sequence (SEQ ID NO:189) derived from the coding sequence of SEQ ID NO: 188 shown in Figure 188.

Figure 190A-B shows a nucleotide sequence (SEQ ID NO:190) of a native sequence PRO71042 cDNA, wherein SEQ ID NO:190 is a clone designated herein as "DNA304464".

Figure 191 shows the amino acid sequence (SEQ ID NO:191) derived from the coding sequence of SEQ ID NO: 190 shown in Figure 190A-B.

Figure 192A-B shows a nucleotide sequence (SEQ ID NO:192) of a native sequence PRO37639 cDNA, wherein SEQ ID NO:192 is a clone designated herein as "DNA227176".

Figure 193 shows the amino acid sequence (SEQ ID NO:193) derived from the coding sequence of SEQ ID NO: 192 shown in Figure 192A-B.

Figure 194 shows a nucleotide sequence (SEQ ID NO:194) of a native sequence PR084693 cDNA, wherein SEQ ID NO:192 is a clone designated herein as "DNA329018".

Figure 195 shows the amino acid sequence (SEQ ID NO:195) derived from the coding sequence of SEQ ID NO: 194 shown in Figure 194.

Figure 196 shows a nucleotide sequence (SEQ ID NO:196) of a native sequence PRO37272 cDNA, wherein SEQ ID NO:196 is a clone designated herein as "DNA226809".

Figure 197 shows the amino acid sequence (SEQ ID NO:197) derived from the coding sequence of SEQ ID NO: 196 shown in Figure 196.

Figure 198 shows a nucleotide sequence (SEQ ID NO:198) of a native sequence PRO84694 cDNA, wherein SEQ ID NO:198 is a clone designated herein as "DNA329019".

Figure 199 shows the amino acid sequence (SEQ ID NO:199) derived from the coding sequence of SEQ ID NO: 198 shown in Figure 198.

Figure 200A-B shows a nucleotide sequence (SEQ ID NO:200) of a native sequence PR04330 cDNA, wherein SEQ ID NO:200 is a clone designated herein as "DNA328454".

Figure 201 shows the amino acid sequence (SEQ ID NO:201) derived from the coding sequence of SEQ ID NO: 200 shown in Figure 200A-B.

Figure 202 shows a nucleotide sequence (SEQ ID NO:202) of a native sequence PR084695 cDNA, wherein SEQ ID NO:202 is a clone designated herein as "DNA329020".

Figure 203 shows the amino acid sequence (SEQ ID NO:203) derived from the coding sequence of SEQ ID NO: 202 shown in Figure 202.

Figure 204 shows a nucleotide sequence (SEQ ID NO:204) of a native sequence PR0285 cDNA, wherein SEQ ID NO:204 is a clone designated herein as "DNA329021".

Figure 205 shows the amino acid sequence (SEQ ID NO:205) derived from the coding sequence of SEQ ID NO: 204 shown in Figure 204.

Figure 206 shows a nucleotide sequence (SEQ ID NO:206) of a native sequence PRO38310 cDNA, wherein SEQ ID NO:206 is a clone designated herein as "DNA329022".

Figure 207 shows the amino acid sequence (SEQ ID NO:207) derived from the coding sequence of SEQ ID NO: 206 shown in Figure 206.

Figure 208 shows a nucleotide sequence (SEQ ID NO:208) of a native sequence PR037657 cDNA, wherein SEQ ID NO:208 is a clone designated herein as "DNA227194".

Figure 209 shows the amino acid sequence (SEQ ID NO:209) derived from the coding sequence of SEQ ID NO: 208 shown in Figure 208.

Figure 210 shows a nucleotide sequence (SEQ ID NO:210) of a native sequence PRO71061 cDNA, wherein SEQ ID NO:210 is a clone designated herein as "DNA304494".

Figure 211 shows the amino acid sequence (SEQ ID NO:211) derived from the coding sequence of SEQ ID NO: 210 shown in Figure 210.

Figure 212 shows a nucleotide sequence (SEQ ID NO:212) of a native sequence PR0209 cDNA, wherein SEQ ID NO:212 is a clone designated herein as "DNA329023".

Figure 213 shows the amino acid sequence (SEQ ID NO:213) derived from the coding sequence of SEQ ID NO: 212 shown in Figure 212.

Figure 214 shows a nucleotide sequence (SEQ ID NO:214) of a native sequence PRO37584 cDNA, wherein SEQ ID NO:214 is a clone designated herein as "DNA227121".

Figure 215 shows the amino acid sequence (SEQ ID NO:215 derived from the coding sequence of SEQ ID NO:214 shown in Figure 214.

Figure 216A-B shows a nucleotide sequence (SEQ ID NO:216) of a native sequence PR084696 cDNA, wherein SEQ ID NO:216 is a clone designated herein as "DNA329024".

Figure 217 shows the amino acid sequence (SEQ ID NO:217) derived from the coding sequence of SEQ ID NO: 216 shown in Figure 216A-B.

Figure 218 shows a nucleotide sequence (SEQ ID NO:218) of a native sequence PRO4860 cDNA, wherein SEQ ID NO:218 is a clone designated herein as "DNA329025".

Figure 219 shows the amino acid sequence (SEQ ID NO:219) derived from the coding sequence of SEQ ID NO: 218 shown in Figure 218.

Figure 220A-B shows a nucleotide sequence (SEQ ID NO:220) of a native sequence PRO38492 cDNA, wherein SEQ ID NO:172 is a clone designated herein as "DNA228029".

Figure 221 shows the amino acid sequence (SEQ ID NO:221) derived from the coding sequence of SEQ ID NO: 220 shown in Figure 220A-B.

Figure 222 shows a nucleotide sequence (SEQ ID NO:222) of a native sequence cDNA, wherein SEQ ID NO:222 is a clone designated herein as "DNA150980".

Figure 223 shows a nucleotide sequence (SEQ ID NO:223) of a native sequence cDNA, wherein SEQ ID NO:223 is a clone designated herein as "DNA329026".

Figure 224 shows a nucleotide sequence (SEQ ID NO:224) of a native sequence PRO11738 cDNA, wherein SEQ ID NO:224 is a clone designated herein as "DNA 151360".

Figure 225 shows the amino acid sequence (SEQ ID NO:225) derived from the coding sequence of SEQ ID NO: 224 shown in Figure 224.

Figure 226 shows a nucleotide sequence (SEQ ID NO:226) of a native sequence PR069476 cDNA, wherein SEQ ID NO:226 is a clone designated herein as "DNA287190".

Figure 227 shows the amino acid sequence (SEQ ID NO:227) derived from the coding sequence of SEQ ID NO: 226 shown in Figure 226.

Figure 228 shows a nucleotide sequence (SEQ ID NO:228) of a native sequence PR012032 cDNA, wherein SEQ ID NO:228 is a clone designated herein as "DNA151744".

Figure 229 shows the amino acid sequence (SEQ ID NO:229) derived from the coding sequence of SEQ ID NO: 228 shown in Figure 228.

Figure 230 shows a nucleotide sequence (SEQ ID NO:230) of a native sequence cDNA, wherein SEQ ID NO:230 is a clone designated herein as "DNA161393".

Figure 231 shows a nucleotide sequence (SEQ ID NO:231) of a native sequence PRO69484 cDNA, wherein SEQ ID NO:231 is a clone designated herein as "DNA287198".

Figure 232 shows the amino acid sequence (SEQ ID NO:232) derived from the coding sequence of SEQ ID NO: 231 shown in Figure 231.

Figure 233 shows a nucleotide sequence (SEQ ID NO:233) of a native sequence cDNA, wherein SEQ ID NO:233 is a clone designated herein as "DNA274998".

Figure 234 shows a nucleotide sequence (SEQ ID NO:234) of a native sequence PRO23460 cDNA, wherein SEQ ID NO:234 is a clone designated herein as "DNA194063".

Figure 235 shows the amino acid sequence (SEQ ID NO:235) derived from the coding sequence of SEQ ID NO:

234 shown in Figure 234.

Figure 236A-B shows a nucleotide sequence (SEQ ID NO:236) of a native sequence PRO84476 cDNA, wherein SEQ ID NO:236 is a clone designated herein as "DNA328720".

Figure 237 shows the amino acid sequence (SEQ ID NO:237) derived from the coding sequence of SEQ ID NO: 236 shown in Figure 236A-B.

Figure 238 shows a nucleotide sequence (SEQ ID NO:238) of a native sequence PRO84697 cDNA, wherein SEQ ID NO:238 is a clone designated herein as "DNA329027".

Figure 239 shows the amino acid sequence (SEQ ID NO:239) derived from the coding sequence of SEQ ID NO: 238 shown in Figure 238.

Figure 240 shows a nucleotide sequence (SEQ ID NO:240) of a native sequence cDNA, wherein SEQ ID NO:240 is a clone designated herein as "DNA329028".

Figure 241 shows a nucleotide sequence (SEQ ID NO:241) of a native sequence cDNA, wherein SEQ ID NO:241 is a clone designated herein as "DNA329029".

Figure 242 shows a nucleotide sequence (SEQ ID NO:242) of a native sequence PRO71207 cDNA, wherein SEQ ID NO:242 is a clone designated herein as "DNA304795".

Figure 243 shows the amino acid sequence (SEQ ID NO:243) derived from the coding sequence of SEQ ID NO: 242 shown in Figure 242.

Figure 244 shows a nucleotide sequence (SEQ ID NO:244) of a native sequence cDNA, wherein SEQ ID NO:244 is a clone designated herein as "DNA323696".

Figure 245 shows a nucleotide sequence (SEQ ID NO:245) of a native sequence PRO28714 cDNA, wherein SEQ ID NO:245 is a clone designated herein as "DNA188014".

Figure 246 shows the amino acid sequence (SEQ ID NO:246) derived from the coding sequence of SEQ ID NO: 245 shown in Figure 245.

Figure 247A-B shows a nucleotide sequence (SEQ ID NO:247) of a native sequence PR084698 cDNA, wherein SEQ ID NO:247 is a clone designated herein as "DNA329030".

Figure 248 shows the amino acid sequence (SEQ ID NO:248) derived from the coding sequence of SEQ ID NO: 247 shown in Figure 247A-B.

Figure 249 shows a nucleotide sequence (SEQ ID NO:249) of a native sequence PR049839 cDNA, wherein SEQ ID NO:249 is a clone designated herein as "DNA254741".

Figure 250 shows the amino acid sequence (SEQ ID NO:250) derived from the coding sequence of SEQ ID NO: 249 shown in Figure 249.

Figure 251 shows a nucleotide sequence (SEQ ID NO:251) of a native sequence PRO84441 cDNA, wherein SEQ ID NO:251 is a clone designated herein as "DNA328669".

Figure 252 shows the amino acid sequence (SEQ ID NO:252) derived from the coding sequence of SEQ ID NO: 251 shown in Figure 251.

Figure 253 shows a nucleotide sequence (SEQ ID NO:253) of a native sequence PRO49214 cDNA, wherein SEQ ID NO:253 is a clone designated herein as "DNA253811".

Figure 254 shows the amino acid sequence (SEQ ID NO:254) derived from the coding sequence of SEQ ID NO: 253 shown in Figure 253.

Figure 255 shows a nucleotide sequence (SEQ ID NO:255) of a native sequence PR050523 cDNA, wherein SEQ ID NO:255 is a clone designated herein as "DNA255456".

Figure 256 shows the amino acid sequence (SEQ ID NO:256) derived from the coding sequence of SEQ ID NO: 255 shown in Figure 255.

Figure 257 shows a nucleotide sequence (SEQ ID NO:257) of a native sequence PRO50241 cDNA, wherein SEQ ID NO:257 is a clone designated herein as "DNA255161".

Figure 258 shows the amino acid sequence (SEQ ID NO:258) derived from the coding sequence of SEQ ID NO: 257 shown in Figure 257.

Figure 259 shows a nucleotide sequence (SEQ ID NO:259) of a native sequence PRO83773 cDNA, wherein SEQ ID NO:259 is a clone designated herein as "DNA327812".

Figure 260 shows the amino acid sequence (SEQ ID NO:260) derived from the coding sequence of SEQ ID NO: 259 shown in Figure 259.

Figure 261 shows a nucleotide sequence (SEQ ID NO:261) of a native sequence PRO84699 cDNA, wherein SEQ ID NO:261 is a clone designated herein as "DNA329031".

Figure 262 shows the amino acid sequence (SEQ ID NO:262) derived from the coding sequence of SEQ ID NO: 261 shown in Figure 261.

Figure 263 shows a nucleotide sequence (SEQ ID NO:263) of a native sequence PR050772 cDNA, wherein SEQ ID NO:263 is a clone designated herein as "DNA329032".

Figure 264 shows the amino acid sequence (SEQ ID NO:264) derived from the coding sequence of SEQ ID NO:

263 shown in Figure 263.

Figure 265 shows a nucleotide sequence (SEQ ID NO:265) of a native sequence PR049326 cDNA, wherein SEQ ID NO:265 is a clone designated herein as "DNA254214".

Figure 266 shows the amino acid sequence (SEQ ID NO:266) derived from the coding sequence of SEQ ID NO: 265 shown in Figure 265.

Figure 267 shows a nucleotide sequence (SEQ ID NO:267) of a native sequence PR049824 cDNA, wherein SEQ ID NO:267 is a clone designated herein as "DNA254725".

Figure 268 shows the amino acid sequence (SEQ ID NO:268) derived from the coding sequence of SEQ ID NO: 267 shown in Figure 267.

Figure 269 shows a nucleotide sequence (SEQ ID NO:269) of a native sequence PR070333 cDNA, wherein SEQ ID NO:269 is a clone designated herein as "DNA290234".

Figure 270 shows the amino acid sequence (SEQ ID NO:270) derived from the coding sequence of SEQ ID NO: 269 shown in Figure 269.

Figure 271 shows a nucleotide sequence (SEQ ID NO:271) of a native sequence PRO83673 cDNA, wherein SEQ ID NO:271 is a clone designated herein as "DNA327690".

Figure 272 shows the amino acid sequence (SEQ ID NO:272) derived from the coding sequence of SEQ ID NO: 271 shown in Figure 271.

Figure 273 shows a nucleotide sequence (SEQ ID NO:273) of a native sequence PR050332 cDNA, wherein SEQ ID NO:273 is a clone designated herein as "DNA255255".

Figure 274 shows the amino acid sequence (SEQ ID NO:274) derived from the coding sequence of SEQ ID NO: 273 shown in Figure 273.

Figure 275 shows a nucleotide sequence (SEQ ID NO:275) of a native sequence PRO51295 cDNA, wherein SEQ ID NO:275 is a clone designated herein as "DNA256251".

Figure 276 shows the amino acid sequence (SEQ ID NO:276) derived from the coding sequence of SEQ ID NO: 275 shown in Figure 275.

Figure 277 shows a nucleotide sequence (SEQ ID NO:277) of a native sequence PRO51621 cDNA, wherein SEQ ID NO:277 is a clone designated herein as "DNA256637".

Figure 278 shows the amino acid sequence (SEQ ID NO:278) derived from the coding sequence of SEQ ID NO: 277 shown in Figure 277.

Figure 279 shows a nucleotide sequence (SEQ ID NO:279) of a native sequence PRO34958 cDNA, wherein SEQ ID NO:279 is a clone designated herein as "DNA210497".

Figure 280 shows the amino acid sequence (SEQ ID NO:280) derived from the coding sequence of SEQ ID NO: 279 shown in Figure 279.

Figure 281 shows a nucleotide sequence (SEQ ID NO:281) of a native sequence PR050821 cDNA, wherein SEQ ID NO:281 is a clone designated herein as "DNA255766".

Figure 282 shows the amino acid sequence (SEQ ID NO:282) derived from the coding sequence of SEQ ID NO: 281 shown in Figure 281.

Figure 283 shows a nucleotide sequence (SEQ ID NO:283) of a native sequence PRO84700 cDNA, wherein SEQ ID NO:283 is a clone designated herein as "DNA329033".

Figure 284 shows the amino acid sequence (SEQ ID NO:284) derived from the coding sequence of SEQ ID NO: 283 shown in Figure 283.

Figure 285 shows a nucleotide sequence (SEQ ID NO:285) of a native sequence PRO84701 cDNA, wherein SEQ ID NO:285 is a clone designated herein as "DNA329043".

Figure 286 shows the amino acid sequence (SEQ ID NO:286) derived from the coding sequence of SEQ ID NO: 285 shown in Figure 285.

Figure 287 shows a nucleotide sequence (SEQ ID NO:287) of a native sequence PRO60333 cDNA, wherein SEQ ID NO:287 is a clone designated herein as "DNA272062".

Figure 288 shows the amino acid sequence (SEQ ID NO:288) derived from the coding, sequence of SEQ ID NO: 287 shown in Figure 287.

Figure 289A-B shows a nucleotide sequence (SEQ ID NO:289) of a native sequence PR050187 cDNA, wherein SEQ ID NO:289 is a clone designated herein as "DNA255105".

Figure 290 shows the amino acid sequence (SEQ ID NO:290) derived from the coding sequence of SEQ ID NO: 289 shown in Figure 289A-B.

Figure 291 shows a nucleotide sequence (SEQ ID NO:291) of a native sequence PRO48357 cDNA, wherein SEQ ID NO:291 is a clone designated herein as "DNA252367".

Figure 292 shows the amino acid sequence (SEQ ID NO:292) derived from the coding sequence of SEQ ID NO: 291 shown in Figure 291.

Figure 293A-B shows a nucleotide sequence (SEQ ID NO:293) of a native sequence PRO50365 cDNA, wherein SEQ ID NO:293 is a clone designated herein as "DNA255292".

Figure 294 shows the amino acid sequence (SEQ ID NO:294) derived from the coding sequence of SEQ ID NO: 293 shown in Figure 293A-B.

Figure 295 shows a nucleotide sequence (SEQ ID NO:295) of a native sequence PRO84702 cDNA, wherein SEQ ID NO:295 is a clone designated herein as "DNA329035".

Figure 296 shows the amino acid sequence (SEQ ID NO:296) derived from the coding sequence of SEQ ID NO: 295 shown in Figure 295.

Figure 297 shows a nucleotide sequence (SEQ ID NO:297) of a native sequence PR049810 cDNA, wherein SEQ ID NO:297 is a clone designated herein as "DNA254710".

Figure 298 shows the amino acid sequence (SEQ ID NO:298) derived from the coding sequence of SEQ ID NO: 297 shown in Figure 297.

Figure 299A-B shows a nucleotide sequence (SEQ ID NO:299) of a native sequence PRO5871 cDNA, wherein SEQ ID NO:299 is a clone designated herein as "DNA270323".

Figure 300 shows the amino acid sequence (SEQ ID NO:300) derived from the coding sequence of SEQ ID NO: 299 shown in Figure 299A-B.

Figure 301 shows a nucleotide sequence (SEQ ID NO:301) of a native sequence PR069503 cDNA, wherein SEQ ID NO:301 is a clone designated herein as "DNA287224".

Figure 302 shows the amino acid sequence (SEQ ID NO:302) derived from the coding sequence of SEQ ID NO: 301 shown in Figure 301.

Figure 303A-B shows a nucleotide sequence (SEQ ID NO:303) of a native sequence PR049564 cDNA, wherein SEQ ID NO:303 is a clone designated herein as "DNA254455".

Figure 304 shows the amino acid sequence (SEQ ID NO:304) derived from the coding sequence of SEQ ID NO: 303 shown in Figure 303A-B.

Figure 305A-B shows a nucleotide sequence (SEQ ID NO:305) of a native sequence PR084703 cDNA, wherein SEQ ID NO:305 is a clone designated herein as "DNA329036".

Figure 306 shows the amino acid sequence (SEQ ID NO:306) derived from the coding sequence of SEQ ID NO: 305 shown in Figure 305A-B.

Figure 307 shows a nucleotide sequence (SEQ ID NO:307) of a native sequence PR084663 cDNA, wherein SEQ ID NO:307 is a clone designated herein as "DNA328952".

Figure 308 shows the amino acid sequence (SEQ ID NO:308) derived from the coding sequence of SEQ ID NO: 307 shown in Figure 307.

Figure 309 shows a nucleotide sequence (SEQ ID NO:309) of a native sequence PRO83800 cDNA, wherein SEQ ID NO:309 is a clone designated herein as "DNA327858".

Figure 310 shows the amino acid sequence (SEQ ID NO:310) derived from the coding sequence of SEQ ID NO: 310 shown in Figure 310.

Figure 311A-D shows a nucleotide sequence (SEQ ID NO:311) of a native sequence PRO84704 cDNA, wherein SEQ ID NO:311 is a clone designated herein as "DNA329037".

Figure 312A-B shows the amino acid sequence (SEQ ID NO:312) derived from the coding sequence of SEQ ID NO:311 shown in Figure 311A-D.

Figure 313 shows a nucleotide sequence (SEQ ID NO:313) of a native sequence PR084705 cDNA, wherein SEQ ID NO:313 is a clone designated herein as "DNA329038".

Figure 314 shows the amino acid sequence (SEQ ID NO:314) derived from the coding sequence of SEQ ID NO: 313 shown in Figure 313.

Figure 315A-B shows a nucleotide sequence (SEQ ID NO:315) of a native sequence PRO84706 cDNA, wherein SEQ ID NO:315 is a clone designated herein as "DNA329039".

Figure 316 shows the amino acid sequence (SEQ ID NO:316) derived from the coding sequence of SEQ ID NO: 315 shown in Figure 315A-B.

Figure 317 shows a nucleotide sequence (SEQ ID NO:317) of a native sequence PRO57996 cDNA, wherein SEQ ID NO:317 is a clone designated herein as "DNA328509".

Figure 318 shows the amino acid sequence (SEQ ID NO:318) derived from the coding sequence of SEQ ID NO: 317 shown in Figure 317.

Figure 319A-B shows a nucleotide sequence (SEQ ID NO:319) of a native sequence PRO84368 cDNA, wherein SEQ ID NO:309 is a clone designated herein as "DNA328574".

Figure 320 shows the amino acid sequence (SEQ ID NO:320) derived from the coding sequence of SEQ ID NO: 319 shown in Figure 319A-B.

Figure 321 shows a nucleotide sequence (SEQ ID NO:321) of a native sequence cDNA, wherein SEQ ID NO:321 is a clone designated herein as "DNA256872".

Figure 322 shows a nucleotide sequence (SEQ ID NO:322) of a native sequence cDNA, wherein SEQ ID NO:322 is a clone designated herein as "DNA256422".

Figure 323 shows a nucleotide sequence (SEQ ID NO:323) of a native sequence PRO84496 cDNA, wherein SEQ ID NO:323 is a clone designated herein as "DNA328744".

Figure 324 shows the amino acid sequence (SEQ ID NO:324) derived from the coding sequence of SEQ ID NO: 323 shown in Figure 323.

Figure 325 shows a nucleotide sequence (SEQ ID NO:325) of a native sequence PR084707 cDNA, wherein SEQ ID NO:325 is a clone designated herein as "DNA329040".

Figure 326 shows the amino acid sequence (SEQ ID NO:326) derived from the coding sequence of SEQ ID NO: 325 shown in Figure 325.

Figure 327 shows a nucleotide sequence (SEQ ID NO:327) of a native sequence cDNA, wherein SEQ ID NO:327 is a clone designated herein as "DNA329041".

Figure 328 shows a nucleotide sequence (SEQ ID NO:328) of a native sequence PR049765 cDNA, wherein SEQ ID NO:328 is a clone designated herein as "DNA329042".

Figure 329 shows the amino acid sequence (SEQ ID NO:329) derived from the coding sequence of SEQ ID NO: 328 shown in Figure 328.

Figure 330 shows a nucleotide sequence (SEQ ID NO:330) of a native sequence cDNA, wherein SEQ ID NO:330 is a clone designated herein as "DNA254286".

Figure 331 shows a nucleotide sequence (SEQ ID NO:331) of a native sequence PR023253 cDNA, wherein SEQ ID NO:331 is a clone designated herein as "DNA329043".

Figure 332 shows the amino acid sequence (SEQ ID NO:332) derived from the coding sequence of SEQ ID NO: 331 shown in Figure 331.

Figure 333 shows a nucleotide sequence (SEQ ID NO:333) of a native sequence PRO84709 cDNA, wherein SEQ ID NO:333 is a clone designated herein as "DNA329044".

Figure 334 shows the amino acid sequence (SEQ ID NO:334) derived from the coding sequence of SEQ ID NO: 333 shown in Figure 333.

Figure 335A-B shows a nucleotide sequence (SEQ ID NO:335) of a native sequence PR082352 cDNA, wherein SEQ ID NO:335 is a clone designated herein as "DNA325896".

Figure 336 shows the amino acid sequence (SEQ ID NO:336) derived from the coding sequence of SEQ ID NO: 335 shown in Figure 335A-B.

Figure 337 shows a nucleotide sequence (SEQ ID NO:337) of a native sequence PRO82903 cDNA, wherein SEQ ID NO:337 is a clone designated herein as "DNA326535".

Figure 338 shows the amino acid sequence (SEQ ID NO:338) derived from the coding sequence of SEQ ID NO: 337 shown in Figure 337.

Figure 339 shows a nucleotide sequence (SEQ ID NO:339) of a native sequence PR083260 cDNA, wherein SEQ ID NO:339 is a clone designated herein as "DNA326942".

Figure 340 shows the amino acid sequence (SEQ ID NO:340) derived from the coding sequence of SEQ ID NO: 339 shown in Figure 339.

Figure 341 shows a nucleotide sequence (SEQ ID NO:341) of a native sequence PRO83681 cDNA, wherein SEQ ID NO:341 is a clone designated herein as "DNA327698".

Figure 342 shows the amino acid sequence (SEQ ID NO:342) derived from the coding sequence of SEQ ID NO: 341 shown in Figure 341.

Figure 343 shows a nucleotide sequence (SEQ ID NO:343) of a native sequence PR069487 cDNA, wherein SEQ ID NO:343 is a clone designated herein as "DNA287203".

Figure 344 shows the amino acid sequence (SEQ ID NO:344) derived from the coding sequence of SEQ ID NO: 343 shown in Figure 343.

Figure 345 shows a nucleotide sequence (SEQ ID NO:345) of a native sequence PRO83148 cDNA, wherein SEQ ID NO:345 is a clone designated herein as "DNA329045".

Figure 346 shows the amino acid sequence (SEQ ID NO:346) derived from the coding sequence of SEQ ID NO: 345 shown in Figure 345.

Figure 347 shows a nucleotide sequence (SEQ ID NO:347) of a native sequence PRO58958 cDNA, wherein SEQ ID NO:347 is a clone designated herein as "DNA270585".

Figure 348 shows the amino acid sequence (SEQ ID NO:348) derived from the coding sequence of SEQ ID NO: 347 shown in Figure 347.

Figure 349 shows a nucleotide sequence (SEQ ID NO:349) of a native sequence PR058399 cDNA, wherein SEQ ID NO:349 is a clone designated herein as "DNA329046".

Figure 350 shows the amino acid sequence (SEQ ID NO:350) derived from the coding sequence of SEQ ID NO: 349 shown in Figure 349.

Figure 351 shows a nucleotide sequence (SEQ ID NO:351) of a native sequence PRO80649 cDNA, wherein SEQ ID NO:351 is a clone designated herein as "DNA327584".

Figure 352 shows the amino acid sequence (SEQ ID NO:352) derived from the coding sequence of SEQ ID NO: 351 shown in Figure 351.

Figure 353 shows a nucleotide sequence (SEQ ID NO:353) of a native sequence PRO58425 cDNA, wherein SEQ ID NO:353 is a clone designated herein as "DNA329047".

Figure 354 shows the amino acid sequence (SEQ ID NO:354) derived from the coding sequence of SEQ ID NO: 353 shown in Figure 353.

Figure 355 shows a nucleotide sequence (SEQ ID NO:355) of a native sequence PR084376 cDNA, wherein SEQ ID NO:355 is a clone designated herein as "DNA328591".

Figure 356 shows the amino acid sequence (SEQ ID NO:356) derived from the coding sequence of SEQ ID NO: 355 shown in Figure 355.

Figure 357 shows a nucleotide sequence (SEQ ID NO:357) of a native sequence PRO84309 cDNA, wherein SEQ ID NO:357 is a clone designated herein as "DNA328483".

Figure 358 shows the amino acid sequence (SEQ ID NO:358) derived from the coding sequence of SEQ ID NO: 357 shown in Figure 357.

Figure 359 shows a nucleotide sequence (SEQ ID NO:359) of a native sequence PRO59142 cDNA, wherein SEQ ID NO:359 is a clone designated herein as "DNA326777".

Figure 360 shows the amino acid sequence (SEQ ID NO:360) derived from the coding sequence of SEQ ID NO: 359 shown in Figure 359.

Figure 361A-B shows a nucleotide sequence (SEQ ID NO:361) of a native sequence PRO58810 cDNA, wherein SEQ ID NO:361 is a clone designated herein as "DNA270430".

Figure 362 shows the amino acid sequence (SEQ ID NO:362) derived from the coding sequence of SEQ ID NO: 361 shown in Figure 361A-B.

Figure 363 shows a nucleotide sequence (SEQ ID NO:363) of a native sequence PRO84710 cDNA, wherein SEQ ID NO:363 is a clone designated herein as "DNA329048".

Figure 364 shows the amino acid sequence (SEQ ID NO:364) derived from the coding sequence of SEQ ID NO: 363 shown in Figure 363.

* shows a nucleotide sequence (SEQ ID NO:365) of a native sequence PR080648 cDNA, wherein SEQ ID NO:365 is a clone designated herein as "DNA323910".

Figure 366 shows the amino acid sequence (SEQ ID NO:366) derived from the coding sequence of SEQ ID NO: 365 shown in Figure 365.

Figure 367A-C shows a nucleotide sequence (SEQ ID NO:367) of a native sequence PRO84711 cDNA, wherein SEQ ID NO:367 is a clone designated herein as "DNA329049".

Figure 368 shows the amino acid sequence (SEQ ID NO:368) derived from the coding sequence of SEQ ID NO: 367 shown in Figure 367A-C.

Figure 369 shows a nucleotide sequence (SEQ ID NO:369) of a native sequence PRO84712 cDNA, wherein SEQ ID NO:369 is a clone designated herein as "DNA329050".

Figure 370 shows the amino acid sequence (SEQ ID NO:370) derived from the coding sequence of SEQ ID NO: 369 shown in Figure 369.

Figure 371 shows a nucleotide sequence (SEQ ID NO:371) of a native sequence PR082872 cDNA, wherein SEQ ID NO:371 is a clone designated herein as "DNA326496".

Figure 372 shows the amino acid sequence (SEQ ID NO:372) derived from the coding sequence of SEQ ID NO: 371 shown in Figure 371.

Figure 373A-B shows a nucleotide sequence (SEQ ID NO:373) of a native sequence cDNA, wherein SEQ ID NO: 373 is a clone designated herein as "DNA329051".

Figure 374 shows a nucleotide sequence (SEQ ID NO:374) of a native sequence PR084714 cDNA, wherein SEQ ID NO:374 is a clone designated herein as "DNA329052".

Figure 375 shows the amino acid sequence (SEQ ID NO:375) derived from the coding sequence of SEQ ID NO: 374 shown in Figure 374.

Figure 376 shows a nucleotide sequence (SEQ ID NO:376) of a native sequence PR084183 cDNA, wherein SEQ ID NO:376 is a clone designated herein as "DNA328315".

Figure 377 shows the amino acid sequence (SEQ ID NO:377) derived from the coding sequence of SEQ ID NO: 376 shown in Figure 376.

Figure 378 shows a nucleotide sequence (SEQ ID NO:378) of a native sequence PRO83879 cDNA, wherein SEQ ID NO:378 is a clone designated herein as "DNA327954".

Figure 379 shows the amino acid sequence (SEQ ID NO:379) derived from the coding sequence of SEQ ID NO: 378 shown in Figure 378.

Figure 380 shows a nucleotide sequence (SEQ ID NO:380) of a native sequence PR084715 cDNA, wherein SEQ ID NO:380 is a clone designated herein as "DNA329053".

Figure 381 shows the amino acid sequence (SEQ ID NO:381) derived from the coding sequence of SEQ ID NO: 380 shown in Figure 380.

Figure 382 shows a nucleotide sequence (SEQ ID NO:382) of a native sequence PRO84716 cDNA, wherein SEQ ID NO:382 is a clone designated herein as "DNA329054".

Figure 383 shows the amino acid sequence (SEQ ID NO:383) derived from the coding sequence of SEQ ID NO: 382 shown in Figure 382.

Figure 384A-B shows a nucleotide sequence (SEQ ID NO:384) of a native sequence PR084717 cDNA, wherein SEQ ID NO:384 is a clone designated herein as "DNA329055".

Figure 385 shows the amino acid sequence (SEQ ID NO:385) derived from the coding sequence of SEQ ID NO: 384 shown in Figure 384A-B.

Figure 386 shows a nucleotide sequence (SEQ ID NO:386) of a native sequence PRO71206 cDNA, wherein SEQ ID NO:386 is a clone designated herein as "DNA304794".

Figure 387 shows the amino acid sequence (SEQ ID NO:387) derived from the coding sequence of SEQ ID NO: 386 shown in Figure 386.

Figure 388 shows a nucleotide sequence (SEQ ID NO:388) of a native sequence PR051950 cDNA, wherein SEQ ID NO:388 is a clone designated herein as "DNA257363".

Figure 389 shows the amino acid sequence (SEQ ID NO:389) derived from the coding sequence of SEQ ID NO: 388 shown in Figure 388.

Figure 390 shows a nucleotide sequence (SEQ ID NO:390) of a native sequence cDNA, wherein SEQ ID NO:390 is a clone designated herein as "DNA259165".

Figure 391 shows a nucleotide sequence (SEQ ID NO:391) of a native sequence PR071035 cDNA, wherein SEQ ID NO:391 is a clone designated herein as "DNA304068".

Figure 392 shows the amino acid sequence (SEQ ID NO:392) derived from the coding sequence of SEQ ID NO: 391 shown in Figure 391.

Figure 393 shows a nucleotide sequence (SEQ ID NO:393) of a native sequence PR052268 cDNA, wherein SEQ ID NO:393 is a clone designated herein as "DNA257714".

Figure 394 shows the amino acid sequence (SEQ ID NO:394) derived from the coding sequence of SEQ ID NO: 393 shown in Figure 393.

Figure 395A-B shows a nucleotide sequence (SEQ ID NO:395) of a native sequence PR052040 cDNA, wherein SEQ ID NO:395 is a clone designated herein as "DNA257461".

Figure 396 shows the amino acid sequence (SEQ ID NO:396) derived from the coding sequence of SEQ ID NO: 395 shown in Figure 395A-B.

Figure 397 shows a nucleotide sequence (SEQ ID NO:397) of a native sequence cDNA, wherein SEQ ID NO:397 is a clone designated herein as "DNA259574".

Figure 398A-B shows a nucleotide sequence (SEQ ID NO:398) of a native sequence PRO71288 cDNA, wherein SEQ ID NO:398 is a clone designated herein as "DNA304990".

Figure 399 shows the amino acid sequence (SEQ ID NO:399) derived from the coding sequence of SEQ ID NO: 398 shown in Figure 398A-B.

Figure 400 shows a nucleotide sequence (SEQ ID NO:400) of a native sequence cDNA, wherein SEQ ID NO:400 is a clone designated herein as "DNA262708".

Figure 401 shows a nucleotide sequence (SEQ ID NO:401) of a native sequence cDNA, wherein SEQ ID NO:401 is a clone designated herein as "DNA269148".

Figure 402A-B shows a nucleotide sequence (SEQ ID NO:402) of a native sequence PR069458 cDNA, wherein SEQ ID NO:402 is a clone designated herein as "DNA304800".

Figure 403 shows the amino acid sequence (SEQ ID NO:403) derived from the coding sequence of SEQ ID NO: 402 shown in Figure 402A-B.

Figure 404 shows a nucleotide sequence (SEQ ID NO:404) of a native sequence PR069903 cDNA, wherein SEQ ID NO:404 is a clone designated herein as "DNA287659".

Figure 405 shows the amino acid sequence (SEQ ID NO:405) derived from the coding sequence of SEQ ID NO: 404 shown in Figure 404.

Figure 406 shows a nucleotide sequence (SEQ ID NO:406) of a native sequence cDNA, wherein SEQ ID NO:406 is a clone designated herein as "DNA268714".

Figure 407 shows a nucleotide sequence (SEQ ID NO:407) of a native sequence cDNA, wherein SEQ ID NO:407 is a clone designated herein as "DNA260031".

Figure 408 shows a nucleotide sequence (SEQ ID NO:408) of a native sequence cDNA, wherein SEQ ID NO:408 is a clone designated herein as "DNA259663".

Figure 409 shows a nucleotide sequence (SEQ ID NO:409) of a native sequence cDNA, wherein SEQ ID NO:409 is a clone designated herein as "DNA263300".

Figure 410 shows a nucleotide sequence (SEQ ID NO:410) of a native sequence PR052672 cDNA, wherein SEQ ID NO:410 is a clone designated herein as "DNA258737".

Figure 411 shows the amino acid sequence (SEQ ID NO:411) derived from the coding sequence of SEQ ID NO: 410 shown in Figure 410.

Figure 412 shows a nucleotide sequence (SEQ ID NO:412) of a native sequence PRO52174 cDNA, wherein SEQ ID NO:412 is a clone designated herein as "DNA287258".

Figure 413 shows the amino acid sequence (SEQ ID NO:413) derived from the coding sequence of SEQ ID NO: 412 shown in Figure 412.

Figure 414 shows a nucleotide sequence (SEQ ID NO:414) of a native sequence PR071289 cDNA, wherein SEQ ID NO:414 is a clone designated herein as "DNA304991".

Figure 415 shows the amino acid sequence (SEQ ID NO:415) derived from the coding sequence of SEQ ID NO: 414 shown in Figure 414.

Figure 416 shows a nucleotide sequence (SEQ ID NO:416) of a native sequence PRO58230 cDNA, wherein SEQ ID NO:41 is a clone designated herein as "DNA269828".

Figure 417 shows the amino acid sequence (SEQ ID NO:417) derived from the coding sequence of SEQ ID NO: 416 shown in Figure 416.

Figure 418 shows a nucleotide sequence (SEQ ID NO:418) of a native sequence PRO71238 cDNA, wherein SEQ ID NO:418 is a clone designated herein as "DNA304831".

Figure 419 shows the amino acid sequence (SEQ ID NO:419) derived from the coding sequence of SEQ ID NO: 418 shown in Figure 418.

Figure 420A-B shows a nucleotide sequence (SEQ ID NO:420) of a native sequence PRO84718 cDNA, wherein SEQ ID NO:420 is a clone designated herein as "DNA329056".

Figure 421 shows the amino acid sequence (SEQ ID NO:421) derived from the coding sequence of SEQ ID NO: 420 shown in Figure 420.

Figure 422 shows a nucleotide sequence (SEQ ID NO:422) of a native sequence PR069632 cDNA, wherein SEQ ID NO:422 is a clone designated herein as "DNA287372".

Figure 423 shows the amino acid sequence (SEQ ID NO:423) derived from the coding sequence of SEQ ID NO: 422 shown in Figure 422.

Figure 424A-B shows a nucleotide sequence (SEQ ID NO:424) of a native sequence PR04913 cDNA, wherein SEQ ID NO:424 is a clone designated herein as "DNA103589".

Figure 425 shows the amino acid sequence (SEQ ID NO:425) derived from the coding sequence of SEQ ID NO: 424 shown in Figure 424A-B.

Figure 426 shows a nucleotide sequence (SEQ ID NO:426) of a native sequence PR084265 cDNA, wherein SEQ ID NO:426 is a clone designated herein as "DNA328427".

Figure 427 shows the amino acid sequence (SEQ ID NO:427) derived from the coding sequence of SEQ ID NO: 426 shown in Figure 426.

Figure 428 shows a nucleotide sequence (SEQ ID NO:428) of a native sequence PRO84719 cDNA, wherein SEQ ID NO:428 is a clone designated herein as "DNA329057".

Figure 429 shows the amino acid sequence (SEQ ID NO:429) derived from the coding sequence of SEQ ID NO: 428 shown in Figure 428.

Figure 430 shows a nucleotide sequence (SEQ ID NO:430) of a native sequence PR057922 cDNA, wherein SEQ ID NO:430 is a clone designated herein as "DNA327568".

Figure 431 shows the amino acid sequence (SEQ ID NO:431) derived from the coding sequence of SEQ ID NO: 430 shown in Figure 430.

Figure 432 shows a nucleotide sequence (SEQ ID NO:432) of a native sequence PRO12613 cDNA, wherein SEQ ID NO:432 is a clone designated herein as "DNA151139".

Figure 433 shows the amino acid sequence (SEQ ID NO:433) derived from the coding sequence of SEQ ID NO: 432 shown in Figure 432.

Figure 434 shows a nucleotide sequence (SEQ ID NO:434) of a native sequence PRO62312 cDNA, wherein SEQ ID NO:434 is a clone designated herein as "DNA329058".

Figure 435 shows the amino acid sequence (SEQ ID NO:435) derived from the coding sequence of SEQ ID NO: 434 shown in Figure 434.

Figure 436A-B shows a nucleotide sequence (SEQ ID NO:436) of a native sequence PRO60891 cDNA, wherein SEQ ID NO:436 is a clone designated herein as "DNA272786".

Figure 437 shows the amino acid sequence (SEQ ID NO:437) derived from the coding sequence of SEQ ID NO:

436 shown in Figure 436A-B.

Figure 438A-B shows a nucleotide sequence (SEQ ID NO:438) of a native sequence PRO84720 cDNA, wherein SEQ ID NO:438 is a clone designated herein as "DNA329059".

Figure 439 shows the amino acid sequence (SEQ ID NO:439) derived from the coding sequence of SEQ ID NO: 438 shown in Figure 438A-B.

Figure 440A-B shows a nucleotide sequence (SEQ ID NO:440) of a native sequence PRO84721 cDNA, wherein SEQ ID NO:440 is a clone designated herein as "DNA329060".

Figure 441 shows the amino acid sequence (SEQ ID NO:441) derived from the coding sequence of SEQ ID NO: 440 shown in Figure 440.

Figure 442A-B shows a nucleotide sequence (SEQ ID NO:442) of a native sequence PR084722 cDNA, wherein SEQ ID NO:442 is a clone designated herein as "DNA329061".

Figure 443 shows the amino acid sequence (SEQ ID NO:443) derived from the coding sequence of SEQ ID NO: 442 shown in Figure 442A-B.

Figure 444A-B shows a nucleotide sequence (SEQ ID NO:444) of a native sequence PR04854 cDNA, wherein SEQ ID NO:444 is a clone designated herein as "DNA103527".

Figure 445 shows the amino acid sequence (SEQ ID NO:445) derived from the coding sequence of SEQ ID NO: 444 shown in Figure 444A-B.

Figure 446A-B shows a nucleotide sequence (SEQ ID NO:446) of a native sequence PRO59611 cDNA, wherein SEQ ID NO:446 is a clone designated herein as "DNA271304".

Figure 447 shows the amino acid sequence (SEQ ID NO:447) derived from the coding sequence of SEQ ID NO: 446 shown in Figure 446A-B.

Figure 448 shows a nucleotide sequence (SEQ ID NO:448) of a native sequence PRO60271 cDNA, wherein SEQ ID NO:448 is a clone designated herein as "DNA271996".

Figure 449 shows the amino acid sequence (SEQ ID NO:449) derived from the coding sequence of SEQ ID NO: 448 shown in Figure 448.

Figure 450A-B shows a nucleotide sequence (SEQ ID NO:450) of a native sequence PRO63074 cDNA, wherein SEQ ID NO:450 is a clone designated herein as "DNA329062".

Figure 451 shows the amino acid sequence (SEQ ID NO:451) derived from the coding sequence of SEQ ID NO: 450 shown in Figure 450A-B.

Figure 452 shows a nucleotide sequence (SEQ ID NO:452) of a native sequence PR084723 cDNA, wherein SEQ ID NO:452 is a clone designated herein as "DNA329063".

Figure 453 shows the amino acid sequence (SEQ ID NO:453) derived from the coding sequence of SEQ ID NO: 452 shown in Figure 452.

Figure 454 shows a nucleotide sequence (SEQ ID NO:454) of a native sequence PRO84724 cDNA, wherein SEQ ID NO:454 is a clone designated herein as "DNA329064".

Figure 455 shows the amino acid sequence (SEQ ID NO:455) derived from the coding sequence of SEQ ID NO: 454 shown in Figure 454.

Figure 456 shows a nucleotide sequence (SEQ ID NO:456) of a native sequence PRO35972 cDNA, wherein SEQ ID NO:456 is a clone designated herein as "DNA225509".

Figure 457 shows the amino acid sequence (SEQ ID NO:457) derived from the coding sequence of SEQ ID NO: 456 shown in Figure 456.

Figure 458 shows a nucleotide sequence (SEQ ID NO:458) of a native sequence cDNA, wherein SEQ ID NO:458 is a clone designated herein as "DNA269599".

Figure 459 shows a nucleotide sequence (SEQ ID NO:459) of a native sequence PR084434 cDNA, wherein SEQ ID NO:459 is a clone designated herein as "DNA328660".

Figure 460 shows the amino acid sequence (SEQ ID NO:460) derived from the coding sequence of SEQ ID NO: 459 shown in Figure 459.

Figure 461 shows a nucleotide sequence (SEQ ID NO:461) of a native sequence PR059476 cDNA, wherein SEQ ID NO:461 is a clone designated herein as "DNA271155".

Figure 462 shows the amino acid sequence (SEQ ID NO:462) derived from the coding sequence of SEQ ID NO: 461 shown in Figure 461.

Figure 463A-B shows a nucleotide sequence (SEQ ID NO:463) of a native sequence PRO84725 cDNA, wherein SEQ ID NO:463 is a clone designated herein as "DNA329065".

Figure 464 shows the amino acid sequence (SEQ ID NO:464) derived from the coding sequence of SEQ ID NO: 463 shown in Figure 463A-B.

Figure 465 shows a nucleotide sequence (SEQ ID NO:465) of a native sequence PR084726 cDNA, wherein SEQ ID NO:465 is a clone designated herein as "DNA329066".

Figure 466 shows the amino acid sequence (SEQ ID NO:466) derived from the coding sequence of SEQ ID NO: 465 shown in Figure 465.

Figure 467 shows a nucleotide sequence (SEQ ID NO:467) of a native sequence PR084727 cDNA, wherein SEQ ID NO:467 is a clone designated herein as "DNA329067".

Figure 468 shows the amino acid sequence (SEQ ID NO:468) derived from the coding sequence of SEQ ID NO: 467 shown in Figure 467.

Figure 469A-B shows a nucleotide sequence (SEQ ID NO:469) of a native sequence cDNA, wherein SEQ ID NO: 469 is a clone designated herein as "DNA329068".

Figure 470A-B shows a nucleotide sequence (SEQ ID NO:470) of a native sequence cDNA, wherein SEQ ID NO: 470 is a clone designated herein as "DNA329069".

Figure 471A-C shows a nucleotide sequence (SEQ ID NO:471) of a native sequence PRO84728 cDNA, wherein SEQ ID NO:471 is a clone designated herein as "DNA329070".

Figure 472 shows the amino acid sequence (SEQ ID NO:472) derived from the coding sequence of SEQ ID NO: 471 shown in Figure 471.

Figure 473 shows a nucleotide sequence (SEQ ID NO:473) of a native sequence PR084729 cDNA, wherein SEQ ID NO:473 is a clone designated herein as "DNA329071".

Figure 474 shows the amino acid sequence (SEQ ID NO:474) derived from the coding sequence of SEQ ID NO: 473 shown in Figure 473.

Figure 475 shows a nucleotide sequence (SEQ ID NO:475) of a native sequence PR084730 cDNA, wherein SEQ ID NO:475 is a clone designated herein as "DNA329072".

Figure 476 shows the amino acid sequence (SEQ ID NO:476) derived from the coding sequence of SEQ ID NO:

475 shown in Figure 475.

Figure 477 shows a nucleotide sequence (SEQ ID NO:477) of a native sequence PRO84731 cDNA, wherein SEQ ID NO:477 is a clone designated herein as "DNA329073".

Figure 478 shows the amino acid sequence (SEQ ID NO:478) derived from the coding sequence of SEQ ID NO: 477 shown in Figure 477.

Figure 479 shows a nucleotide sequence (SEQ ID NO:479) of a native sequence PRO21326 cDNA, wherein SEQ ID NO:479 is a clone designated herein as "DNA329074".

Figure 480 shows the amino acid sequence (SEQ ID NO:480) derived from the coding sequence of SEQ ID NO: 479 shown in Figure 479.

Figure 481A-B shows a nucleotide sequence (SEQ ID NO:481) of a native sequence PR069478 cDNA, wherein SEQ ID NO:481 is a clone designated herein as "DNA287192".

Figure 482 shows the amino acid sequence (SEQ ID NO:482) derived from the coding sequence of SEQ ID NO: 481 shown in Figure 481A-B.

Figure 483 shows a nucleotide sequence (SEQ ID NO:483) of a native sequence PR080846 cDNA, wherein SEQ ID NO:483 is a clone designated herein as "DNA324145".

Figure 484 shows the amino acid sequence (SEQ ID NO:484) derived from the coding sequence of SEQ ID NO: 483 shown in Figure 483.

Figure 485 shows a nucleotide sequence (SEQ ID NO:485) of a native sequence PRO84611 cDNA, wherein SEQ ID NO:485 is a clone designated herein as "DNA328887".

Figure 486 shows the amino acid sequence (SEQ ID NO:486) derived from the coding sequence of SEQ ID NO: 485 shown in Figure 485.

Figure 487 shows a nucleotide sequence (SEQ ID NO:487) of a native sequence PR061948 cDNA, wherein SEQ ID NO:487 is a clone designated herein as "DNA274002".

Figure 488 shows the amino acid sequence (SEQ ID NO:488) derived from the coding sequence of SEQ ID NO: 487 shown in Figure 487.

Figure 489 shows a nucleotide sequence (SEQ ID NO:489) of a native sequence PRO84732 cDNA, wherein SEQ ID NO:489 is a clone designated herein as "DNA329075".

Figure 490 shows the amino acid sequence (SEQ ID NO:490) derived from the coding sequence of SEQ ID NO: 489 shown in Figure 489.

Figure 491A-B shows a nucleotide sequence (SEQ ID NO:491) of a native sequence PRO84733 cDNA, wherein SEQ ID NO:491 is a clone designated herein as "DNA329076".

Figure 492 shows the amino acid sequence (SEQ ID NO:492) derived from the coding sequence of SEQ ID NO: 491 shown in Figure 491.

Figure 493 shows a nucleotide sequence (SEQ ID NO:493) of a native sequence PRO59776 cDNA, wherein SEQ ID NO:493 is a clone designated herein as "DNA271483".

Figure 494 shows the amino acid sequence (SEQ ID NO:494) derived from the coding sequence of SEQ ID NO: 493 shown in Figure 493.

Figure 495 shows a nucleotide sequence (SEQ ID NO:495) of a native sequence PR084734 cDNA, wherein SEQ ID NO:495 is a clone designated herein as "DNA329077".

Figure 496 shows the amino acid sequence (SEQ ID NO:496) derived from the coding sequence of SEQ ID NO: 495 shown in Figure 495.

Figure 497 shows a nucleotide sequence (SEQ ID NO:497) of a native sequence PR083839 cDNA, wherein SEQ ID NO:497 is a clone designated herein as "DNA327904".

Figure 498 shows the amino acid sequence (SEQ ID NO:498) derived from the coding sequence of SEQ ID NO: 497 shown in Figure 497.

Figure 499 shows a nucleotide sequence (SEQ ID NO:499) of a native sequence PR069472 cDNA, wherein SEQ ID NO:499 is a clone designated herein as "DNA287186".

Figure 500 shows the amino acid sequence (SEQ ID NO:500) derived from the coding sequence of SEQ ID NO: 499 shown in Figure 499.

Figure 501 shows a nucleotide sequence (SEQ ID NO:501) of a native sequence PRO23253 cDNA, wherein SEQ ID NO:501 is a clone designated herein as "DNA329078".

Figure 502 shows the amino acid sequence (SEQ ID NO:502) derived from the coding sequence of SEQ ID NO: 501 shown in Figure 501.

Figure 503 shows a nucleotide sequence (SEQ ID NO:503) of a native sequence PRO84735 cDNA, wherein SEQ ID NO:503 is a clone designated herein as "DNA329079".

Figure 504 shows the amino acid sequence (SEQ ID NO:504) derived from the coding sequence of SEQ ID NO: 503 shown in Figure 503.

Figure 505 shows a nucleotide sequence (SEQ ID NO:505) of a native sequence PR037583 cDNA, wherein SEQ ID NO:505 is a clone designated herein as "DNA227120".

Figure 506 shows the amino acid sequence (SEQ ID NO:506) derived from the coding sequence of SEQ ID NO: 505 shown in Figure 505.

The sequences identified as designated Figures with the corresponding SEQ ID NOs are shown in the form of a sequence listing, indicating the SEQ ID NOs, in the description.

DETECTED DESCRIPTION OF THE PREFERRED EMBODIMENTS

1. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.
A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g.*, an extracellular domain sequence), naturally-occurring variant forms (*e.g.*, alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table I below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table I below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 $\mu$g/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$-$V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues ofN-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, etc.

The term "B cell mediated disease" means a disease in which B cells directly or indirectly mediate or otherwise contribute to a morbidity in a mammal. The B cell mediated disease may be associated with cell mediated effects, Ig mediated effects, and even effects associated with T cells if the T cells are stimulated, for example, by the lymphokines secreted by B cells.

Examples of immune-related and inflammatory diseases which are immune or B cell mediated, which may be treated according to the invention include: systemic lupus erythematosis, X-linked infantile hypogammaglobulinemia, polysaccharide antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g.*, $I^{131}$, $I^{125}$, $Y^{90}$ and Re $^{186}$), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as 1L-1, IL-1$\alpha$, IL-2, IL-3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

## Table 1

```
/*
 *
 * C-C increased from 12 to 15
 * Z is average of EQ
 * B is average of ND
 * match with stop is _M; stop-stop = 0; J (joker) match = 0
 */
#define   _M        -8        /* value of a match with a stop */

int      _day[26][26] = {
/*       A   B   C   D   E   F   G   H   I   J   K   L   M   N   O   P   Q   R   S   T   U   V   W   X   Y   Z */
/* A */  { 2, 0,-2, 0, 0,-4, 1,-1,-1, 0,-1,-2,-1, 0,_M, 1, 0,-2, 1, 1, 0, 0,-6, 0,-3, 0},
/* B */  { 0, 3,-4, 3, 2,-5, 0, 1,-2, 0, 0,-3,-2, 2,_M,-1, 1, 0, 0, 0, 0,-2,-5, 0,-3, 1},
/* C */  {-2,-4,15,-5,-5,-4,-3,-3,-2, 0,-5,-6,-5,-4,_M,-3,-5,-4, 0,-2, 0,-2,-8, 0, 0,-5},
/* D */  { 0, 3,-5, 4, 3,-6, 1, 1,-2, 0, 0,-4,-3, 2,_M,-1, 2,-1, 0, 0, 0,-2,-7, 0,-4, 2},
/* E */  { 0, 2,-5, 3, 4,-5, 0, 1,-2, 0, 0,-3,-2, 1,_M,-1, 2,-1, 0, 0, 0,-2,-7, 0,-4, 3},
/* F */  {-4,-5,-4,-6,-5, 9,-5,-2, 1, 0,-5, 2, 0,-4,_M,-5,-5,-4,-3,-3, 0,-1, 0, 0, 7,-5},
/* G */  { 1, 0,-3, 1, 0,-5, 5,-2,-3, 0,-2,-4,-3, 0,_M,-1,-1,-3, 1, 0, 0,-1,-7, 0,-5, 0},
/* H */  {-1, 1,-3, 1, 1,-2,-2, 6,-2, 0, 0,-2,-2, 2,_M, 0, 3, 2,-1,-1, 0,-2,-3, 0, 0, 2},
/* I */  {-1,-2,-2,-2,-2, 1,-3,-2, 5, 0,-2, 2, 2,-2,_M,-2,-2,-2,-1, 0, 0, 4,-5, 0,-1,-2},
/* J */  { 0, 0, 0, 0, 0, 0, 0, 0, 0, 0, 0, 0, 0, 0,_M, 0, 0, 0, 0, 0, 0, 0, 0, 0, 0, 0},
/* K */  {-1, 0,-5, 0, 0,-5,-2, 0,-2, 0, 5,-3, 0, 1,_M,-1, 1, 3, 0, 0, 0,-2,-3, 0,-4, 0},
/* L */  {-2,-3,-6,-4,-3, 2,-4,-2, 2, 0,-3, 6, 4,-3,_M,-3,-2,-3,-3,-1, 0, 2,-2, 0,-1,-2},
/* M */  {-1,-2,-5,-3,-2, 0,-3,-2, 2, 0, 0, 4, 6,-2,_M,-2,-1, 0,-2,-1, 0, 2,-4, 0,-2,-1},
/* N */  { 0, 2,-4, 2, 1,-4, 0, 2,-2, 0, 1,-3,-2, 2,_M,-1, 1, 0, 1, 0, 0,-2,-4, 0,-2, 1},
/* O */  {_M,_M,_M,_M,_M,_M,_M,_M,_M,_M,_M,_M,_M,_M,
0,_M,_M,_M,_M,_M,_M,_M,_M,_M,_M,_M},
/* P */  { 1,-1,-3,-1,-1,-5,-1, 0,-2, 0,-1,-3,-2,-1,_M, 6, 0, 0, 1, 0, 0,-1,-6, 0,-5, 0},
/* Q */  { 0, 1,-5, 2, 2,-5,-1, 3,-2, 0, 1,-2,-1, 1,_M, 0, 4, 1,-1,-1, 0,-2,-5, 0,-4, 3},
/* R */  {-2, 0,-4,-1,-1,-4,-3, 2,-2, 0, 3,-3, 0, 0,_M, 0, 1, 6, 0,-1, 0,-2, 2, 0,-4, 0},
/* S */  { 1, 0, 0, 0, 0,-3, 1,-1,-1, 0, 0,-3,-2, 1,_M, 1,-1, 0, 2, 1, 0,-1,-2, 0,-3, 0},
/* T */  { 1, 0,-2, 0, 0,-3, 0,-1, 0, 0, 0,-1,-1, 0,_M, 0,-1,-1, 1, 3, 0, 0,-5, 0,-3, 0},
/* U */  { 0, 0, 0, 0, 0, 0, 0, 0, 0, 0, 0, 0, 0, 0,_M, 0, 0, 0, 0, 0, 0, 0, 0, 0, 0, 0},
/* V */  { 0,-2,-2,-2,-2,-1,-1,-2, 4, 0,-2, 2, 2,-2,_M,-1,-2,-2,-1, 0, 0, 4,-6, 0,-2,-2},
/* W */  {-6,-5,-8,-7,-7, 0,-7,-3,-5, 0,-3,-2,-4,-4,_M,-6,-5, 2,-2,-5, 0,-6,17, 0, 0,-6},
/* X */  { 0, 0, 0, 0, 0, 0, 0, 0, 0, 0, 0, 0, 0, 0,_M, 0, 0, 0, 0, 0, 0, 0, 0, 0, 0, 0},
/* Y */  {-3,-3, 0,-4,-4, 7,-5, 0,-1, 0,-4,-1,-2,-2,_M,-5,-4,-4,-3,-3, 0,-2, 0, 0,10,-4},
/* Z */  { 0, 1,-5, 2, 3,-5, 0, 2,-2, 0, 0,-2,-1, 1,_M, 0, 3, 0, 0, 0, 0,-2,-6, 0,-4, 4}
};
```

## Table 1 (cont')

```
/*
*/
#include <stdio.h>
#include <ctype.h>

#define  MAXJMP    16      /* max jumps in a diag */
#define  MAXGAP    24      /* don't continue to penalize gaps larger than this */
#define  JMPS      1024    /* max jmps in an path */
#define  MX        4       /* save if there's at least MX-1 bases since last jmp */

#define  DMAT      3       /* value of matching bases */
#define  DMIS      0       /* penalty for mismatched bases */
#define  DINS0     8       /* penalty for a gap */
#define  DINS1     1       /* penalty per base */
#define  PINS0     8       /* penalty for a gap */
#define  PINS1     4       /* penalty per residue */

struct jmp {
         short           n[MAXJMP];      /* size of jmp (neg for dely) */
         unsigned short  x[MAXJMP];      /* base no. of jmp in seq x */
};                                       /* limits seq to 2^16 -1 */

struct diag {
         int             score;          /* score at last jmp */
         long            offset;         /* offset of prev block */
         short           ijmp;           /* current jmp index */
         struct jmp      jp;             /* list of jmps */
};

struct path {
         int     spc;             /* number of leading spaces */
         short   n[JMPS];/* size of jmp (gap) */
         int     x[JMPS];/* loc of jmp (last elem before gap) */
};

char            *ofile;                  /* output file name */
char            *namex[2];               /* seq names: getseqs() */
char            *prog;                    /* prog name for err msgs */
char            *seqx[2];                /* seqs: getseqs() */
int             dmax;                    /* best diag: nw() */
int             dmax0;                   /* final diag */
int             dna;                     /* set if dna: main() */
int             endgaps;                 /* set if penalizing end gaps */
int             gapx, gapy;              /* total gaps in seqs */
int             len0, len1;              /* seq lens */
int             ngapx, ngapy;            /* total size of gaps */
int             smax;                    /* max score: nw() */
int             *xbm;                    /* bitmap for matching */
long            offset;                  /* current offset in jmp file */
struct  diag    *dx;                     /* holds diagonals */
struct  path    pp[2];                   /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *    where file1 and file2 are two dna or two protein sequences.
 *    The sequences can be in upper- or lower-case an may contain ambiguity
 *    Any lines beginning with ';', '>' or '<' are ignored
 *    Max file length is 65535 (limited by unsigned short x in the jmp struct)
 *    A sequence with 1/3 or more of its elements ACGTU is assumed to be DNA
 *    Output is in the file "align.out"
 *
 * The program may create a tmp file in /tmp to hold info about traceback.
 * Original version developed under BSD 4.3 on a vax 8650
 */
#include "nw.h"
#include "day.h"

static   _dbval[26] = {
         1,14,2,13,0,0,4,11,0,0,12,0,3,15,0,0,0,5,6,8,8,7,9,0,10,0
};

static   _pbval[26] = {
         1, 2|(1<<('D'-'A'))|(1<<('N'-'A')), 4, 8, 16, 32, 64,
         128, 256, 0xFFFFFFF, 1<<10, 1<<11, 1<<12, 1<<13, 1<<14,
         1<<15, 1<<16, 1<<17, 1<<18, 1<<19, 1<<20, 1<<21, 1<<22,
         1<<23, 1<<24, 1<<25|(1<<('E'-'A'))|(1<<('Q'-'A'))
};

main(ac, av)
         main
         int      ac;
         char     *av[ ];
{
         prog = av[0];
         if (ac != 3) {
                  fprintf(stderr,"usage: %s file1 file2\n", prog);
                  fprintf(stderr,"where file1 and file2 are two dna or two protein sequences.\n");
                  fprintf(stderr,"The sequences can be in upper- or lower-case\n");
                  fprintf(stderr,"Any lines beginning with ';' or '<' are ignored\n");
                  fprintf(stderr,"Output is in the file \"align.out\"\n");
                  exit(1);
         }
         namex[0] = av[1];
         namex[1] = av[2];
         seqx[0] = getseq(namex[0], &len0);
         seqx[1] = getseq(namex[1], &len1);
         xbm = (dna)? _dbval : _pbval;

         endgaps = 0;              /* 1 to penalize endgaps */
         ofile = "align.out";      /* output file */

         nw();                     /* fill in the matrix, get the possible jmps */
         readjmps();               /* get the actual jmps */
         print();                  /* print stats, alignment */

         cleanup(0);               /* unlink any tmp files */

}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()

        nw

{
        char        *px, *py;           /* seqs and ptrs */
        int         *ndely, *dely;      /* keep track of dely */
        int         ndelx, delx;        /* keep track of delx */
        int         *tmp;               /* for swapping row0, row1 */
        int         mis;                /* score for each type */
        int         ins0, ins1;         /* insertion penalties */
        register    id;                 /* diagonal index */
        register    ij;                 /* jmp index */
        register    *col0, *col1;       /* score for curr, last row */
        register    xx, yy;             /* index into seqs */


        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;


        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;            /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

## Table 1 (cont')

...**nw**

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];


        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }


        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

## Table 1 (cont')

...nw

```
            id = xx - yy + len1 - 1;
            if (mis > = delx && mis > = dely[yy])
                        col1[yy] = mis;
            else if (delx > = dely[yy]) {
                        col1[yy] = delx;
                        ij = dx[id].ijmp;
                        if (dx[id].jp.n[0] && (!dna || (ndelx > = MAXJMP
                        && xx > dx[id].jp.x[ij]+MX) || mis > dx[id].score+DINS0)) {
                                    dx[id].ijmp++;
                                    if (++ij > = MAXJMP) {
                                                writejmps(id);
                                                ij = dx[id].ijmp = 0;
                                                dx[id].offset = offset;
                                                offset += sizeof(struct jmp) + sizeof(offset);
                                    }
                        }
                        dx[id].jp.n[ij] = ndelx;
                        dx[id].jp.x[ij] = xx;
                        dx[id].score = delx;
            }
            else {
                        col1[yy] = dely[yy];
                        ij = dx[id].ijmp;
            if (dx[id].jp.n[0] && (!dna || (ndely[yy] > = MAXJMP
                        && xx > dx[id].jp.x[ij]+MX) || mis > dx[id].score+DINS0)) {
                                    dx[id].ijmp++;
                                    if (++ij > = MAXJMP) {
                                                writejmps(id);
                                                ij = dx[id].ijmp = 0;
                                                dx[id].offset = offset;
                                                offset += sizeof(struct jmp) + sizeof(offset);
                                    }
                        }
                        dx[id].jp.n[ij] = -ndely[yy];
                        dx[id].jp.x[ij] = xx;
                        dx[id].score = dely[yy];
            }
            if (xx == len0 && yy < len1) {
                        /* last col
                        */
                        if (endgaps)
                                    col1[yy] -= ins0+ins1*(len1-yy);
                        if (col1[yy] > smax) {
                                    smax = col1[yy];
                                    dmax = id;
                        }
            }
        }
        if (endgaps && xx < len0)
                    col1[yy-1] -= ins0+ins1*(len0-xx);
        if (col1[yy-1] > smax) {
                    smax = col1[yy-1];
                    dmax = id;
        }
        tmp = col0; col0 = col1; col1 = tmp;
}
(void) free((char *)ndely);
(void) free((char *)dely);
(void) free((char *)col0);
(void) free((char *)col1);                              }
```

## Table 1 (cont')

```
/*
 *
 * print() -- only routine visible outside this module
 *
 * static:
 * getmat() -- trace back best path, count matches: print()
 * pr_align() -- print alignment of described in array p[ ]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */


#include "nw.h"


#define SPC         3
#define P_LINE      256     /* maximum output line */
#define P_SPC       3       /* space between name or num and seq */


extern    _day[26][26];
int       olen;             /* set output line length */
FILE      *fx;              /* output file */


print()

print

{

    int     lx, ly, firstgap, lastgap;      /* overlap */

    if ((fx = fopen(ofile, "w")) == 0) {
            fprintf(stderr,"%s: can't write %s\n", prog, ofile);
            cleanup(1);
    }
    fprintf(fx, "<first sequence: %s (length = %d)\n", namex[0], len0);
    fprintf(fx, "<second sequence: %s (length = %d)\n", namex[1], len1);
    olen = 60;
    lx = len0;
    ly = len1;
    firstgap = lastgap = 0;
    if (dmax < len1 - 1) {          /* leading gap in x */
            pp[0].spc = firstgap = len1 - dmax - 1;
            ly -= pp[0].spc;
    }
    else if (dmax > len1 - 1) {   /* leading gap in y */
            pp[1].spc = firstgap = dmax - (len1 - 1);
            lx -= pp[1].spc;
    }
    if (dmax0 < len0 - 1) {         /* trailing gap in x */
            lastgap = len0 - dmax0 -1;
            lx -= lastgap;
    }
    else if (dmax0 > len0 - 1) { /* trailing gap in y */
            lastgap = dmax0 - (len0 - 1);
            ly -= lastgap;
    }
    getmat(lx, ly, firstgap, lastgap);
    pr_align();

}
```

## Table 1 (cont')

```
/*
 * trace back the best path, count matches
 */
static
getmat(lx, ly, firstgap, lastgap)
        int     lx, ly;                 /* "core" (minus endgaps) */
        int     firstgap, lastgap;      /* leading trailing overlap */
{
        int     nm, i0, i1, siz0, siz1;
        char    outx[32];
        double  pct;
        register n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, " < %d match%s in an overlap of %d: %.2f percent similarity\n",
                nm, (nm == 1)? "" : "es", lx, pct);
```

getmat

## Table 1 (cont')

```
fprintf(fx, " <gaps in first sequence: %d", gapx);
```
**...getmat**
```
if (gapx) {
        (void) sprintf(outx, " (%d %s%s)",
                ngapx, (dna)? "base":"residue", (ngapx == 1)? "":"s");
        fprintf(fx,"%s", outx);


fprintf(fx, ", gaps in second sequence: %d", gapy);
if (gapy) {
        (void) sprintf(outx, " (%d %s%s)",
                ngapy, (dna)? "base":"residue", (ngapy == 1)? "":"s");
        fprintf(fx,"%s", outx);

}
if (dna)
        fprintf(fx,
        "\n <score: %d (match = %d, mismatch = %d, gap penalty = %d + %d per base)\n",
        smax, DMAT, DMIS, DINS0, DINS1);

else
        fprintf(fx,
        "\n <score: %d (Dayhoff PAM 250 matrix, gap penalty = %d + %d per residue)\n",
        smax, PINS0, PINS1);

if (endgaps)
        fprintf(fx,
        " <endgaps penalized. left endgap: %d %s%s, right endgap: %d %s%s\n",
        firstgap, (dna)? "base" : "residue", (firstgap == 1)? "" : "s",
        lastgap, (dna)? "base" : "residue", (lastgap == 1)? "" : "s");

else
        fprintf(fx, " <endgaps not penalized\n");
}
static          nm;                     /* matches in core -- for checking */
static          lmax;                   /* lengths of stripped file names */
static          ij[2];                  /* jmp index for a path */
static          nc[2];                  /* number at start of current line */
static          ni[2];                  /* current elem number -- for gapping */
static          siz[2];
static char     *ps[2];                 /* ptr to current element */
static char     *po[2];                 /* ptr to next output char slot */
static char     out[2][P_LINE];         /* output line */
static char     star[P_LINE];           /* set by stars() */


/*
* print alignment of described in struct path pp[ ]
*/
static
pr_align()
```
**pr_align**
```
{
        int             nn;     /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];                                 }
```

## Table 1 (cont')

```
for (nn = nm = 0, more = 1; more; ) {
...pr_align
            for (i = more = 0; i < 2; i++) {
                    /*
                     * do we have more of this sequence?
                     */
                    if (!*ps[i])
                                continue;
                    more++;

                    if (pp[i].spc) {      /* leading space */
                            *po[i]++ = ' ';
                            pp[i].spc--;
                    }
                    else if (siz[i]) {      /* in a gap */
                            *po[i]++ = '-';
                            siz[i]--;
                    }
                    else {                  /* we're putting a seq element
                                             */
                            *po[i] = *ps[i];
                            if (islower(*ps[i]))
                                    *ps[i] = toupper(*ps[i]);
                            po[i]++;
                            ps[i]++;


                            /*
                             * are we at next gap for this seq?
                             */
                            if (ni[i] == pp[i].x[ij[i]]) {
                                    /*
                                     * we need to merge all gaps
                                     * at this location
                                     */
                                    siz[i] = pp[i].n[ij[i]++];
                                    while (ni[i] == pp[i].x[ij[i]])
                                                siz[i] += pp[i].n[ij[i]++];
                            }
                            ni[i]++;
                    }
            }
            if (++nn == olen || !more && nn) {
                    dumpblock();
                    for (i = 0; i < 2; i++)
                            po[i] = out[i];
                    nn = 0;
            }
    }
}

/*
 * dump a block of lines, including numbers, stars: pr_align()
 */
static
dumpblock()
dumpblock
{
        register  i;
        for (i = 0; i < 2; i++)
                *po[i]-- = '\0';
```

## Table 1 (cont')

**...dumpblock**

```
            (void) putc('\n', fx);
            for (i = 0; i < 2; i++) {
                    if (*out[i] && (*out[i] != ' ' || *(po[i]) != ' ')) {
                            if (i == 0)
                                    nums(i);
                            if (i == 0 && *out[1])
                                    stars();
                            putline(i);
                            if (i == 0 && *out[1])
                                    fprintf(fx, star);
                            if (i == 1)
                                    nums(i);
                    }
            }
    }


    /*
     * put out a number line: dumpblock()
     */
    static
    nums(ix)                                                                    nums
            int        ix;        /* index in out[ ] holding seq line */
    {
            char              nline[P_LINE];
            register          i, j;
            register char     *pn, *px, *py;

            for (pn = nline, i = 0; i < lmax+P_SPC; i++, pn++)
                    *pn = ' ';
            for (i = nc[ix], py = out[ix]; *py; py++, pn++) {
                    if (*py == ' ' || *py == '-')
                            *pn = ' ';
                    else {
                            if (i%10 == 0 || (i == 1 && nc[ix] != 1)) {
                                    j = (i < 0)? -i : i;
                                    for (px = pn; j; j /= 10, px--)
                                            *px = j%10 + '0';
                                    if (i < 0)
                                            *px = '-';
                            }
                            else
                                    *pn = ' ';
                            i++;
                    }
            }
            *pn = '\0';
            nc[ix] = i;
            for (pn = nline; *pn; pn++)
                    (void) putc(*pn, fx);
            (void) putc('\n', fx);
    }


    /*
     * put out a line (name, [num], seq, [num]): dumpblock()
     */
    static
    putline(ix)                                                                 putline
            int        ix;                                {
```

## Table 1 (cont')

...putline

```
            int            i;
            register char  *px;

            for (px = namex[ix], i = 0; *px && *px != ':'; px++, i++)
                    (void) putc(*px, fx);
            for (; i < lmax+P_SPC; i++)
                    (void) putc(' ', fx);

            /* these count from 1:
             * ni[ ] is current element (from 1)
             * nc[ ] is number at start of current line
             */
            for (px = out[ix]; *px; px++)
                    (void) putc(*px&0x7F, fx);
            (void) putc('\n', fx);
    }


    /*
     * put a line of stars (seqs always in out[0], out[1]): dumpblock()
     */
    static
    stars()

        stars

    {
            int            i;
            register char  *p0, *p1, cx, *px;

            if (!*out[0] || (*out[0] == ' ' && *(po[0]) == ' ') ||
                !*out[1] || (*out[1] == ' ' && *(po[1]) == ' '))
                    return;
            px = star;
            for (i = lmax+P_SPC; i; i--)
                    *px++ = ' ';

            for (p0 = out[0], p1 = out[1]; *p0 && *p1; p0++, p1++) {
                    if (isalpha(*p0) && isalpha(*p1)) {

                            if (xbm[*p0-'A']&xbm[*p1-'A']) {
                                    cx = '*';
                                    nm++;
                            }
                            else if (!dna && _day[*p0-'A'][*p1-'A'] > 0)
                                    cx = '.';
                            else
                                    cx = ' ';
                    }
                    else
                            cx = ' ';
                    *px++ = cx;
            }
            *px++ = '\n';
            *px = '\0';
    }
```

47

## Table 1 (cont')

```
/*
 * strip path or prefix from pn, return len: pr_align()
 */
static
stripname(pn)

stripname
        char      *pn;        /* file name (may be path) */
{

        register char      *px, *py;

        py = 0;
        for (px = pn; *px; px++)
                if (*px == '/')
                        py = px + 1;
        if (py)
                (void) strcpy(pn, py);
        return(strlen(pn));

}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";        /* tmp file for jmps */
FILE    *fj;

int     cleanup();                          /* cleanup tmp file */
long    lseek();

/*
 * remove any tmp file if we blow
 */
cleanup(i)                                                          cleanup
        int     i;
{
        if (fj)
                (void) unlink(jname);
        exit(i);
}


/*
 * read, return ptr to seq, set dna, len, maxlen
 * skip lines starting with ';', '<', or '>'
 * seq in upper or lower case
 */
char    *
getseq(file, len)                                                   getseq
        char    *file;    /* file name */
        int     *len;     /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

## Table 1 (cont')

...getseq

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}


char    *
g_calloc(msg, nx, sz)                                               g_calloc
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}


/*
 * get final jmps from dx[ ] or tmp file, set pp[ ], reset dmax: main()
 */
readjmps()
                readjmps
{
        int             fd = -1;
        int             siz, i0, i1;
        register  i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

## Table 1 (cont')

...readjmps

```
                if (j < 0 && dx[dmax].offset && fj) {
                        (void) lseek(fd, dx[dmax].offset, 0);
                        (void) read(fd, (char *)&dx[dmax].jp, sizeof(struct jmp));
                        (void) read(fd, (char *)&dx[dmax].offset, sizeof(dx[dmax].offset));
                        dx[dmax].ijmp = MAXJMP-1;
                }
                else
                        break;
        }
        if (i > = JMPS) {
                fprintf(stderr, "%s: too many gaps in alignment\n", prog);
                cleanup(1);
        }
        if (j > = 0) {
                siz = dx[dmax].jp.n[j];
                xx = dx[dmax].jp.x[j];
                dmax + = siz;
                if (siz < 0) {                 /* gap in second seq */
                        pp[1].n[i1] = -siz;
                        xx + = siz;
                        /* id = xx - yy + len1 - 1
                        */
                        pp[1].x[i1] = xx - dmax + len1 - 1;
                        gapy++;
                        ngapy -= siz;
/* ignore MAXGAP when doing endgaps */
                        siz = (-siz < MAXGAP || endgaps)? -siz : MAXGAP;
                        i1++;
                }
                else if (siz > 0) {  /* gap in first seq */
                        pp[0].n[i0] = siz;
                        pp[0].x[i0] = xx;
                        gapx++;
                        ngapx += siz;
/* ignore MAXGAP when doing endgaps */
                        siz = (siz < MAXGAP || endgaps)? siz : MAXGAP;
                        i0++;
                }
        }
        else
                break;
}

/* reverse the order of jmps
*/
for (j = 0, i0--; j < i0; j++, i0--) {
        i = pp[0].n[j]; pp[0].n[j] = pp[0].n[i0]; pp[0].n[i0] = i;
        i = pp[0].x[j]; pp[0].x[j] = pp[0].x[i0]; pp[0].x[i0] = i;
}
for (j = 0, i1--; j < i1; j++, i1--) {
        i = pp[1].n[j]; pp[1].n[j] = pp[1].n[i1]; pp[1].n[i1] = i;
        i = pp[1].x[j]; pp[1].x[j] = pp[1].x[i1]; pp[1].x[i1] = i;
}
if (fd > = 0)
        (void) close(fd);
if (fj) {
        (void) unlink(jname);
        fj = 0;
        offset = 0;
}                                       }
```

## Table 1 (cont')

```
/*
 * write a filled jmp struct offset of the prev one (if any): nw()
 */
writejmps(ix)
writejmps
int     ix;
{
    char    *mktemp();

    if (!fj) {
        if (mktemp(jname) < 0) {
            fprintf(stderr, "%s: can't mktemp() %s\n", prog, jname);
            cleanup(1);
        }
        if ((fj = fopen(jname, "w")) == 0) {
            fprintf(stderr, "%s: can't write %s\n", prog, jname);
            exit(1);
        }
    }
    (void) fwrite((char *)&dx[ix].jp, sizeof(struct jmp), 1, fj);
    (void) fwrite((char *)&dx[ix].offset, sizeof(dx[ix].offset), 1, fj);
}
```

### Table 2

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =
(the number of identically matching amino acid residues between the two polypeptide sequences as determined by ALIGN-2) divided by (the total number of amino acid residues of the PRO polypeptide) =
5 divided by 15 = 33.3%

### Table 3

| | | |
|---|---|---|
| PRO | XXXXXXXXXX | (Length = 10 amino acids) |
| Comparison Protein | XXXXXYYYYYYYZZYZ | (Length = 15 amino acids) |

% amino acid sequence identity =
(the number of identically matching amino acid residues between the two polypeptide sequences as determined by ALIGN-2) divided by (the total number of amino acid residues of the PRO polypeptide) =
5 divided by 10 = 50%

### Table 4

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
6 divided by 14 = 42.9%

EP 2 444 409 A2

#### Table 5

| PRO-DNA | NNNNNNNNNNNN | (Length = 12 nucleotides) |
|---|---|---|
| Comparison DNA | NNNNLLLVV | (Length = 9 nucleotides) |

% nucleic acid sequence identity =
(the number of identically matching nucleotides between the two nucleic acid sequences as determined by
ALIGN-2) divided by (the total number of nucleotides of the PRO-DNA nucleic acid sequence) =
4 divided by 12 = 33.3%

II. Compositions and Methods of the Invention

A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been disclosed. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science 244: 1081-1085 (1989)]. Alanine is also typically preferred because it is the most common amino acid. Further, it is frequently found in both buried and exposed positions [Creighton, The Proteins, (W.H.

Freeman & Co., N.Y.); Chothia, J. Mol. Biol., 150:1 (1976)]. If alanine substitution does not yield adequate amounts of variant, an isoteric amino acid can be used.

C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl-terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}$P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan$^r$; E. coli* W3110 strain 37D6, which has the complete genotype *tonA ptr3 phoA E15 (argF-lac)169 degP ompT rbs7 ilvG kan$^r$; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp1* gene present in the yeast plasmid YRp7 [Stinchcomb et al., Nature, 282:39 (1979); Kingsman et al., Gene, 7:141 (1979); Tschemper et al., Gene, 10: 157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275: 615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg. 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology 182 (1990); Scopes, Protein Purification: Principles and Practice, Springer-Verlag, New York (1982). The purification step(s) selected will depend, for example, on the nature of the production process used and the particular PRO produced.

E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.*, US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate B-cell proliferation or Ig production. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.,* Small *et al., Mol. Cell. Biol.* 5: 642-648 [1985]).

A cell based assay for B cells involves incubation of B cells with test polypeptides thought to be inhibitory of IgE production. The amount of inhibition by test polypeptides is compared with IgE production of B cells inhibited by E25 antibody. Human primary PBMCs (1 x 10e6 cell/mL- 1 mL final) are isolated and incubated at 37°C. On Day 1, PMBCs (500 ul- 2 x 10e6/mL) in assay medium containing IL-4 [20 ng/mL] and anti-CD40 [100 ng/mL] are combined with 500 ul test polypeptide (2X desired final concentration) into wells. Currently assay is 24 well with a 1 mL volume. Media is PSO4 with 15% horse serum (Intergen, Atlanta GA), 100 units/mL penicillin with 100 mg/mL streptomycin (Gibco, Gaithersburg MD), and 200 mM glutamine. On Day 14 cells are centrifuged and supernatant removed for quantitation of IgE. The quantity of IgE is determined by ELISA. A test polypeptide is considered positive if IgE synthesis is decreased by greater than 50% and/or 50% of maximum inhibition by E25. The test polypeptides are run in singlet and the IgE ELISA is run in duplicate for each well.

On the other hand, PRO polypeptides, as well as other compounds of the invention, which are direct inhibitors of B cell proliferation/activation and/or Ig secretion can be directly used to suppress the immune response. These compounds are useful to reduce the degree of the immune response and to treat immune related diseases characterized by a hyperactive, superoptimal, or autoimmune response. The use of compound which suppress Ig production would be expected to reduce inflammation. Such uses would be beneficial in treating conditions associated with excessive inflammation.

Alternatively, compounds, *e.g.*, antibodies, which bind to stimulating PRO polypeptides and block the stimulating effect of these molecules produce a net inhibitory effect and can be used to suppress the B cell mediated immune response by inhibiting B cell proliferation/activation, lymphokine secretion and/or Ig secretion. Blocking the stimulating effect of the polypeptides suppresses the immune response of the mammal.

H. Animal Models

The results of cell based in vitro assays can be further verified using *in vivo* animal models and assays for B-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e.g.*, murine models. Such models can be generated by introducing cells into syngeneic mice using standard techniques, *e.g.*, subcutaneous injection, tail vein injection, spleen implantation, intraperitoneal implantation, implantation under the renal capsule, *etc.*

An animal model of Systemic Lupus Erythematosus (SLE) was developed specifically for studying this disease. The NZB mouse was the first strain to be described and is the one most like SLE. Female NZB mice develop kidney lesions and hemolytic anemia and produce anti-DNA antibodies, much like SLE in humans. The B cells of these mice are extremely responsive to antigens and cytokines and this abnormal sensitivity has been proposed for the immunologic aberrancy in these mice.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines *(e.g.,* Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the B cell proliferation, stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.*, Thomas and Capecchi, Cell, 51:503 (1987) for a description of homologous recombination vectors]. The vector is introduced into an embryonic stem cell line (*e.g.,* by electroporation) and cells in which the introduced DNA has homologously recombined with the endogenous DNA are selected [see *e.g.,* Li et al., Cell, 69:915 (1992)]. The selected cells are then injected into a blastocyst of an animal (*e.g.,* a mouse or rat) to form aggregation chimeras [see *e.g.*, Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

I. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly-and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the polypeptide and drying. Alternatively, an immobilized antibody, *e.g.*, a monoclonal antibody, specific for the polypeptide to be immobilized can be used to anchor it to a solid surface. The assay is performed by adding the non-immobilized component, which may be labeled by a detectable label, to the immobilized component, *e.g.,* the coated surface containing the anchored component. When the reaction is complete, the non-reacted components are removed, *e.g.,* by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

J. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, B cell proliferation/activation, lymphokine release, or Ig production.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g.*, between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.*, Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e.g.*, PCT publication No. WO 97/33551, *supra.*

These molecules can be identified by any or any combination of the screening assays discussed above and/or by any other screening techniques well known for those skilled in the art.

K. Anti-PRO Antibodies

The present invention further provides anti-PRO antibodies. Exemplary antibodies include polyclonal, monoclonal, humanized, bispecific, and heteroconjugate antibodies.

1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')$_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')$_2$ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')$_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')$_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al. , Proc. Natl. Acad. Sci. USA 90:6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al. , J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

7. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}$Bi, $^{131}$I, $^{131}$In, $^{90}$Y, and $^{186}$Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.,* a radionucleotide).

8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

L. Pharmaceutical Compositions

The active PRO molecules of the invention (*e.g.*, PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers *(*Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (*e.g.*, Zn-protein complexes); and/or non-ionic surfactants such as TWEEN™, PLURONICS™ or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.,* Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

M. Methods of Treatment

It is contemplated that the polypeptides, antibodies and other active compounds of the present invention may be used to treat various immune related diseases and conditions, such as B cell mediated diseases, including those characterized by stimulation of B-cell proliferation, inhibition of B-cell proliferation, increased or decreased Ig production or the inhibition thereof.

Exemplary conditions or disorders to be treated with the polypeptides, antibodies and other compounds of the invention, include, but are not limited to: systemic lupus erythematosis, X-linked infantile hypogammaglobulinemia, polysaccharide antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

In systemic lupus erythematosus (SLE), the central mediator of disease is the production of auto-reactive antibodies to self proteins/tissues and the subsequent generation of immune-mediated inflammation. Antibodies either directly or indirectly mediate tissue injury. Multiple organs and systems that are affected clinically include kidney, lung, musculoskeletal system, mucocutaneous, eye, central nervous system, cardiovascular system, gastrointestinal tract, bone marrow and blood.

In patients with X-linked infantile hypogammaglobulinemia, the B cells have a deficient kinase which leads to a lack of differentiation from the pre-B cell stage. The consequences of this is that these cells do not secrete immunoglobulin. Children with this disease usually show no symptoms until 6 months of age, an age which corresponds to the loss of maternal antibodies. Symptoms consist of pneumonia, meningitis, dermatitis with some instances of arthritis and malabsorption. Treatment at this time involves the use of intravenous gamma globulin replacement therapy.

Other diseases in which intervention of the immune and/or inflammatory response have benefit are infectious disease including but not limited to viral infection (including but not limited to Epstein-Barr virus) which stimulate the proliferation/Ig secretion of B-cells can be utilized therapeutically to enhance the immune response to infectious agents, diseases of immunodeficiency (molecules/derivatives/agonists) which stimulate B-cell proliferation/Ig secretion can be utilized therapeutically to enhance the immune response for conditions of inherited, acquired, infectious induced (as in HIV infection), or iatrogenic (*i.e.*, as from chemotherapy) immunodeficiency, and neoplasia.

B-cell leukemias can be treated by antibodies against surface proteins. This is illustrated in a regimen using antibodies to CD9 or CD10 which are often expressed at high levels in B-cell leukemias. Bone marrow is removed from patients with this type of leukemia and is treated with toxin-conjugated anti-CD9/anti-CD10, while the patient is treated with high doses of chemotherapy or radiation therapy. The treated marrow now devoid of leukemic cells, is reintroduced into the patient to repopulate the hematopoeitic lineage.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g.*, polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 $\mu$g/kg to 15 mg/kg (*e.g.*, 0.1-20 mg/kg) of polypeptide or antibody is an initial candidate dosage for administration to the patient, whether, for example, by one or more separate administrations, or by continuous infusion. A typical daily dosage might range from about 1 $\mu$g/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

N. Articles of Manufacture

In another embodiment of the invention, an article of manufacture containing materials (*e.g.*, comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

O. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, *e.g.*, fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

EXAMPLE 1: Microarray analysis of stimulated B-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this example, stimulated B cells) sample is greater than hybridization signal of a probe from a control (in this instance, non-stimulated B cells) sample, the gene or genes overexpressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In this experiment, primary B cells were isolated from peripheral blood provided by 3 normal male donors. B cells were isolated by negative selection using the B Cell Isolation Kit with the MACS™ magnetic cell sorting system (Miltenyi Biotec, Auburn CA). The cell purity was determined by fluorescence antibody staining with anti-CD19 vs isotype antibody control and subsequent FACS analysis to determine purity. The purity of the B cell population was above 90% for each donor.

The isolated cells were suspended in RPMI1640 media supplemented with 10% FBS, 2 mM L-glutamine, 55 mM 2-ME, 100 units/mL of Penicillin, 100 mg/mL of streptomycin. Cells were cultured at a density of $3 \times 10^5$ cells/mL in 5 mL/well in 6 well FALCON™ polystyrene tissue culture plates. Cells were cultured for 23 hours at 37°C either in the presence and absence of anti-CD40 (10mg/mL) and IL-4 (100 ng/mL). The immune competence of the isolated B cells to respond to stimulation by anti-CD40/IL-4 was determined by induction of expression of the cell surface protein, CD69. The increase in expression of CD69 was monitored at a 0 timepoint and 23 hours after culture with anti-CD40/IL-4, using fluorescence staining with anti-CD69 antibodies.

Total RNA was extracted from the cultured B cells at the 0 timepoint and at 23 hours with and without the anti-CD40/IL-4 stimulation using the Qiagen Rneasy Maxi Kit™. The RNA was extracted from columns treated with DNAse I as per Qiagen protocol and eluted using DEPC treated water. The extracted RNA was run on Affimax (Affymetrix Inc. Santa Clara, CA) microarrays. Non-stimulated cells harvested at the 0 timepoint were subjected to the same analysis. Genes were compared whose expression was upregulated at the 23 hour timepoint in stimulated vs. non-stimulated cells.

Below are the results of these experiments, demonstrating that various PRO polypeptides of the present invention are significantly overexpressed in isolated B cells stimulated by anti-CD40/IL-4 as compared to isolated, non-stimulated B cells. As described above, these data demonstrate that the PRO polypeptides of the present invention are useful not only as diagnostic markers for the presence of one or more immune disorders, but also serve as therapeutic targets for the treatment of those immune disorders.

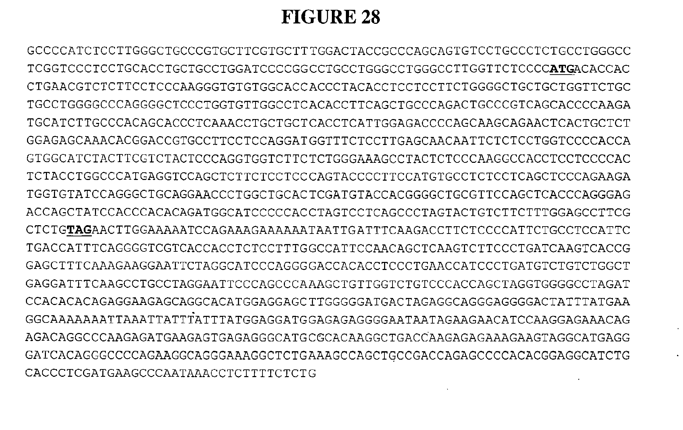

The nucleic acids in Figure 1 (SEQ ID NO:1), Figure 2A-B (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36A-B (SEQ ID NO:36), Figure 38 (SEQ ID NO:38), Figure 40 (SEQ, ID NO:40), Figure 42 (SEQ ID NO:42), Figure 44A-B (SEQ ID NO:44), Figure 46A-B (SEQ ID NO:46), Figure 48 (SEQ ID NO:48), Figure 50A-B (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72A-B (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94A-B (SEQ ID NO:94), Figure 96 (SEQ ID NO:96), Figure 98 (SEQ ID NO:98), Figure 100 (SEQ ID NO:100), Figure 102 (SEQ ID NO: 102), Figure 104 (SEQ ID NO:104), Figure 106 (SEQ ID NO:106), Figure 108 (SEQ ID NO:108), Figure 110A-B (SEQ ID NO:110), Figure 112 (SEQ ID NO:112), Figure 114 A-B (SEQ ID NO:114), Figure 116 (SEQ ID NO:116), Figure 118 (SEQ ID NO:118), Figure 120 (SEQ ID NO:120), Figure 122A-B (SEQ ID NO:122), Figure 124A-B (SEQ ID NO:124), Figure 126 (SEQ ID NO:126), Figure 128A-B (SEQ ID NO: 128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144A-B (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156A-B (SEQ ID NO:156), Figure 158 (SEQ ID NO:158), Figure 160 (SEQ ID NO:160), Figure 162 (SEQ ID NO:162), Figure 164 (SEQ ID NO:164), Figure 166A-B (SEQ ID NO: 166), Figure 168A-B (SEQ ID NO:168), Figure 170 (SEQ ID NO:170), Figure 172 (SEQ ID NO:172), Figure 174A-B (SEQ ID NO:174), Figure 176 (SEQ ID NO:176), Figure 178A-B (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190A-B (SEQ ID NO:190), Figure 192A-B (SEQ ID NO:192), Figure 194 (SEQ ID NO:194), Figure 196 (SEQ ID NO: 196), Figure 198 (SEQ ID NO:198), Figure 200A-B (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216A-B (SEQ ID NO:216), Figure 218 (SEQ ID NO:218), Figure 220A-B (SEQ ID NO:220), Figure 222 (SEQ ID NO:222), Figure 223 (SEQ ID NO:223), Figure 224 (SEQ ID NO:224), Figure 226 (SEQ ID NO:226), Figure 228 (SEQ ID NO:228), Figure 230 (SEQ ID NO:230), Figure 231 (SEQ ID NO:231), Figure 233 (SEQ ID NO:233), Figure 234 (SEQ ID NO:234), Figure 236A-B (SEQ ID NO:236), Figure 238 (SEQ ID NO:238), Figure 240 (SEQ ID NO:240), Figure 241 (SEQ ID NO:241), Figure 242 (SEQ ID NO:242), Figure 244 (SEQ ID NO: 244), Figure 245 (SEQ ID NO:245), Figure 247A-B (SEQ ID NO:247), Figure 249 (SEQ ID NO:249), Figure 251 (SEQ ID NO:251), Figure 253 (SEQ ID NO:253), Figure 255 (SEQ ID NO:255), Figure 257 (SEQ ID NO:257), Figure 259 (SEQ ID NO:259), Figure 261 (SEQ ID NO:261), Figure 263 (SEQ ID NO:263), Figure 265 (SEQ ID NO:265), Figure 267 (SEQ ID NO:267), Figure 269 (SEQ ID NO:269), Figure 271 (SEQ ID NO:271), Figure 273 (SEQ ID NO:273), Figure 275 (SEQ ID NO:275), Figure 277 (SEQ ID NO:277), Figure 279 (SEQ ID NO:279), Figure 281 (SEQ ID NO:281), Figure 283 (SEQ ID NO:283), Figure 285 (SEQ ID NO:285), Figure 287 (SEQ ID NO:287), Figure 289A-B (SEQ ID NO:289), Figure 291 (SEQ ID NO:291), Figure 293A-B (SEQ ID NO:293), Figure 295 (SEQ ID NO:295), Figure 297 (SEQ ID NO:297), Figure 299A-B (SEQ ID NO:299), Figure 301 (SEQ ID NO:301), Figure 303A-B (SEQ ID NO:303), Figure 305A-B (SEQ ID NO: 305), Figure 307 (SEQ ID NO:307), Figure 309 (SEQ ID NO:309), Figure 311A-D (SEQ ID NO:311), Figure 313 (SEQ ID NO:313), Figure 315A-B (SEQ ID NO:315), Figure 317 (SEQ ID NO:317), Figure 319A-B (SEQ ID NO:319), Figure 321 (SEQ ID NO:321), Figure 322 (SEQ ID NO:322), Figure 323 (SEQ ID NO:323), Figure 325 (SEQ ID NO:325), Figure 327 (SEQ ID NO:327), Figure 328 (SEQ ID NO:328), Figure 330 (SEQ ID NO:330), Figure 331 (SEQ ID NO:331), Figure 333 (SEQ ID NO:333), Figure 335A-B (SEQ ID NO:335), Figure 337 (SEQ ID NO:337), Figure 339 (SEQ ID NO:339), Figure 341 (SEQ ID NO:341), Figure 343 (SEQ ID NO:343), Figure 345 (SEQ ID NO:345), Figure 347 (SEQ ID NO: 347), Figure 349 (SEQ ID NO:349), Figure 351 (SEQ ID NO:351), Figure 353 (SEQ ID NO:353), Figure 355 (SEQ ID NO:355), Figure 357 (SEQ ID NO:357), Figure 359 (SEQ ID NO:359), Figure 361A-B (SEQ ID NO:361), Figure 363 (SEQ ID NO:363), Figure 365 (SEQ ID NO:365), Figure 367A-C (SEQ ID NO:367), Figure 369 (SEQ ID NO:369), Figure 371 (SEQ ID NO:371), Figure 373A-B (SEQ ID NO:373), Figure 374 (SEQ ID NO:374), Figure 376 (SEQ ID NO:376), Figure 378 (SEQ ID NO:378), Figure 380 (SEQ ID NO:380), Figure 382 (SEQ ID NO:382), Figure 384A-B (SEQ ID NO: 384), Figure 386 (SEQ ID NO:386), Figure 388 (SEQ ID NO:388), Figure 390 (SEQ ID NO:390), Figure 391 (SEQ ID NO:391), Figure 393 (SEQ ID NO:393), Figure 395A-B (SEQ ID NO:395), Figure 397 (SEQ ID NO:397), Figure 398A-B (SEQ ID NO:398), Figure 400 (SEQ ID NO:400), Figure 401 (SEQ ID NO:401), Figure 402A-B (SEQ ID NO:402), Figure 404 (SEQ ID NO:404), Figure 406 (SEQ ID NO:406), Figure 407 (SEQ ID NO:407), Figure 408 (SEQ ID NO: 408), Figure 409 (SEQ ID NO:409), Figure 410 (SEQ ID NO:410), Figure 412 (SEQ ID NO:412), Figure 414 (SEQ ID NO:414), Figure 416 (SEQ ID NO:416), Figure 418 (SEQ ID NO:418), Figure 420A-B (SEQ ID NO:420), Figure 422 (SEQ ID NO:422), Figure 424A-B (SEQ ID NO:424), Figure 426 (SEQ ID NO:426), Figure 428 (SEQ ID NO:428), Figure 430 (SEQ ID NO:430), Figure 432 (SEQ ID NO:432), Figure 434 (SEQ ID NO:434), Figure 436A-B (SEQ ID NO:436), Figure 438A-B (SEQ ID NO:438), Figure 440A-B (SEQ ID NO:440), Figure 442A-B (SEQ ID NO:442), Figure 444A-B (SEQ ID NO:444), Figure 446A-B (SEQ ID NO:446), Figure 448 (SEQ ID NO:448), Figure 450A-B (SEQ ID NO:450), Figure 452 (SEQ ID NO:452), Figure 454 (SEQ ID NO:454), Figure 456 (SEQ ID NO:456), Figure 458 (SEQ ID NO: 458), Figure 459 (SEQ ID NO:459), Figure 461 (SEQ ID NO:461), Figure 463A-B (SEQ ID NO:463), Figure 465 (SEQ ID NO:465), Figure 467 (SEQ ID NO:467), Figure 469A-B (SEQ ID NO:469), Figure 470A-B (SEQ ID NO:470), Figure 471A-C (SEQ ID NO:471), Figure 473 (SEQ ID NO:473), Figure 475 (SEQ ID NO:475), Figure 477 (SEQ ID NO:477), Figure 479 (SEQ ID NO:479), Figure 481A-B (SEQ ID NO:481), Figure 483 (SEQ ID NO:483), Figure 485 (SEQ ID NO: 485), Figure 487 (SEQ ID NO:487), Figure 489 (SEQ ID NO:489), Figure 491A-B (SEQ ID NO:491), Figure 493 (SEQ ID NO:493), Figure 495 (SEQ ID NO:495), Figure 497 (SEQ ID NO:497), Figure 499 (SEQ ID NO:499), Figure 501 (SEQ ID NO:501), Figure 503 (SEQ ID NO:503) and Figure 505 (SEQ ID NO:505) are upregulated upon stimulation with anti-CD40/IL-4.

EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50 % formamide, 5x SSC, 0.1 % SDS, 0.1 % sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1 % SDS at 42°C.

DNAs having a desired sequence identity with the DNA encoding full-length native sequence PRO can then be identified using standard techniques known in the art.

EXAMPLE 3: Expression of PRO in *E. coli*

This example illustrates preparation of an unglycosylated form of PRO by recombinant expression in *E. coli*.

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) Ion galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate•2H2O, 1.07 g KCl, 5 .36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-PRO DNA is mixed with about 1 μg DNA encoding the VA RNA gene [Thimmappaya et al., Cell, 31:543 (1982)] and dissolved in 500 μl of 1 mM Tris-HCl, 0.1 mM EDTA, 0.227 M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 $\mu$Ci/ml $^{35}$S-cysteine and 200 $\mu$Ci/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 $\mu$g pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 $\mu$g/ml bovine insulin and 0.1 $\mu$g/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by $Ni^{2+}$-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Quiagen), Dosper® or Fugene ® (Boehringer Mannheim). The cells are grown as described in Lucas et al., supra. Approximately 3 x 10[-7] cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 $\mu$m filtered PS20 with 5% 0.2 $\mu$m diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2'-3 days, 250 mL, 500 mL and 2000 mL spinners are seeded with 3 x 10[5] cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10[6] cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Coming 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 $\mu$m filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 $\mu$l of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin '(commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by $Ni^{2+}$-chelate affinity chromatography as follows. Extracts are prepared from recombinant virus-infected Sf9 cells as described by Rupert et al., Nature, 362: 175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1 % NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading

buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO are pooled and dialyzed against loading buffer.

Alternatively, purification of the IgG tagged (or Fc tagged) PRO can be performed using known. chromatography techniques, including for instance, Protein A or protein G column chromatography.

Many of the PRO polypeptides disclosed herein were successfully expressed as described above.

EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms.

Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35 % polyethylene glycol) to a selected murine myeloma cell line such as P3X63Ag U.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed,

EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g., high ionic strength buffers in the presence of detergent). Then, the column is eluted under conditions that disrupt antibody/PRO polypeptide binding (e.g., a low pH buffer such as approximately pH 2-3, or a high concentration of a chaotrope such as urea or thiocyanate ion), and PRO polypeptide is collected.

EXAMPLE 9: Drugs Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

EXAMPLE 10: Rational Drug Design

The goal of rational drug design is to produce structural analogs of biologically active polypeptide of interest (i.e., a PRO polypeptide) or of small molecules with which they interact, e.g., agonists, antagonists, or inhibitors. Any of these examples can be used to fashion drugs which are more active or stable forms of the PRO polypeptide or which enhance or interfere with the function of the PRO polypeptide *in vivo* (*c.f.*, Hodgson, Bio/Technology, 9: 19-21 (1991)).

In one approach, the three-dimensional structure of the PRO polypeptide, or of a PRO polypeptide-inhibitor complex, is determined by'x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

The following numbered paragraphs (paras.) contain further statements of various aspects of the present invention:-

1. Isolated nucleic acid having at least 80% nucleic acid sequence identity to:

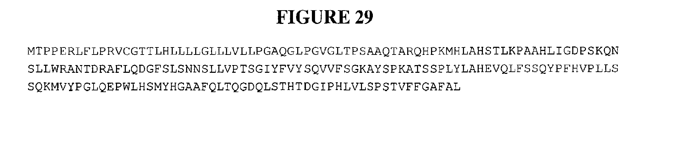

a nucleotide sequence encoding the polypeptide shown in Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEQ ID NO:113), Figure 115 (SEQ ID NO:115), Figure 117 (SEQ ID NO:117), Figure 119 (SEQ ID NO:119), Figure 121 (SEQ ID NO:121), Figure 123 (SEQ ID NO: 123), Figure 125 (SEQ ID NO:125), Figure 127 (SEQ ID NO:127), Figure 129 (SEQ ID NO:129), Figure 131 (SEQ ID NO:131), Figure 133 (SEQ ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO: 159), Figure 161 (SEQ ID NO:161), Figure 163 (SEQ ID NO: 163), Figure 165 (SEQ ID NO: 165), Figure 167 (SEQ ID NO: 167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191), Figure 193 (SEQ ID NO: 193), Figure 195 (SEQ ID NO: 195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEQ ID NO:213), Figure 215 (SEQ ID NO:215, Figure 217 (SEQ ID NO:217), Figure 219 (SEQ ID NO:219), Figure 221 (SEQ ID NO:221), Figure 225 (SEQ ID NO:225), Figure 227 (SEQ ID NO:227), Figure 229 (SEQ ID NO:229), Figure 232 (SEQ ID NO:232), Figure 235 (SEQ ID NO: 235), Figure 237 (SEQ ID NO:237), Figure 239 (SEQ ID NO:239), Figure 243 (SEQ ID NO:243), Figure 246 (SEQ ID NO:246), Figure 248 (SEQ ID NO:248), Figure 250 (SEQ ID NO:250), Figure 252 (SEQ ID NO:252), Figure 254 (SEQ ID NO:254), Figure 256 (SEQ ID NO:256), Figure 258 (SEQ ID NO:258), Figure 260 (SEQ ID NO:260), Figure 262 (SEQ ID NO:262), Figure 264 (SEQ ID NO:264), Figure 266 (SEQ ID NO:266), Figure 268 (SEQ ID NO:268), Figure 270 (SEQ ID NO:270), Figure 272 (SEQ ID NO:272), Figure 274 (SEQ ID NO: 274), Figure 276 (SEQ ID NO:276), Figure 278 (SEQ ID NO:278), Figure 280 (SEQ ID NO:280), Figure 282 (SEQ ID NO:282), Figure 284 (SEQ ID NO:284), Figure 286 (SEQ ID NO:286), Figure 288 (SEQ ID NO:288), Figure 290 (SEQ ID NO:290), Figure 292 (SEQ ID NO:292), Figure 294 (SEQ ID NO:294), Figure 296 (SEQ ID NO:296), Figure 298 (SEQ ID NO:298), Figure 300 (SEQ ID NO:300), Figure 302 (SEQ ID NO:302), Figure 304 (SEQ ID NO:304), Figure 306 (SEQ ID NO:306), Figure 308 (SEQ ID NO:308), Figure 310 (SEQ ID NO: 310), Figure 312A-B (SEQ ID NO:312), Figure 314 (SEQ ID NO:314), Figure 316 (SEQ ID NO:316), Figure 318 (SEQ ID NO:318), Figure 320 (SEQ ID NO:320), Figure 324 (SEQ ID NO:324), Figure 326 (SEQ ID NO:326), Figure 329 (SEQ ID NO:329), Figure 332 (SEQ ID NO:332), Figure 334 (SEQ ID NO:334), Figure 336 (SEQ ID NO:336), Figure 338 (SEQ ID NO:338), Figure 340 (SEQ ID NO:340), Figure 342 (SEQ ID NO:342), Figure 344 (SEQ ID NO:344), Figure 346 (SEQ ID NO:346), Figure 348 (SEQ ID NO:348), Figure 350 (SEQ ID NO: 350), Figure 352 (SEQ ID NO:352), Figure 354 (SEQ ID NO:354), Figure 356 (SEQ ID NO:356), Figure 358 (SEQ ID NO:358), Figure 360 (SEQ ID NO:360), Figure 362 (SEQ ID NO:362), Figure 364 (SEQ ID NO:364), Figure 366 (SEQ ID NO:366), Figure 368 (SEQ ID NO:368), Figure 370 (SEQ ID NO:370), Figure 372 (SEQ ID NO:372), Figure 375 (SEQ ID NO:375), Figure 377 (SEQ ID NO:377), Figure 379 (SEQ ID NO:379), Figure 381 (SEQ ID NO:381), Figure 383 (SEQ ID NO:383), Figure 385 (SEQ ID NO:385), Figure 387 (SEQ ID NO: 387), Figure 389 (SEQ ID NO:389), Figure 392 (SEQ ID NO:392), Figure 394 (SEQ ID NO:394), Figure 396 (SEQ ID NO:396), Figure 399 (SEQ ID NO:399), Figure 403 (SEQ ID NO:403), Figure 405 (SEQ ID NO:405), Figure 411 (SEQ ID N0:411), Figure 413 (SEQ ID NO:413), Figure 415 (SEQ ID NO:415), Figure 417 (SEQ ID NO:417), Figure 419 (SEQ ID NO:419), Figure 421 (SEQ ID NO:42 1), Figure 423 (SEQ ID NO:423), Figure 425 (SEQ ID NO:425), Figure 427 (SEQ ID NO:427), Figure 429 (SEQ ID NO:429), Figure 431 (SEQ ID N0: 431), Figure 433 (SEQ ID NO:433), Figure 435 (SEQ ID NO:435), Figure 437 (SEQ ID NO:437), Figure 439 (SEQ ID NO:439), Figure 441 (SEQ ID NO:441), Figure 443 (SEQ ID NO:443), Figure 445 (SEQ ID NO:445), Figure 447 (SEQ ID NO:447), Figure 449 (SEQ ID NO:449), Figure 451 (SEQ ID NO:451), Figure 453 (SEQ ID NO:453), Figure 455 (SEQ ID NO:455), Figure 457 (SEQ ID NO:457), Figure 460 (SEQ ID NO:460), Figure 462 (SEQ ID NO:462), Figure 464 (SEQ ID NO:464), Figure 466 (SEQ ID NO:466), Figure 468 (SEQ ID NO: 468), Figure 472 (SEQ ID NO:472), Figure 474 (SEQ ID NO:474), Figure 476 (SEQ ID NO:476), Figure 478 (SEQ ID NO:478), Figure 480 (SEQ ID NO:480), Figure 482 (SEQ ID NO:482), Figure 484 (SEQ ID NO:484), Figure 486 (SEQ ID NO:486), Figure 488 (SEQ ID NO:488), Figure 490 (SEQ ID NO:490), Figure 492 (SEQ ID NO:492), Figure 494 (SEQ ID NO:494), Figure 496 (SEQ ID NO:496), Figure 498 (SEQ ID NO:498), Figure 500 (SEQ ID NO:500), Figure 502 (SEQ ID NO:502), Figure 504 (SEQ ID NO:504) or Figure 506 (SEQ ID NO: 506).

2. Isolated nucleic acid having at least 80% nucleic acid sequence identity to a nucleotide sequence selected from the group consisting of the nucleotide sequence shown in Figure 1 (SEQ ID NO:1), Figure 2A-B (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36A-B (SEQ ID NO:36), Figure 38 (SEQ ID NO:38), Figure 40 (SEQ ID NO:40), Figure 42 (SEQ ID NO:42), Figure 44A-B (SEQ ID NO:44), Figure 46A-B (SEQ ID NO:46), Figure 48 (SEQ ID NO:48), Figure 50A-B (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72A-B (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94A-B (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO: 106), Figure 108 (SEQ ID NO:108), Figure 110A-B (SEQ ID NO:110), Figure 112 (SEQ ID NO:112), Figure 114 A-B (SEQ ID NO:114), Figure 116 (SEQ ID NO:116), Figure 118 (SEQ ID NO:118), Figure 120 (SEQ ID NO:120), Figure 122A-B (SEQ ID NO:122), Figure 124A-B (SEQ ID NO:124), Figure 126 (SEQ ID NO:126), Figure 128A-B (SEQ ID NO:128), Figure 130 (SEQ ID NO: 130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144A-B (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156A-B (SEQ ID NO:156), Figure 158 (SEQ ID NO:158), Figure 160 (SEQ ID NO:160), Figure 162 (SEQ ID NO:162), Figure 164 (SEQ ID NO:164), Figure 166A-B (SEQ ID NO:166), Figure 168A-B (SEQ ID NO:168), Figure 170 (SEQ ID NO:170), Figure 172 (SEQ ID NO:172), Figure 174A-B (SEQ ID NO:174), Figure 176 (SEQ ID NO:176), Figure 178A-B (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190A-B (SEQ ID NO:190), Figure 192A-B (SEQ ID NO:192), Figure 194 (SEQ ID NO:194), Figure 196 (SEQ ID NO:196), Figure 198 (SEQ ID NO:198), Figure 200A-B (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216A-B (SEQ ID NO:216), Figure 218 (SEQ ID NO:218), Figure 220A-B (SEQ ID NO:220), Figure 222 (SEQ ID NO:222), Figure 223 (SEQ ID NO:223), Figure 224 (SEQ ID NO:224), Figure 226 (SEQ ID NO:226), Figure 228 (SEQ ID NO:228), Figure 230 (SEQ ID NO:230), Figure 231 (SEQ ID NO:23 1), Figure 233 (SEQ ID NO:233), Figure 234 (SEQ ID NO:234), Figure 236A-B (SEQ ID NO:236), Figure 238 (SEQ ID NO:238), Figure 240 (SEQ ID NO:240), Figure 241 (SEQ ID NO:241), Figure 242 (SEQ ID NO:242), Figure 244 (SEQ ID NO:244), Figure 245 (SEQ ID NO:245), Figure 247A-B (SEQ ID NO:247), Figure 249 (SEQ ID NO:249), Figure 251 (SEQ ID NO:251), Figure 253 (SEQ ID NO:253), Figure 255 (SEQ ID NO:255), Figure 257 (SEQ ID NO:257), Figure 259 (SEQ ID NO:259), Figure 261 (SEQ ID NO:261), Figure 263 (SEQ ID NO:263), Figure 265 (SEQ ID NO:265), Figure 267 (SEQ ID NO:267), Figure 269 (SEQ ID NO:269), Figure 271 (SEQ ID NO:271), Figure 273 (SEQ ID NO:273), Figure 275 (SEQ ID NO:275), Figure 277 (SEQ ID NO:277), Figure 279 (SEQ ID NO:279), Figure 281 (SEQ ID NO:281), Figure 283 (SEQ ID NO:283), Figure 285 (SEQ ID NO:285), Figure 287 (SEQ ID NO:287), Figure 289A-B (SEQ ID NO:289), Figure 291 (SEQ ID NO:291), Figure 293A-B (SEQ ID NO:293), Figure 295 (SEQ ID NO:295), Figure 297 (SEQ ID NO:297), Figure 299A-B (SEQ ID NO:299), Figure 301 (SEQ ID NO:301), Figure 303A-B (SEQ ID NO:303), Figure 305A-B (SEQ ID NO:305), Figure 307 (SEQ ID NO:307), Figure 309 (SEQ ID NO:309), Figure 311A-D (SEQ ID NO:311), Figure 313 (SEQ ID NO:313), Figure 315A-B (SEQ ID NO:315), Figure 317 (SEQ ID NO:317), Figure 319A-B (SEQ ID NO:319), Figure 321 (SEQ ID NO:321), Figure 322 (SEQ ID NO:322), Figure 323 (SEQ ID NO:323), Figure 325 (SEQ ID NO:325), Figure 327 (SEQ ID NO:327), Figure 328 (SEQ ID NO:328), Figure 330 (SEQ ID NO:330), Figure 331 (SEQ ID NO:331), Figure 333 (SEQ ID NO:333), Figure 335A-B (SEQ ID NO:335), Figure 337 (SEQ ID NO:337), Figure 339 (SEQ ID NO:339), Figure 341 (SEQ ID NO:341), Figure 343 (SEQ ID NO:343), Figure 345 (SEQ ID NO:345), Figure 347 (SEQ ID NO:347), Figure 349 (SEQ ID NO:349), Figure 351 (SEQ ID NO:351), Figure 353 (SEQ ID NO:353), Figure 355 (SEQ ID NO:355), Figure 357 (SEQ ID NO:357), Figure 359 (SEQ ID NO:359), Figure 361A-B (SEQ ID NO:361), Figure 363 (SEQ ID NO:363), Figure 365 (SEQ ID NO:365), Figure 367A-C (SEQ ID NO:367), Figure 369 (SEQ ID NO:369), Figure 371 (SEQ ID NO:371), Figure 373A-B (SEQ ID NO:373), Figure 374 (SEQ ID NO:374), Figure 376 (SEQ ID NO:376), Figure 378 (SEQ ID NO:378), Figure 380 (SEQ ID NO:380), Figure 382 (SEQ ID NO:382), Figure 384A-B (SEQ ID NO:384), Figure 386 (SEQ ID NO:386), Figure 388 (SEQ ID NO:388), Figure 390 (SEQ ID NO:390), Figure 391 (SEQ ID NO:391), Figure 393 (SEQ ID NO:393), Figure 395A-B (SEQ ID NO:395), Figure 397 (SEQ ID NO:397), Figure 398A-B (SEQ ID NO:398), Figure 400 (SEQ ID NO:400), Figure 401 (SEQ ID NO:401), Figure 402A-B (SEQ ID NO:402), Figure 404 (SEQ ID NO:404), Figure 406 (SEQ ID NO:406), Figure 407 (SEQ ID NO:407), Figure 408 (SEQ ID NO:408), Figure 409 (SEQ ID NO:409), Figure 410 (SEQ ID NO:410), Figure 412 (SEQ ID NO:412), Figure 414 (SEQ ID NO:414), Figure 416 (SEQ ID NO:416), Figure 418 (SEQ m NO:418), Figure 420A-B (SEQ ID NO:420), Figure 422 (SEQ ID NO:422), Figure 424A-B (SEQ ID NO:424), Figure 426 (SEQ ID NO:426), Figure 428 (SEQ ID NO:428), Figure 430 (SEQ ID NO:430), Figure 432 (SEQ ID NO:432), Figure 434 (SEQ ID NO:434), Figure 436A-B (SEQ ID NO:436), Figure 438A-B (SEQ ID NO:438), Figure 440A-B (SEQ ID NO:440), Figure 442A-B (SEQ ID NO:442), Figure 444A-B (SEQ ID NO:444), Figure 446A-B (SEQ ID NO:446), Figure 448 (SEQ ID NO :448), Figure 450A-B (SEQ ID NO:450), Figure 452 (SEQ ID NO:452), Figure 454 (SEQ ID NO:454), Figure 456 (SEQ ID NO:456), Figure 458 (SEQ ID NO:458), Figure 459 (SEQ ID NO:459), Figure 461 (SEQ ID NO:461), Figure 463A-B (SEQ ID NO:463), Figure 465 (SEQ ID NO:465), Figure 467 (SEQ ID NO:467), Figure 469A-B (SEQ ID NO:469), Figure 470A-B (SEQ ID NO:470), Figure 471A-C (SEQ ID NO:471), Figure 473 (SEQ ID NO:473), Figure 475 (SEQ ID NO:475), Figure 477 (SEQ ID NO:477), Figure 479 (SEQ ID NO:479), Figure 481A-B (SEQ ID NO:481), Figure 483 (SEQ ID NO:483), Figure 485 (SEQ ID NO:485), Figure 487 (SEQ ID NO:487), Figure 489 (SEQ ID NO:489), Figure 491A-B (SEQ ID NO:491), Figure 493 (SEQ ID NO:493), Figure 495 (SEQ ID NO:495), Figure 497 (SEQ ID NO:497), Figure 499 (SEQ ID NO:499), Figure 501 (SEQ ID NO:501), Figure 503 (SEQ ID NO:503) and Figure 505 (SEQ ID NO:505)

3. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 2A-B (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36A-B (SEQ ID NO:36), Figure 38 (SEQ ID NO:38), Figure 40 (SEQ ID NO:40), Figure 42 (SEQ ID NO:42), Figure 44A-B (SEQ ID NO:44), Figure 46A-B (SEQ ID NO:46), Figure 48 (SEQ ID NO:48), Figure 50A-B (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72A-B (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94A-B (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110A-B (SEQ ID NO:110), Figure 112 (SEQ ID NO:112), Figure 114 A-B (SEQ ID NO:114), Figure 116 (SEQ ID NO:116), Figure 118 (SEQ ID NO:118), Figure 120 (SEQ ID NO:120), Figure 122A-B (SEQ ID NO:122), Figure 124A-B (SEQ ID NO:124), Figure 126 (SEQ ID NO:

126), Figure 128A-B (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144A-B (SEQ ID NO:144), Figure 146 (SEQ ID NO:146), Figure 148 (SEQ ID NO: 148), Figure 150 (SEQ ID NO:150), Figure 152 (SEQ ID NO:152), Figure 154 (SEQ ID NO:154), Figure 156A-B (SEQ ID NO:156), Figure 158 (SEQ ID NO:158), Figure 160 (SEQ ID NO:160), Figure 162 (SEQ ID NO:162), Figure 164 (SEQ ID NO:164), Figure 166A-B (SEQ ID NO:166), Figure 168A-B (SEQ ID NO:168), Figure 170 (SEQ ID NO:170), Figure 172 (SEQ ID NO:172), Figure 174A-B (SEQ ID NO:174), Figure 176 (SEQ ID NO:176), Figure 178A-B (SEQ ID NO:178), Figure 180 (SEQ ID NO:180), Figure 182 (SEQ ID NO:182), Figure 184 (SEQ ID NO: 184), Figure 186 (SEQ ID NO:186), Figure 188 (SEQ ID NO:188), Figure 190A-B (SEQ ID NO:190), Figure 192A-B (SEQ ID NO:192), Figure 194 (SEQ ID NO:194), Figure 196 (SEQ ID NO:196), Figure 198 (SEQ ID NO:198), Figure 200A-B (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216A-B (SEQ ID NO:216), Figure 218 (SEQ ID NO:218), Figure 220A-B (SEQ ID NO: 220), Figure 222 (SEQ ID NO:222), Figure 223 (SEQ ID NO:223), Figure 224 (SEQ ID NO:224), Figure 226 (SEQ ID NO:226), Figure 228 (SEQ ID NO:228), Figure 230 (SEQ ID NO:230), Figure 231 (SEQ ID NO:231), Figure 233 (SEQ ID NO:233), Figure 234 (SEQ ID NO:234), Figure 236A-B (SEQ ID NO:236), Figure 238 (SEQ ID NO:238), Figure 240 (SEQ ID NO:240), Figure 241 (SEQ ID NO:241), Figure 242 (SEQ ID NO:242), Figure 244 (SEQ ID NO: 244), Figure 245 (SEQ ID NO:245), Figure 247A-B (SEQ ID NO:247), Figure 249 (SEQ ID NO:249), Figure 251 (SEQ ID NO:251), Figure 253 (SEQ ID NO:253), Figure 255 (SEQ ID NO:255), Figure 257 (SEQ ID NO:257), Figure 259 (SEQ ID NO:259), Figure 261 (SEQ ID NO:261), Figure 263 (SEQ ID NO:263), Figure 265 (SEQ ID NO:265), Figure 267 (SEQ ID NO:267), Figure 269 (SEQ ID NO:269), Figure 271 (SEQ ID NO:271), Figure 273 (SEQ ID NO: 273), Figure 275 (SEQ ID NO:275), Figure 277 (SEQ ID NO:277), Figure 279 (SEQ ID NO:279), Figure 281 (SEQ ID NO:281), Figure 283 (SEQ ID NO:283), Figure 285 (SEQ ID NO:285), Figure 287 (SEQ ID NO:287), Figure 289A-B (SEQ ID NO:289), Figure 291 (SEQ ID NO:291), Figure 293A-B (SEQ ID NO:293), Figure 295 (SEQ ID NO:295), Figure 297 (SEQ ID NO:297), Figure 299A-B (SEQ ID NO:299), Figure 301 (SEQ ID NO:301), Figure 303A-B (SEQ ID NO:303), Figure 305A-B (SEQ ID NO:305), Figure 307 (SEQ ID NO:307), Figure 309 (SEQ ID NO:309), Figure 311A-D (SEQ ID NO:311), Figure 313 (SEQ ID NO:313), Figure 315A-B (SEQ ID NO:315), Figure 317 (SEQ ID NO:317), Figure 319A-B (SEQ ID NO:319), Figure 321 (SEQ ID NO:321), Figure 322 (SEQ ID NO:322), Figure 323 (SEQ ID NO:323), Figure 325 (SEQ ID NO:325), Figure 327 (SEQ ID NO:327), Figure 328 (SEQ ID NO:328), Figure 330 (SEQ ID NO:330), Figure 331 (SEQ ID NO:33 1), Figure 333 (SEQ ID NO:333), Figure 335A-B (SEQ ID NO: 335), Figure 337 (SEQ ID NO:337), Figure 339 (SEQ ID NO:339), Figure 341 (SEQ ID NO:341), Figure 343 (SEQ ID NO:343), Figure 345 (SEQ ID NO:345), Figure 347 (SEQ ID NO:347), Figure 349 (SEQ ID NO:349), Figure 351 (SEQ ID NO:351), Figure 353 (SEQ ID NO:353), Figure 355 (SEQ ID NO:355), Figure 357 (SEQ ID NO:357), Figure 359 (SEQ ID NO:359), Figure 361A-B (SEQ ID NO:361), Figure 363 (SEQ ID NO:363), Figure 365 (SEQ ID NO: 365), Figure 367A-C (SEQ ID NO:367), Figure 369 (SEQ ID NO:369), Figure 371 (SEQ ID NO:371), Figure 373A-B (SEQ ID NO:373), Figure 374 (SEQ ID NO:374), Figure 376 (SEQ ID NO:376), Figure 378 (SEQ ID NO:378), Figure 380 (SEQ ID NO:380), Figure 382 (SEQ ID NO:382), Figure 384A-B (SEQ ID NO:384), Figure 386 (SEQ ID NO:386), Figure 388 (SEQ ID NO:388), Figure 390 (SEQ ID NO:390), Figure 391 (SEQ ID NO:391), Figure 393 (SEQ ID NO:393), Figure 395A-B (SEQ ID NO:395), Figure 397 (SEQ ID NO:397), Figure 398A-B (SEQ ID NO: 398), Figure 400 (SEQ ID NO:400), Figure 401 (SEQ ID NO:401), Figure 402A-B (SEQ ID NO:402), Figure 404 (SEQ ID NO:404), Figure 406 (SEQ ID NO:406), Figure 407 (SEQ ID NO:407), Figure 408 (SEQ ID NO:408), Figure 409 (SEQ ID NO:409), Figure 410 (SEQ ID NO:410), Figure 412 (SEQ ID NO:412), Figure 414 (SEQ ID NO:414), Figure 416 (SEQ ID N0:416), Figure 418 (SEQ ID NO:418), Figure 420A-B (SEQ ID NO:420), Figure 422 (SEQ ID NO:422), Figure 424A-B (SEQ ID NO:424), Figure 426 (SEQ ID NO:426), Figure 428 (SEQ ID NO:428), Figure 430 (SEQ ID NO:430), Figure 432 (SEQ ID NO:432), Figure 434 (SEQ ID NO:434), Figure 436A-B (SEQ ID NO:436), Figure 438A-B (SEQ ID NO:43 8), Figure 440A-B (SEQ ID NO:440), Figure 442A-B (SEQ ID NO:442), Figure 444A-B (SEQ ID NO:444), Figure 446A-B (SEQ ID NO:446), Figure 448 (SEQ ID NO:448), Figure 450A-B (SEQ ID NO: 450), Figure 452 (SEQ ID NO:452), Figure 454 (SEQ ID NO:454), Figure 456 (SEQ ID NO:456), Figure 458 (SEQ ID NO:458), Figure 459 (SEQ ID NO:459), Figure 461 (SEQ ID NO:461), Figure 463A-B (SEQ ID NO:463), Figure 465 (SEQ ID NO:465), Figure 467 (SEQ ID NO:467), Figure 469A-B (SEQ ID NO:469), Figure 470A-B (SEQ ID NO:470), Figure 471A-C (SEQ ID NO:471), Figure 473 (SEQ ID NO:473), Figure 475 (SEQ ID NO:475), Figure 477 (SEQ ID NO:477), Figure 479 (SEQ ID NO:479), Figure 481A-B (SEQ ID NO:481), Figure 483 (SEQ ID NO:483), Figure 485 (SEQ ID NO:485), Figure 487 (SEQ ID NO:487), Figure 489 (SEQ ID NO:489), Figure 491A-B (SEQ ID NO:491), Figure 493 (SEQ ID NO:493), Figure 495 (SEQ ID NO:495), Figure 497 (SEQ ID NO:497), Figure 499 (SEQ ID NO:499), Figure 501 (SEQ ID NO:501), Figure 503 (SEQ ID NO:503) and Figure 505 (SEQ ID NO:505).

5. A vector comprising the nucleic acid of Para. 1.

6. The vector of Para. 5 operably linked to control sequences recognized by a host cell transformed with the vector.

7. A host cell comprising the vector of Para. 5.

8. The host cell of Para. 7, wherein said cell is a CHO cell, an *E. coli* cell or a yeast cell.

9. A process for producing a PRO polypeptide comprising culturing the host cell of Para. 7 under conditions suitable for expression of said PRO polypeptide and recovering said PRO polypeptide from the cell culture.

10. An isolated polypeptide having at least 80% amino acid sequence identity to:

(a) an amino acid sequence of the polypeptide shown Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID N0:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEQ ID NO:113), Figure 115 (SEQ ID NO:115), Figure 117 (SEQ ID NO:117), Figure 119 (SEQ ID NO:119), Figure 121 (SEQ ID NO:121), Figure 123 (SEQ ID NO:123), Figure 125 (SEQ ID NO:125), Figure 127 (SEQ ID NO:127), Figure 129 (SEQ ID NO:129), Figure 131 (SEQ ID NO:131), Figure 133 (SEQ ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO:159), Figure 161 (SEQ ID NO:161), Figure 163 (SEQ ID NO:163), Figure 165 (SEQ ID NO:165), Figure 167 (SEQ ID NO:167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191), Figure 193 (SEQ ID NO:193), Figure 195 (SEQ ID NO:195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEQ ID NO:213), Figure 215 (SEQ ID NO:215, Figure 217 (SEQ ID NO:217), Figure 219 (SEQ ID N0:219), Figure 221 (SEQ ID NO:221), Figure 225 (SEQ ID NO:225), Figure 227 (SEQ ID NO:227), Figure 229 (SEQ ID NO:229), Figure 232 (SEQ ID NO:232), Figure 235 (SEQ ID NO:235), Figure 237 (SEQ ID NO:237), Figure 239 (SEQ ID NO:239), Figure 243 (SEQ ID NO:243), Figure 246 (SEQ ID NO:246), Figure 248 (SEQ ID NO:248), Figure 250 (SEQ ID NO:250), Figure 252 (SEQ ID NO:252), Figure 254 (SEQ ID NO:254), Figure 256 (SEQ ID NO:256), Figure 258 (SEQ ID NO:258), Figure 260 (SEQ ID NO:260), Figure 262 (SEQ ID NO:262), Figure 264 (SEQ ID NO:264), Figure 266 (SEQ ID NO:266), Figure 268 (SEQ ID NO:268), Figure 270 (SEQ ID NO:270), Figure 272 (SEQ ID NO:272), Figure 274 (SEQ ID NO:274), Figure 276 (SEQ ID NO:276), Figure 278 (SEQ ID NO:278), Figure 280 (SEQ ID NO:280), Figure 282 (SEQ ID NO:282), Figure 284 (SEQ ID NO:284), Figure 286 (SEQ ID NO:286), Figure 288 (SEQ ID NO:288), Figure 290 (SEQ ID NO:290), Figure 292 (SEQ ID NO:292), Figure 294 (SEQ ID NO:294), Figure 296 (SEQ ID NO:296), Figure 298 (SEQ ID NO:298), Figure 300 (SEQ ID NO:300), Figure 302 (SEQ ID NO:302), Figure 304 (SEQ ID NO:304), Figure 306 (SEQ ID NO:306), Figure 308 (SEQ ID NO:308), Figure 310 (SEQ ID NO:310), Figure 312A-B (SEQ ID NO:312), Figure 314 (SEQ ID NO:314), Figure 316 (SEQ ID NO:316), Figure 318 (SEQ ID NO:318), Figure 320 (SEQ ID NO:320), Figure 324 (SEQ ID NO:324), Figure 326 (SEQ ID NO:326), Figure 329 (SEQ ID NO:329), Figure 332 (SEQ ID NO:332), Figure 334 (SEQ ID NO:334), Figure 336 (SEQ ID NO:336), Figure 338 (SEQ ID NO:338), Figure 340 (SEQ ID NO:340), Figure 342 (SEQ ID NO:342), Figure 344 (SEQ ID NO:344), Figure 346 (SEQ ID NO:346), Figure 348 (SEQ ID NO:348), Figure 350 (SEQ ID NO:350), Figure 352 (SEQ ID NO:352), Figure 354 (SEQ ID NO:354), Figure 356 (SEQ ID NO:356), Figure 358 (SEQ ID NO:358), Figure 360 (SEQ ID NO:360), Figure 362 (SEQ ID NO:362), Figure 364 (SEQ ID NO:364), Figure 366 (SEQ ID NO:366), Figure 368 (SEQ ID NO:368), Figure 370 (SEQ ID NO:370), Figure 372 (SEQ ID NO: 372), Figure 375 (SEQ ID NO:375), Figure 377 (SEQ ID NO:377), Figure 379 (SEQ ID NO:379), Figure 381 (SEQ ID NO:381), Figure 383 (SEQ ID NO:383), Figure 385 (SEQ ID NO:385), Figure 387 (SEQ ID NO:387), Figure 389 (SEQ ID NO:389), Figure 392 (SEQ ID NO:392), Figure 394 (SEQ ID NO:394), Figure 396 (SEQ ID NO:396), Figure 399 (SEQ ID NO:399), Figure 403 (SEQ ID NO:403), Figure 405 (SEQ ID NO:405), Figure 411 (SEQ ID NO:411), Figure 413 (SEQ ID NO:413), Figure 415 (SEQ ID NO:415), Figure 417 (SEQ ID NO: 417), Figure 419 (SEQ ID NO:419), Figure 421 (SEQ ID NO:421), Figure 423 (SEQ ID NO:423), Figure 425 (SEQ ID NO:425), Figure 427 (SEQ ID NO:427), Figure 429 (SEQ ID NO:429), Figure 431 (SEQ ID N0:431), Figure 433 (SEQ ID NO:433), Figure 435 (SEQ ID NO:435), Figure 437 (SEQ ID NO:437), Figure 439 (SEQ ID N0:439), Figure 441 (SEQ ID NO:441), Figure 443 (SEQ ID NO:443), Figure 445 (SEQ ID NO:445), Figure 447 (SEQ ID NO:447), Figure 449 (SEQ ID NO:449), Figure 451 (SEQ ID NO:451), Figure 453 (SEQ ID NO: 453), Figure 455 (SEQ ID NO:455), Figure 457 (SEQ ID NO:457), Figure 460 (SEQ ID NO:460), Figure 462 (SEQ ID NO:462), Figure 464 (SEQ ID NO:464), Figure 466 (SEQ ID NO:466), Figure 468 (SEQ ID NO:468), Figure 472 (SEQ ID NO:472), Figure 474 (SEQ ID NO:474), Figure 476 (SEQ ID NO:476), Figure 478 (SEQ ID NO:478), Figure 480 (SEQ ID NO:480), Figure 482 (SEQ ID NO:482), Figure 484 (SEQ ID NO:484), Figure 486 (SEQ ID NO:486), Figure 488 (SEQ ID NO:488), Figure 490 (SEQ ID NO:490), Figure 492 (SEQ ID NO: 492), Figure 494 (SEQ ID NO:494), Figure 496 (SEQ ID NO:496), Figure 498 (SEQ ID NO:498), Figure 500 (SEQ ID NO:500), Figure 502 (SEQ ID NO:502), Figure 504 (SEQ ID NO:504) or Figure 506 (SEQ ID NO:506).

12. A chimeric molecule comprising a polypeptide according to Para. 10 fused to a heterologous amino acid sequence.

13. The chimeric molecule of Para. 12, wherein said heterologous amino acid sequence is an epitope tag sequence or an Fc region of an immunoglobulin.

14. An antibody which specifically binds to a polypeptide according to Para. 10.

15. The antibody of Para. 14, wherein said antibody is a monoclonal antibody, a humanized antibody or a single-chain antibody.

16. A composition of matter comprising (a) a polypeptide of Para. 10, (b) an agonist of said polypeptide, (c) an antagonist of said polypeptide, or (d) an antibody that binds to said polypeptide, in combination with a carrier.

17. The composition of matter of Para. 16, wherein said carrier is a pharmaceutically acceptable carrier.

18. The composition of matter of Para. 16 comprising a therapeutically effective amount of (a), (b), (c) or (d).

19. An article of manufacture, comprising:

a container;
a label on said container; and
a composition of matter comprising (a) a polypeptide of Para. 10, (b) an agonist of said polypeptide, (c) an antagonist of said polypeptide, or (d) an antibody that binds to said polypeptide, contained within said container, wherein label on said container indicates that said composition of matter can be used for treating an immune related disease.

20. A method of treating a B cell related disorder in a mammal in need thereof comprising administering to said mammal a therapeutically effective amount of (a) a polypeptide of Para. 10, (b) an agonist of said polypeptide, (c) an antagonist of said polypeptide, or (d) an antibody that binds to said polypeptide.

21. The method of Para. 20, wherein the immune related disorder is; systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typeII hypersensitivity, rheumatoid arthritis, autoimmune mediated hemolytic anemia, myesthenia gravis, hypoadrenocorticism, glomerulonephritis and ankylosing spondylitis.

22. A method for determining the presence of a PRO polypeptide in a sample suspected of containing said polypeptide, said method comprising exposing said sample to an anti-PRO12560, anti-PRO329, anti-PRO71236, anti-PRO1265, anti-PRO71045, anti-PRO071049, anti-PRO37162, anti-PRO160, anti-PRO37534, anti-PRO37544, anti-PRO21787, anti-PRO34330, anti-PRO2540, anti-PR07, anti-PRO34288, anti-PRO84690, anti-PRO2134, anti-PRO21928, anti-PRO23974, anti-PRO34457, anti-PRO37158, anti-PR02537, anti-PRO36827, anti-PRO26296, anti-PRO36766, anti-PRO4912, anti-PRO4769, anti-PRO36899, anti-PRO33667, anti-PRO37695, anti-PRO38069, anti-PRO21716, anti-PRO36124, anti-PRO77694, anti-PRO37957, anti-PRO025114, anti-PRO37553, anti-PRO81979, anti-PRO6013, anti-PRO21960, anti-PRO34276, anti-PRO1717, anti-PRO34107, anti-PRO12612, anti-PRO37946, anti-PRO12865, anti-PRO37029, anti-PRO38337, anti-PRO4710, anti-PRO12570, anti-PRO12890, anti-PRO37121, anti-PRO3813, anti-PRO24934, anti-PRO12458, anti-PRO37843, anti-PRO37547, anti-PR036300, anti-PRO37192, anti-PRO12832, anti-PRO25147, anti-PRO12876, anti-PRO12155, anti-PRO12370, anti-PRO12320, anti-PRO23370, anti-PRO25266, anti-PRO4785, anti-PRO4660, anti-PRO71095, anti-PRO4658, anti-PRO2757, anti-PRO12789, anti-PRO20128, anti-PRO2834, anti-PRO9998, anti-PRO84691, anti-PRO12904, anti-PRO4887, anti-PRO12082, anti-PRO37975, anti-PRO37653, anti-PRO4776, anti-PRO12073, anti-PRO38457, anti-PRO4767, anti-PRO12884, anti-PRO12586, anti-PRO84692, anti-PRO12280, anti-PRO23859, anti-PRO2577, anti-PRO37696, anti-PRO24075, anti-PRO71042, anti-PRO37639, anti-PRO84693, anti-PRO37272, anti-PRO84694, anti-PRO4330, anti-PRO84695, anti-PRO285, anti-PRO38310, anti-PRO37657, anti-PRO71061, anti-PRO209, anti-PRO37584, anti-PRO84696, anti-PRO4860, anti-PRO38492, anti-PRO11738, anti-PRO69476, anti-PRO12032, anti-PRO69484, anti-PRO23460, anti-PRO84476, anti-PRO84697, anti-PRO71207, anti-PRO28714, anti-PRO84698, anti-PRO49839, anti-PRO84441, anti-PRO49214, anti-PRO50523, anti-PRO50241, anti-PRO83773, anti-PRO84699, anti-PRO50772, anti-PRO49326, anti-PRO49824, anti-PRO70333, anti-PRO83673, anti-PRO50332, anti-PRO51295, anti-PRO51621, anti-PRO34958, anti-PRO50821, anti-PRO84700, anti-PRO84701, anti-PRO60333, anti-PRO50187, anti-PRO48357, anti-PRO50365, anti-PRO84702, anti-PRO49810, anti-PRO58710, anti-PRO69503, anti-PRO49564, anti-PRO84703, anti-PRO84663, anti-PRO83800, anti-PRO84704, anti-PRO84705, anti-PRO84706, anti-PRO57996, anti-PRO84368, anti-PRO84496, anti-PRO84707, anti-PRO49765, anti-PRO23253, anti-PRO84709, anti-PRO82352, anti-PRO82903, anti-PRO83260, anti-PRO83681, anti-PRO69487, anti-PRO83148, anti-PRO58958, anti-PRO58399, anti-PRO80649, anti-PRO58425, anti-PRO84376, anti-PRO84309, anti-PRO59142, anti-PRO58810, anti-PRO84710, anti-PRO80648, anti-PRO84711, anti-PRO84712, anti-PRO82872, anti-PRO84714, anti-PRO84183, anti-PRO83879, anti-PRO84715, anti-PRO84716, anti-PRO84717, anti-PRO71206, anti-PRO51950, anti-PRO71035, anti-PRO52268, anti-PRO52040, anti-PRO71288, anti-PRO69458, anti-PRO69903, anti-PRO52672, anti-PRO52174, anti-PRO71289, anti-PRO58230, anti-PRO71238, anti-PRO84718, anti-PRO69632, anti-PRO4913, anti-PRO84265, anti-PRO84719, anti-PRO57922, anti-PRO12613, anti-PR062312, anti-PRO60891, anti-PRO84720, anti-PRO84721, anti-PRO84722, anti-PRO4854, anti-PRO59611, anti-PRO60271, anti-PRO63074, anti-PRO84723, anti-PRO84724, anti-PRO35972, anti-PR084434, anti-PRO59476, anti-PRO84725, anti-PRO84726, anti-PRO84727, anti-PRO84728, anti-PRO84729, anti-PRO84730, anti-PRO84731, anti-PRO21326, anti-PRO69478, anti-PRO80846, anti-PRO84611, anti-PRO61948, anti-PRO84732, anti-PRO84733, anti-PRO59776, anti-PRO84734, anti-PRO83839, anti-PRO69472, anti-PRO23253, anti-PRO84735 or anti-PRO37583 antibody and determining binding of said antibody to a component of said sample.

23. A method of diagnosing a B cell related disease in a mammal, said method comprising detecting the level of expression of a gene encoding PRO12560, PRO329, PRO71236, PRO1265, PR071045, PR071049, PRO37162, PRO160, PRO37534, PRO37544, PR021787, PR034330, PRO2540, PR07, PRO34288, PRO84690, PRO2134, PRO21928, PR023974, PR034457, PR037158, PRO2537, PR036827, PRO26296, PR036766, PRO4912, PRO4769, PR036899, PRO33667, PRO37695, PRO38069, PR021716, PRO36124, PRO77694, PRO37957, PR025114, PRO37553, PRO81979, PRO6013, PR021960, PRO34276, PRO1717, PRO34107, PRO12612, PRO37946, PRO12865, PRO37029, PRO38337, PRO4710, PRO12570, PRO12890, PRO37121, PRO3813, PR024934, PRO12458, PRO37843, PRO37547, PRO36300, PR037192, PRO12832, PRO25147, PRO12876, PRO12155, PRO12370, PRO12320, PRO23370, PR025266, PRO4785, PRO4660, PRO71095, PRO4658, PR02757, PRO12789, PRO20128, PR02834, PRO9998, PRO84691, PRO12904, PRO4887, PRO12082, PR037975, PRO37653, PRO4776, PRO12073, PRO38457, PRO4767, PRO12884, PRO12586, .PRO84692, PRO12280, PRO23859, PRO2577, PRO37696, PRO24075, PRO71042, PRO37639, PRO84693, PR037272, PRO84694, PRO4330, PRO84695, PRO285, PRO38310, PRO37657, PRO71061, PRO209, PR037584, PR084696, PRO4860, PRO38492, PRO11738, PRO69476, PRO12032, PRO69484, PRO23460, PRO84476, PRO84697, PRO71207, PRO28714, PRO84698, PRO49839, PRO84441, PRO49214, PRO50523, PR050241, PRO83773, PRO84699, PRO50772, PRO49326, PRO49824, PRO70333, PRO83673, PRO50332, PRO51295, PRO51621, PRO34958, PRO50821, PRO84700, PRO84701, PRO60333, PRO50187, PRO48357, PRO50365, PRO84702, PRO49810, PRO58710, PRO69503, PRO49564, PRO84703, PRO84663, PRO83800, PRO84704, PRO84705, PRO84706, PRO57996, PRO84368, PRO84496, PRO84707, PRO49765, PRO23253, PRO84709, PRO82352, PRO82903, PRO83260, PRO83681, PRO69487, PRO83148, PRO58958, PRO58399, PRO80649, PRO58425, PRO84376, PRO84309, PRO59142, PRO58810, PRO84710, PRO80648, PRO84711, PRO84712, PRO82872, PRO84714, PRO84183, PR083879, PRO84715, PRO84716, PRO84717, PRO71206, PRO51950, PRO71035, PRO52268, PR052040, PRO71288, PR069458, PRO69903, PRO52672, PRO52174, PRO71289, PRO58230, PRO71238, PRO84718, PRO69632, PRO4913, PRO84265, PRO84719, PRO57922, PRO12613, PRO62312, PRO60891, PRO84720, PRO84721, PRO84722, PRO4854, PRO59611, PRO60271, PRO63074, POP84723, PRO84724, PRO35972, PRO84434, PR059476, PRO84725, PRO84726, PRO84727, PRO84728, PRO84729, PRO84730, PRO84731, PRO21326, PRO69478, PRO80846, PRO84611, PRO61948, PRO84732, PRO84733, PRO59776, PRO84734, PRO83839, PR069472, PRO23253, PRO84735 or PRO37583 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of B cell related disease in the mammal from which the test tissue cells were obtained.

24. A method of diagnosing a B cell related disease in a mammal, said method comprising (a) contacting an anti-PRO12560, anti-PR0329, anti-PR071236, anti-PRO1265, anti-PR071045, anti-PR071049, anti-PRO37162, anti-PRO160, anti-PR037534, anti-PRO37544, anti-PRO21787, anti-PR034330, anti-PRO2540, anti-PR07, anti-PR034288, anti-PR084690, anti-PR02134, anti-PRO21928, anti-PRO23974, anti-PRO34457, anti-PRO37158, anti-PRO2537, anti-PRO36827, anti-PRO26296, anti-PRO36766, anti-PRO4912, anti-PRO4769, anti-PRO36899, anti-PRO33667, anti-PRO37695, anti-PRO38069, anti-PRO21716, anti-PRO36124, anti-PRO77694, anti-PRO37957, anti-PRO25114, anti-PRO37553, anti-PRO81979, anti-PRO6013, anti-PRO21960, anti-PRO34276, anti-PRO1717, anti-PRO34107, anti-PRO12612, anti-PR037946, anti-PRO12865, anti-PRO37029, anti-PRO38337, anti-PRO4710, anti-PRO12570, anti-PRO12890, anti-PRO37121, anti-PRO3813, anti-PRO24934, anti-PRO12458, anti-PR037843, anti-PR037547, anti-PRO36300, anti-PRO37192, anti-PRO12832, anti-PRO25147, anti-PRO12876, anti-PRO12155, anti-PRO12370, anti-PRO12320, anti-PRO23370, anti-PRO25266, anti-PRO4785, anti-PRO4660, anti-PRO71095, anti-PRO4658, anti-PRO2757, anti-PRO12789, anti-PRO20128, anti-PRO2834, anti-PRO9998, anti-PRO84691, anti-PRO12904, anti-PRO4887, anti-PRO12082, anti-PRO37975, anti-PRO37653, anti-PRO4776, anti-PRO12073, anti-PRO38457, anti-PRO4767, anti-PRO12884, anti-PRO12586, anti-PRO84692, anti-PRO12280, anti-PRO23859, anti-PRO2577, anti-PRO37696, anti-PRO24075, anti-PRO71042, anti-PRO37639, anti-PRO84693, anti-PRO37272, anti-PRO84694, anti-PRO4330, anti-PRO84695, anti-PRO285, anti-PRO38310, anti-PRO37657, anti-PRO71061, anti-PRO209, anti-PRO37584, anti-PRO84696, anti-PRO4860, anti-PRO38492, anti-PRO11738, anti-PRO69476, anti-PRO12032, anti-PRO69484, anti-PRO23460, anti-PR084476, anti-PRO84697, anti-PRO71207, anti-PRO28714, anti-PRO84698, anti-PRO49839, anti-PRO84441, anti-PRO49214, anti-PRO50523, anti-PRO50241, anti-PRO83773, anti-PRO84699, anti-PRO50772, anti-PRO49326, anti-PRO49824, anti-PRO70333, anti-PRO83673, anti-PRO50332, anti-PRO51295, anti-PRO51621, anti-PRO34958, anti-PRO50821, anti-PRO84700, anti-PRO84701, anti-PRO60333, anti-PRO50187, anti-PRO48357, anti-PRO50365, anti-PRO84702, anti-PRO49810, anti-PRO58710, anti-PRO69503, anti-PRO49564, anti-PRO84703, anti-PRO84663, anti-PRO83800, anti-PRO84704, anti-PRO84705, anti-PRO84706, anti-PRO57996, anti-PRO84368, anti-PRO084496, anti-PRO84707, anti-PRO049765, anti-PRO23253, anti-PRO84709, anti-PRO82352, anti-PRO82903, anti-PRO83260, anti-PRO83681, anti-PRO69487, anti-PRO83148, anti-PRO58958, anti-PRO58399, anti-PRO80649, anti-PRO58425, anti-PRO84376, anti-PRO84309, anti-PRO59142, anti-PRO58810, anti-PRO84710, anti-PRO080648, anti-PRO84711, anti-PRO84712, anti-PRO82872, anti-PRO84714, anti-PRO84183, anti-PR083879, anti-PRO84715, anti-PRO84716, anti-PRO84717, anti-PRO71206, anti-PRO51950, anti-PRO71035, anti-PRO52268, anti-PRO52040, anti-PRO71288, anti-PRO69458, anti-PRO69903, anti-PRO52672, anti-PRO52174, anti-PRO71289, anti-PRO58230, anti-PRO71238, anti-PRO84718, anti-PRO69632, anti-PRO4913, anti-PRO84265, anti-PRO84719, anti-PRO57922, anti-PRO12613, anti-PRO62312, anti-PRO60891, anti-PRO84720, anti-PRO84721, anti-PRO84722, anti-PRO4854, anti-PRO59611, anti-PRO60271, anti-.PRO63074, anti-PRO84723, anti-PRO84724, anti-PRO35972, anti-PRO84434, anti-PRO59476, anti-PRO84725, anti-PRO84726, anti-PRO84727, anti-PRO84728, anti-PRO84729, anti-PRO84730, anti-PRO84731, anti-PRO21326, anti-PRO69478, anti-PRO80846, anti-PRO84611, anti-PRO61948, anti-PRO84732, ariti-PRO84733, anti-PR059776, anti-PRO84734, anti-PRO83839, anti-PRO69472, anti-PRO23253, anti-PRO84735 or anti-PRO37583 antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.

25. A method of identifying a compound that inhibits the activity of PRO12560, PRO329, PR071236, PRO1265, PRO71045, PRO71049, PRO37162, PRO160, PRO37534, PRO37544, PRO21787, PRO34330, PRO2540, PRO7, PR034288, PRO84690, PRO2134, PR021928, PRO23974, PRO34457, PRO37158, PRO2537, PRO36827, PRO26296, PRO36766, PRO4912, PRO4769, PRO36899, PRO33667, PRO37695, PRO38069, PRO21716, PRO36124, PRO77694, PRO37957, PRO25114, PRO37553, PRO81979, PRO6013, PRO21960, PRO34276, PRO1717, PRO34107, PRO12612, PRO37946, PRO12865, PRO37029, PRO38337, PRO4710, PRO12570, PRO12890, PRO37121, PRO3813, PRO24934, PRO12458, PRO37843, PRO37547, PRO36300, PRO37192, PRO12832, PR025147, PRO12876, PRO12155, PRO12370, PRO12320, PRO23370, PRO25266, PRO4785, PRO4660, PRO71095, PRO4658, PRO2757, PRO12789, PRO20128, PRO2834, PRO9998, PRO84691, PRO12904, PRO4887, PRO12082, PRO37975, PRO37653, PRO4776, PRO12073, PRO38457, PRO4767, PRO12884, PRO12586, PRO84692, PRO12280, PRO23859, PRO2577, PR037696, PRO24075, PRO71042, PRO37639, PRO84693, PRO37272, PRO84694, PRO4330, PRO84695, PRO285, PR038310, PRO37657, PR071061, PRO209, PRO37584, PRO84696, PRO4860, PRO38492, PRO11738, PRO69476, PRO12032, PRO69484, PRO23460, PRO84476, PRO84697, PRO71207, PRO28714, PRO84698, PRO49839, PRO84441, PRO49214, PRO50523, PRO50241, PRO83773, PRO84699, PRO50772, PRO49326, PRO49824, PRO70333, PRO83673, PRO50332, PRO51295, PRO51621, PRO34958, PR050821, PRO84700, PRO84701, PRO60333, PRO50187, PRO48357, PRO50365, PRO84702, PRO49810, PR058710, PRO69503, PRO49564, PRO84703, PRO84663, PRO83800, PRO84704, PRO84705, PRO84706, PRO57996, PRO84368, PRO84496, PRO84707, PRO49765, PRO23253, PRO84709, PRO82352, PRO82903, PR083260, PRO83681, PR069487, PRO83148, PR058958, PR058399, PRO80649, PRO58425, PRO84376, PRO84309, PRO59142, PRO58810, PRO84710, PRO80648, PRO84711, PRO84712, PRO82872, PRO84714, PRO84183, PRO83879, PRO84715, PRO84716, PRO84717, PRO71206, PRO51950, PRO71035, PRO52268, PRO52040, PRO71288, PRO69458, PRO69903, PRO52672, PRO52174, PRO71289, PRO58230, PRO71238, PRO84718, PR069632, PR04913, PRO84265, PRO84719, PRO57922, PRO12613, PRO62312, PRO60891, PRO84720, PRO84721, PRO84722, PRO4854, PRO59611, PRO60271, PRO63074, PRO84723, PR084724, PRO35972, PRO84434, PRO59476, PRO84725, PRO84726, PRO84727, PRO84728, PRO84729, PRO84730, PRO84731, PRO21326, PRO69478, PR080846, PRO84611, PRO61948, PRO84732, PRO84733, PRO59776, PRO84734, PRO83839, PRO69472, PRO23253, PRO84735 or PRO37583 polypeptide, said method comprising contacting cells which normally respond to said polypeptide with (a) said polypeptide and (b) a candidate compound, and determining the lack responsiveness by said cell to (a).

26. A method of identifying a compound that inhibits the expression of a gene encoding a PRO12560, PR0329, PR071236, PRO1265, PRO71045, PRO71049, PR037162, PRO160, PRO37534, PRO37544, PRO21787, PRO34330, PRO2540, PRO7, PRO34288, PRO84690, PR02134, PRO21928, PRO23974, PRO34457, PRO37158, PRO2537, PRO36827, PRO26296, PRO36766, PRO4912, PRO4769, PRO36899, PRO33667, PRO37695, PRO38069, PRO21716, PRO36124, PRO77694, PRO37957, PRO25114, PRO37553, PRO81979, PRO6013, PRO21960, PRO34276, PRO1717, PRO34107, PRO12612, PRO37946, PRO12865, PRO37029, PRO38337, PR04710, PRO12570, PRO12890, PRO37121, PRO3813, PRO24934, PRO12458, PRO37843, PR037547, PRO36300, PR037192, PRO12832, PR025147, PRO12876, PRO12155, PRO12370, PRO12320, PRO23370, PRO25266, PRO4785, PRO4660, PRO71095, PRO4658, PRO2757, PRO12789, PRO20128, PRO2834, PRO9998, PRO84691, PRO12904, PR04887, PRO12082, PRO37975, PRO37653, PRO4776, PRO12073, PRO38457, PR04767, PRO12884, PRO12586, PRO84692, PRO12280, PR023859, PR02577, PR037696, PR024075, PR071042, PR037639, PRO84693, PRO37272, PRO84694, PRO4330, PRO84695, PRO285, PRO38310, PRO37657, PRO71061, PRO209, PRO37584, PRO84696, PRO4860, PR038492, PRO11738, PRO69476, PRO12032, PRO69484, PRO23460, PRO84476, PRO84697, PRO71207, PRO28714, PRO84698, PRO49839, PRO84441, PRO49214, PRO50523, PRO50241, PRO83773, PRO84699, PRO50772, PRO49326, PRO49824, PRO70333, PRO83673, PRO50332, PRO51295, PRO51621, PRO34958, PRO50821, PRO84700, PRO84701, PR060333, PRO50187, PRO48357, PRO50365, PRO84702, PRO49810, PRO58710, PRO69503, PRO49564, PRO84703, PRO84663, PRO83800, PRO84704, PR084705, PRO84706, PRO57996, PRO84368, PRO84496, PRO84707, PRO49765, PRO23253, PRO84709, PRO82352, PR082903, PR083260, PRO83681, PR069487, PRO83148, PR058958, PRO58399, PRO80649, PRO58425, PRO84376, PRO84309, PRO59142, PRO58810, PRO84710, PRO80648, PRO84711, PRO84712, PR082872, PRO84714, PRO84183, PRO83879, PRO84715, PRO84716, PRO84717, PRO71206, PRO51950, PR071035, PRO52268, PRO52040, PRO71288, PRO69458, PRO69903, PR052672, PR052174, PR071289, PRO58230, PRO71238, PRO84718, PRO69632, PRO4913, PRO84265, PRO84719, PR057922, PRO12613, PRO62312, PRO60891, PR084720, PRO84721, PRO84722, PRO4854, PR059611, PRO60271, PRO63074, PR084723, PR084724, PRO35972, PRO84434, PRO59476, PRO84725, PRO84726, PRO84727, PR084728, PRO84729, PRO84730, PRO84731, PR021326, PR069478, PR080846, PRO84611, PR061948, PRO84732, PRO84733, PRO59776, PRO84734, PRO83839, PRO69472, PRO23253, PRO84735 or PRO37583 polypeptide, said method comprising contacting cells which normally express said polypeptide with a candidate compound, and determining the lack of expression said gene.

27. The method of Para. 26, wherein said candidate compound is an antisense nucleic acid.

28. A method of identifying a compound that mimics the activity of a PRO12560, PRO329, PRO71236, PRO1265, PRO71045, PRO71049, PRO37162, PRO160, PRO37534, PRO37544, PRO21787, PRO34330, PRO2540, PRO7, PR034288, PRO84690, PRO2134, PRO21928, PRO23974, PRO34457, PRO37158, PRO2537, PRO36827, PRO26296, PRO36766, PRO4912, PRO4769, PRO36899, PRO33667, PRO37695, PRO38069, PRO21716, PRO36124, PRO77694, PRO37957, PRO25114, PRO37553, PRO81979, PRO6013, PRO21960, PRO34276, PRO1717, PRO34107, PRO12612, PRO37946, PRO12865, PRO37029, PRO38337, PRO4710, PRO12570, PRO12890, PRO37121, PRO3813, PRO24934, PRO12458, PRO37843, PRO37547, PRO36300, PRO37192, PRO12832, PRO25147, PRO12876, PRO12155, PRO12370, PRO12320, PRO23370, PRO25266, PRO4785, PRO4660, PRO71095, PRO4658, PRO2757, PRO12789, PRO20128, PRO2834, PRO9998, PRO84691, PRO12904, PRO4887, PRO12082, PRO37975, PRO37653, PRO4776, PRO12073, PR038457, PR04767, PRO12884, PRO12586, PR084692, PRO12280, PRO23859, PRO2577, PRO37696, PRO24075, PRO71042, PRO37639, PRO84693, PRO37272, PRO84694, PRO4330, PRO84695, PRO285, PRO38310, PRO37657, PRO71061, PRO209, PRO37584, PRO84696, PR04860, PRO38492, PRO11738, PRO69476, PRO12032, PRO69484, PRO23460, PRO84476, PRO84697, PRO71207, PRO28714, PRO84698, PRO49839, PRO84441, PRO49214, PRO50523, PRO50241, PRO83773, PRO84699, PRO50772, PRO49326, PRO49824, PRO70333, PRO83673, PRO50332, PRO51295, PRO51621, PRO34958, PRO50821, PRO84700, PRO84701, PRO60333, PRO50187, PRO48357, PRO50365, PRO84702, PRO49810, PRO58710, PRO69503, PRO49564, PRO84703, PRO84663, PRO83800, PRO84704, PRO84705, PRO84706, PRO57996, PRO84368, PRO84496, PRO84707, PRO49765, PRO23253, PRO84709, PRO82352, PRO82903, PRO83260, PRO83681, PRO69487, PRO83148, PRO58958, PRO58399, PRO80649, PRO58425, PRO84376, PRO84309, PRO59142, PRO58810, PRO84710, PRO80648, PRO84711, PRO84712, PR082872, PRO84714, PRO84183, PRO83879, PRO84715, PRO84716, PRO84717, PRO71206, PRO51950, PRO71035, PRO52268, PR052040, PRO71288, PRO69458, PRO69903, PRO52672, PRO52174, PRO71289, PRO58230, PRO71238, PRO84718, PRO69632, PRO4913, PRO84265, PRO84719, PR057922, PRO12613, PRO62312, PRO60891, PRO84720, PRO84721, PRO84722, PRO4854, PRO59611, PRO60271, PRO63074, PRO84723, PRO84724, PRO35972, PRO84434, PRO59476, PRO84725, PRO84726, PRO84727, PRO84728, PRO84729, PRO84730, PRO84731, PRO21326, PRO69478, PRO80846, PRO84611, PRO61948, PRO84732, PRO84733, PRO59776, PRO84734, PRO83839, PRO69472, PRO23253, PRO84735 or PRO37583 polypeptide, said method comprising contacting cells which normally respond to said polypeptide with a candidate compound, and determining the responsiveness by said cell to said candidate compound.

31. A method of stimulating the B cell response in a mammal, said method comprising administering to said mammal an effective amount of a PRO12560, PR0329, PRO71236, PRO1265, PRO71045, PRO71049, PRO37162, PRO160, PR037534, PRO37544, PR021787, PRO34330, PRO2540, PRO7, PRO34288, PRO84690, PRO2134, PRO21928, PRO23974, PRO34457, PRO37158, PRO2537, PRO36827, PRO26296, PRO36766, PRO4912, PRO4769, PRO36899, PRO33667, PRO37695, PRO38069, PRO21716, PRO36124, PRO77694, PRO37957, PRO25114, PRO37553, PRO81979, PRO6013, PRO21960, PRO34276, PRO1717, PRO34107, PRO12612, PRO37946, PRO12865, PRO37029, PRO38337, PRO4710, PRO12570, PRO12890, PRO37121, PRO3813, PRO24934, PRO12458, PRO37843, PRO37547, PRO36300, PRO37192, PRO12832, PRO25147, PRO12876, PRO12155, PRO12370, PRO12320, PRO23370, PR025266, PRO4785, PRO4660, PRO71095, PRO4658, PR02757, PRO12789, PRO20128, PRO2834, PRO9998, PRO84691, PRO12904, PRO4887, PRO12082, PRO37975, PRO37653, PRO4776, PRO12073, PRO38457, PRO4767, PRO12884, PRO12586, PRO84692, PRO12280, PRO23859, PRO2577, PRO37696, PRO24075, PRO71042, PRO37639, PRO84693, PRO37272, PRO84694, PRO4330, PRO84695, PRO285, PRO38310, PRO37657, PRO71061, PRO209, PRO37584, PRO84696, PRO4860, PRO38492, PRO11738, PRO69476, PRO12032, PRO69484, PRO23460, PRO84476, PRO84697, PRO71207, PRO28714, PRO84698, PRO49839, PRO84441, PRO49214, PRO50523, PRO50241, PRO83773, PR084699, PRO50772, PR049326, PRO49824, PRO70333, PRO83673, PRO50332, PRO51295, PRO51621, PRO34958, PRO50821, PRO84700, PRO84701, PRO60333, PRO50187, PRO48357, PRO50365, PRO84702, PRO49810, PRO58710, PRO69503, PRO49564, PRO84703, PRO84663, PRO83800, PRO84704, PRO84705, PRO84706, PRO57996, PRO84368, PRO84496, PRO84707, PRO49765, PRO23253, PRO84709, PRO82352, PRO82903, PRO83260, PRO83681, PRO69487, PRO83148, PRO58958, PRO58399, PRO80649, PRO58425, PRO84376, PRO84309, PRO59142, PRO58810, PRO84710, PRO80648, PRO84711, PRO84712, PRO82872, PRO84714, PRO84183, PRO83879, PRO84715, PRO84716, PRO84717, PRO71206, PRO51950, PRO71035, PRO52268, PRO52040, PRO71288, PRO69458, PRO69903, PRO52672, PRO52174, PRO71289, PRO58230, PRO71238, PRO84718, PR069632, PRO4913, PRO84265, PRO84719, PRO57922, PRO12613, PRO62312, PRO60891, PRO84720, PRO84721, PRO84722, PRO4854, PRO59611, PRO60271, PRO63074, PRO84723, PRO84724, PRO35972, PRO84434, PRO59476, PRO84725, PRO84726, PRO84727, PRO84728, PRO84729, PRO84730, PRO84731, PRO21326, PRO69478, PRO80846, PRO84611, PRO61948, PRO84732, PRO84733, PR059776, PRO84734, PRO83839, PRO69472, PR023253, PRO84735 or PRO37583 polypeptide antagonist, wherein said B cell response is stimulated.

32. A method of diagnosing a B-cell mediated immune response in a mammal, said method comprising detecting the level of expression of a gene encoding PRO12560, PRO329, PRO71236, PRO1265, PRO71045, PRO71049, PR037162, PRO160, PR037534, PRO37544, PRO21787, PRO34330, PRO2540, PRO7, PRO34288, PRO84690, PRO2134, PRO21928, PR023974, PRO34457, PRO37158, PRO2537, PRO36827, PRO26296, PRO36766, PRO4912, PRO4769, PRO36899, PRO33667, PRO37695, PRO38069, PRO21716, PRO36124, PRO77694, PRO37957, PRO25114, PRO37553, PRO81979, PRO6013, PRO21960, PRO34276, PRO1717, PRO34107, PRO12612, PRO37946, PRO12865, PRO37029, PRO38337, PRO4710, PRO12570, PRO12890, PRO37121, PRO3813, PRO24934, PRO12458, PRO37843, PRO37547, PRO36300, PRO37192, PRO12832, PRO25147, PRO12876, PRO12155, PRO12370, PRO12320, PRO23370, PRO25266, PRO4785, PRO4660, PRO71095, PRO4658, PRO2757, PRO12789, PRO20128, PRO2834, PR09998, PRO84691, PRO12904, PR04887, PRO12082, PR037975, PR037653, PR04776, PRO12073, PRO38457, PRO4767, PRO12884, PRO12586, PR084692, PRO12280, PRO23859, PRO2577, PRO37696, PRO24075, PRO71042, PR037639, PRO84693, PR037272, PRO84694, PRO4330, PRO84695, PRO285, PRO38310, PR037657, PRO71061, PRO209, PRO37584, PRO84696, PRO4860, PRO38492, PRO11738, PR069476, PRO12032, PR069484, PRO23460, PRO84476, PRO84697, PRO71207, PRO28714, PRO84698, PRO49839, PRO84441, PRO49214, PRO50523, PRO50241, PRO83773, PRO84699, PRO50772, PRO49326, PRO49824, PRO70333, PRO83673, PRO50332, PRO51295, PRO51621, PRO34958, PR050821, PRO84700, PRO84701, PRO60333, PR050187, PR048357, PR050365, PR084702, PR049810, PR058710, PR069503, PRO49564, PRO84703, PRO84663, PRO83800, PRO84704, PRO84705, PRO84706, PRO57996, PRO84368, PRO84496, PRO84707, PRO49765, PRO23253, PRO84709, PRO82352, PRO82903, PRO83260, PRO83681, PRO69487, PRO83148, PRO58958, PRO58399, PRO80649, PRO58425, PRO84376, PRO84309, PRO59142, PRO58810, PRO84710, PRO80648, PRO84711, PRO84712, PRO82872, PRO84714, PRO84183, PRO83879, PRO84715, PRO84716, PRO84717, PRO71206, PRO51950, PRO71035, PR052268, PRO52040, PRO71288, PRO69458, PRO69903, PRO52672, PRO52174, PRO71289, PR058230, PRO71238, PRO84718, PRO69632, PRO4913, PRO84265, PRO84719, PRO57922, PRO12613, PRO62312, PRO60891, PRO84720, PRO84721, PRO84722, PRO4854, PRO59611, PRO60271, PRO63074, PRO84723, PRO84724, PRO35972, PRO84434, PRO59476, PRO84725, PRO84726, PRO84727, PRO84728, PRO84729, PRO84730, PRO84731, PRO21326, PRO69478, PRO80846, PRO84611, PRO61948, PRO84732, PRO84733, PRO59776, PRO84734, PRO83839, PRO69472, PRO23253, PRO84735 or PR037583 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of a B cell mediated immune response in the mammal from which the test tissue cells were obtained.

**Claims**

1. A method of diagnosing an immune related disease in a mammal, said method comprising:

   (1) detecting the level of expression of a gene encoding a PRO7 polypeptide, wherein the gene (i) has at least 80% nucleic acid sequence identity to the nucleic acid sequence shown as Figure 28 (SEQ ID NO: 28) or (ii) encodes a polypeptide having at least 80% amino acid sequence identity to the amino acid sequence shown as Figure 29 (SEQ ID NO: 29), (a) in a test sample of tissue cells obtained from the mammal, and (b) in a control sample of known normal tissue cells of the same cell type, wherein a higher level of expression of said gene in the test sample as compared to the control sample is indicative of the presence of said immune related disease in the mammal from which the test tissue cells were obtained; or
   (2) (a) contacting an anti-PR07 antibody to a polypeptide having at least 80% amino acid sequence identity to (i) the polypeptide sequence shown as Figure 29 (SEQ ID NO: 29) or (ii) a polypeptide encoded by the full length coding region of the nucleotide sequence shown as Figure 28 (SEQ ID NO: 28) with a test sample of tissue cells obtained from said mammal and with a control sample of known normal tissue cells of the same cell type and (b) detecting the formation of a complex between the antibody and the polypeptide in the test sample and in the control sample, wherein a larger quantity of complexes formed in the test sample is indicative of the presence of an immune related disease in the mammal from which the test tissue cells were obtained.

**2.** The method of claim 1, wherein the gene encoding a PRO7 polypeptide (i) has at least 90% or at least 95% nucleic acid sequence identity to the nucleic acid sequence shown as Figure 28 (SEQ ID NO: 28) or (ii) encodes a polypeptide having at least 90% or at least 95% amino acid sequence identity to the amino acid sequence shown as Figure 29 (SEQ ID NO: 29).

**3.** The method of claim 1, wherein the gene encoding a PRO7 polypeptide (i) has the nucleic acid sequence shown as Figure 28 (SEQ ID NO: 28) or (ii) encodes a polypeptide having the amino acid sequence shown as Figure 29 (SEQ ID NO: 29).

**4.** The method of claim 1, wherein the anti-PR07 antibody is to a polypeptide (i) having the sequence shown as Figure 29 (SEQ ID NO: 29) or (ii) encoded by the full length coding region of the nucleotide sequence shown as Figure 28 (SEQ ID NO: 28).

**5.** The method of any one of claims 1 to 4, wherein the immune related disorder is a B cell related disorder.

**6.** The method of any one of claims 1 to 5, wherein the disorder is: rheumatoid arthritis, systemic lupus erythematosis, X-linked infantile hypogammaglobulinemia, polysaccaride antigen unresponsiveness, selective IgA deficiency, selective IgM deficiency, selective deficiency of IgG subclasses, immunodeficiency with hyper Ig-M, transient hypogammaglobulinemia of infancy, Burkitt's lymphoma, Intermediate lymphoma, follicular lymphoma, typell hypersensitivity, autoimmune mediated hemolytic anemia, myesthenia gravis, hypoadrenocorticism, glomerulonephritis and ankylosing spondylitis.

**7.** A method of diagnosing a B-cell mediated immune response in a mammal, said method comprising detecting the level of expression of a gene encoding a PRO7 polypeptide, wherein the gene (i) has at least 80% nucleic acid sequence identity to the nucleic acid sequence shown as Figure 28 (SEQ ID NO: 28) or (ii) encodes a polypeptide having at least 80% amino acid sequence identity to the amino acid sequence shown as Figure 29 (SEQ ID NO: 29), (a) in a test sample of tissue cells obtained from the mammal, and (b) in a control sample of known normal tissue cells of the same cell type, wherein a higher level of expression of said gene in the test sample as compared to the control sample is indicative of the presence of a B cell mediated immune response in the mammal from which the test tissue cells were obtained.

**8.** The method of claim 1 (1), wherein nucleic acid levels are determined by hybridization of nucleic acid obtained from the test and control samples to one or more probes specific for the nucleic acid encoding said PRO polypeptide.

**9.** The method of claim 8, wherein hybridization is performed under stringent conditions using 50% formamide, 5x SSC, 50 mM sodium phosphate (pH 6.8), 0.1% sodium pyrophosphate, 5x Denhardt's solution, sonicated salmon sperm DNA (50 $\mu$g/ml), 0.1% SDS, and 10% dextran sulfate at 42°C, with washes at 42°C in 0.2x SSC and 50% formamide at 55°C, followed by a wash consisting of 0.1x SSC containing EDTA at 55°C.

**10.** The method of claim 8, wherein the nucleic acids obtained from the test and normal biological samples are mRNAs.

**11.** The method of claim 1 (1), wherein polypeptide levels are detected with an anti-PR07 antibody to a polypeptide (i) having the sequence shown as Figure 29 (SEQ ID NO: 29) or (ii) encoded by the full length coding region of the nucleotide sequence shown as Figure 28 (SEQ ID NO: 28).

**12.** The method of claim 1, 4 or 11, wherein the anti-PR07 antibody is a monoclonal antibody, a humanized antibody, an antibody fragment, a single chain antibody or is detectably labelled.

# FIGURE 28

GCCCCATCTCCTTGGGCTGCCCGTGCTTCGTGCTT TGGACTACCGCCCAGCAGTGTCCTGCCCTCTGCCTGGGCC
TCGGTCCCTCCTGCACCTGCTGCCTGGATCCCCGGCCTGCCTGGGCCTGGGCCTTGGTTCTCCCC**ATG**ACACCAC
CTGAACGTCTCTTCCTCCCAAGGGTGTGTGGCACCACCCTACACCTCCTCCTTCTGGGGCTGCTGCTGGTTCTGC
TGCCTGGGGCCCAGGGGCTCCCTGGTGTTGGCCTCACACCTTCAGCTGCCCAGACTGCCCGTCAGCACCCCAAGA
TGCATCTTGCCCACAGCACCCTCAAACCTGCTGCTCACCTCATTGGAGACCCCAGCAAGCAGAACTCACTGCTCT
GGAGAGCAAACACGGACCGTGCCTTCCTCCAGGATGGTTTCTCCTTGAGCAACAATTCTCTCCTGGTCCCCACCA
GTGGCATCTACTTCGTCTACTCCCAGGTGGTCTTCTCTGGGAAAGCCTACTCTCCCAAGGCCACCTCCTCCCCAC
TCTACCTGGCCCATGAGGTCCAGCTCTTCTCCTCCAGTACCCCTTCCATGTGCCTCTCCTCAGCTCCCAGAAGA
TGGTGTATCCAGGGCTGCAGGAACCCTGGCTGCACTCGATGTACCACGGGGCTGCGTTCCAGCTCACCCAGGGAG
ACCAGCTATCCACCCACACAGATGGCATCCCCCACCTAGTCCTCAGCCCTAGTACTGTCTTCTTTGGAGCCTTCG
CTCTG**TAG**AACTTGGAAAAATCCAGAAAGAAAAAATAATTGATTTCAAGACCTTCTCCCCATTCTGCCTCCATTC
TGACCATTTCAGGGGTCGTCACCACCTCTCCTTTGGCCATTCCAACAGCTCAAGTCTTCCCTGATCAAGTCACCG
GAGCTTTCAAAGAAGGAATTCTAGGCATCCCAGGGGACCACACCTCCCTGAACCATCCCTGATGTCTGTCTGGCT
GAGGATTTCAAGCCTGCCTAGGAATTCCCAGCCCAAAGCTGTTGGTCTGTCCCACCAGCTAGGTGGGGCCTAGAT
CCACACACAGAGGAAGAGCAGGCACATGGAGGAGC TTGGGGGATGACTAGAGGCAGGGAGGGGACTATTTATGAA
GGCAAAAAAATTAAATTATTTATTTATTTATGGAGGATGGAGAGAGGGGAATAATAGAAGAACATCCAAGGAGAAACAG
AGACAGGCCCAAGAGATGAAGAGTGAGAGGGCATGCGCACAAGGCTGACCAAGAGAGAAAGAAGTAGGCATGAGG
GATCACAGGCCCCAGAAGGCAGGGAAAGGCTCTGAAAGCCAGCTGCCGACCAGAGCCCCACACGGAGGCATCTG
CACCCTCGATGAAGCCCAATAAACCTCTTTTCTCTG

# FIGURE 29

MTPPERLFLPRVCGTTLHLLLLGLLLVLLPGAQGLPGVGLTPSAAQTARQHPKMHLAHSTLKPAAHLIGDPSKQN
SLLWRANTDRAFLQDGFSLSNNSLLVPTSGIYFVYSQVVFSGKAYSPKATSSPLYLAHEVQLFSSQYPFHVPLLS
SQKMVYPGLQEPWLHSMYHGAAFQLTQGDQLSTHTDGIPHLVLSPSTVFFGAFAL

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 404097 A
- WO 9311161 A
- US 4275149 A
- US 4675187 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- US 4376110 A
- US 4873191 A
- US 4736866 A
- WO 9733551 A
- US 4816567 A
- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- US 3773919 A
- EP 616812 A
- US 0110482 W
- EP 307247 A
- US 5122469 A
- WO 8403564 A

### Non-patent literature cited in the description

- **NIELSEN et al.** *Prot. Eng.,* 1997, vol. 10, 1-6
- **VON HEINJE et al.** *Nucl. Acids. Res.,* 1986, vol. 14, 4683-4690
- **ALTSCHUL et al.** *Methods in Enzymology,* 1996, vol. 266, 460-480
- **ALTSCHUL et al.** *Nucleic Acids Res.,* 1997, vol. 25, 3389-3402
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **ZAPATA et al.** *Protein Eng.,* 1995, vol. 8 (10), 1057-1062
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- Cell cycle regulation, oncogens, and antineoplastic drugs. **MURAKAMI et al.** The Molecular Basis of Cancer. WB Saunders, 1995, 13
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415

- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **SAMBROOK et al.** Molecular Cloning: A Laboratory Manual. Cold Spring Harbor Laboratory Press, 1989
- **DIEFFENBACH et al.** PCR Primer: A Laboratory Manual. Cold Spring Harbor Laboratory Press, 1995
- Mammalian Cell Biotechnology: a Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact.,* 1977, vol. 130, 946
- **HSIAO et al.** *Proc. Natl. Acad. Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 154 (2), 737-742
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBURN et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, 269
- **GRAHAM et al.** *J. Gen Virol.,* 1977, vol. 36, 59
- **URLAUB ; CHASIN.** *Proc. Natl. Acad. Sci. USA,* 1980, vol. 77, 4216
- **MATHER.** *Biol. Reprod.,* 1980, vol. 23, 243-251
- **URLAUB et al.** *Proc. Natl. Acad. Sci. USA,* 1980, vol. 77, 4216
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **THOMAS.** *Proc. Natl. Acad. Sci. USA,* 1980, vol. 77, 5201-5205
- **DEUTSCHER.** *Methods in Enzymology,* 1990, 182
- **SCOPES.** Protein Purification: Principles and Practice. Springer-Verlag, 1982
- **ZOLA.** Monoclonal Antibodies: A Manual of Techniques. CRC Press, Inc, 1987, 147-158
- **VAN DER PUTTEN et al.** *Proc. Natl. Acad. Sci. USA,* 1985, vol. 82, 6148-615
- **THOMPSON et al.** *Cell,* 1989, vol. 56, 313-321
- **LO.** *Mol. Cel. Biol.,* 1983, vol. 3, 1803-1814
- **LAVITRANO et al.** *Cell,* 1989, vol. 57, 717-73
- **LASKO et al.** *Proc. Natl. Acad. Sci. USA,* 1992, vol. 89, 6232-636

- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **FIELDS ; SONG.** *Nature (London),* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **ROSSI.** *Current Biology,* 1994, vol. 4, 469-471
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **RIECHMANN et al.** *Nature,* 1988, vol. 332, 323-327
- **VERHOEYEN et al.** *Science,* 1988, vol. 239, 1534-1536
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **MILSTEIN ; CUELLO.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CARON et al.** *J. Exp Med.,* 1992, vol. 176, 1191-1195
- **SHOPES.** *J. Immunol,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- Remington's Pharmaceutical Sciences. 1980
- **MARASCO et al.** *Proc. Natl. Acad. Sci. USA,* 1993, vol. 90, 7889-7893
- Chemotherapy Service. Williams & Wilkins, 1992
- **BOLIVAR et al.** *Gene,* 1977, vol. 2, 95
- **THIMMAPPAYA et al.** *Cell,* 1982, vol. 31, 543
- **SOMPARYRAC et al.** *Proc. Natl. Acad. Sci.,* 1981, vol. 12, 7575
- **AUSUBEL et al.** Current Protocols of Molecular Biology. John Wiley and Sons, 1997
- **LUCAS et al.** *Nucl. Acids Res.,* 1996, vol. 24 (9), 1774-1779
- **O'REILLEY et al.** Baculovirus expression vectors: A Laboratory Manual. Oxford University Press, 1994
- **RUPERT et al.** *Nature,* 1993, vol. 362, 175-179
- **HODGSON.** *Bio/Technology,* 1991, vol. 9, 19-21
- **BRAXTON ; WELLS.** *Biochemistry,* 1992, vol. 31, 7796-7801
- **ATHAUDA et al.** *J. Biochem.,* 1993, vol. 113, 742-746